# EUROPEAN PATENT SPECIFICATION

(11) **EP 4 077 640 B1**
(45) Date of publication and mention of the grant of the patent: **08.05.2024**
(21) Application number: 20838028.7
(22) Date of filing: 18.12.2020
(51) Int. Cl.: C07K 16/28, C12N 5/0783, A61K 35/00, A61K 35/17, A61P 31/12, A61P 35/00

(54) **IMPROVED PROCESS FOR CULTURING TUMOR-INFILTRATING LYMPHOCYTES FOR THERAPEUTIC USE**
VERBESSERTES VERFAHREN ZUR ZÜCHTUNG VON TUMORINFILTRIERENDEN LYMPHOZYTEN FÜR THERAPEUTISCHE ZWECKE
PROCÉDÉ AMÉLIORÉ DE CULTURE DE LYMPHOCYTES INFILTRANT LES TUMEURS À USAGE THÉRAPEUTIQUE

(30) Priority: 18.12.2019 EP 19217356
(43) Date of publication of application: 26.10.2022
(73) Proprietor: CBIO A/S, 2860 Søborg (DK)
(72) Inventor: CORDES, Ulrik, 2920 Charlottenlund (DK); FRIESE, Christina, 2100 Copenhagen Ø (DK); KIRKETERP-MØLLER, Nikolaj, 2200 Copenhagen N (DK); HEEKE, Christina, 2400 Copenhagen NV (DK)
(74) Representative: Aera A/S
(86) International application number: PCT/EP2020/087151
(87) International publication number: WO 2021/123255

(56) References cited:
- WO-A1-2018/075664
- PARK JUNSIK ET AL: "Immune Checkpoint Inhibitor-Induced Reinvigoration of Tumor-Infiltrating CD8 + T Cells is Determined by Their Differentiation Status in Glioblastoma", CLINICAL CANCER RESEARCH, 18 January 2019 (2019-01-18), XP055788766, US ISSN: 1078-0432, DOI: 10.1158/1078-0432.CCR-18-2564 Retrieved from the Internet: URL:https://clincancerres.aacrjournals.org /content/clincanres/early/2019/02/25/1078- 0432.CCR-18-2564.full.pdf>
- MACLEAN HALL ET AL: "Expansion of tumor-infiltrating lymphocytes (TIL) from human pancreatic tumors", JOURNAL FOR IMMUNOTHERAPY OF CANCER, BIOMED CENTRAL LTD, LONDON, UK, vol. 4, no. 1, 18 October 2016 (2016-10-18), pages 1-12, XP021241432, DOI: 10.1186/S40425-016-0164-7
- JESSICA ANN CHACON ET AL: "Triggering co-stimulation directly in melanoma tumor fragments drives CD8 + tumor-infiltrating lymphocyte expansion with improved effector-memory properties", ONCOIMMUNOLOGY, vol. 4, no. 12, 1 July 2015 (2015-07-01), page e1040219, XP055459996, DOI: 10.1080/2162402X.2015.1040219
- FRIESE CHRISTINA ET AL: "CTLA-4 blockade boosts the expansion of tumor-reactive CD8+ tumor-infltrating lymphocytes in ovarian cancer", SCIENTIFIC REPORTS, vol. 10, no. 1, 3 March 2020 (2020-03-03), XP055788756, DOI: 10.1038/s41598-020-60738-4 Retrieved from the Internet: URL:http://www.nature.com/articles/s41598- 020-60738-4>

## Description

### FIELD

The present invention is targeted towards reinvigorating exhausted Tumor Infiltrating Lymphocytes (TILs) *in vitro* by co-culturing excised TIL containing tumor fragments with Tumor Microenvironment (TME) Stimulators, such as Immune Checkpoint Inhibitors (ICIs), stimulating the TILs with other interleukins known to revert T cell exhaustion, and/or inhibiting the effect of regulatory T cells secreted factors (such as inhibiting IL-10) thereby creating a favorable tumor microenvironment where exhausted T-cells can expand faster and to higher numbers than currently established TIL expansion protocols.

### BACKGROUND

Tumor infiltrating lymphocytes are associated with improved prognosis and progression free survival in cancer patients undergoing immunotherapy such as the use of immune checkpoint inhibitors (ICIs) against CTLA-4 and PD-1/PD-L1.

However, still only a fraction of patients has a durable long-term response to such therapies as many other factors seems to be involved in the tumor microenvironment in the down regulation of the immune response. One of the key factors seems to be exhaustion of T-cells resulting in the physical elimination and/or dysfunction of antigen specific T-cells. Factors involved in this exhaustion phenomenon involve surface markers expressed on tumor cells, lymphoid and mononuclear cells and soluble molecules secreted from regulatory T-cells and NK cells in the tumor microenvironment (TME). But, also the lack of stimulatory factors such as interferon gamma and IL-2 is evident in the TME.

Reversal of T-cell exhaustion is a key target in the development of new classes of ICIs either as a mono therapy or in combination with already established therapies. However, as these targets often are also responsible for inducing immune tolerance avoiding autoimmune responses, systemic administration of inhibitors can cause serious side effects. In addition, administering T-cell stimulatory molecules such as IL-2 can also cause serious and sometimes fatal side effects and therefore needs to be managed by skilled clinicians. Some approaches have been taken to administer drug candidates locally into the tumor thereby possibly avoiding systemic side effects. However, as cancer cells are distributed all over the body in many metastatic patients, the likelihood of this approach to be successful under such circumstances can be questioned.

The use of Tumor Infiltrating Lymphocyte (TIL) therapy has shown significant clinical benefit with objective response rates of up to 55% and complete responses in up to 20% of patients in various malignancies such as metastatic melanoma, head and neck and cervical cancer. In short, this kind of therapy leverages the in vitro expansion of autologous T lymphocytes by initially stimulating fragments from the excised tumor with IL-2, anti-CD3 antibodies and feeder cells and thereby growing these cells to the billions before re-administering the T cells back to the patients that have received lymphodepleting therapy where after regression of the tumor is promoted.

The TIL therapy is costly and takes time. It would therefore be advantageous to optimize the current methods and identify ways to shorten the duration for expansion of the TILs, increase the expansion rate, and also achieve more favorable phenotypes.

### SUMMARY

The present invention relates to a method for promoting regression of a cancer in a mammal by expanding tumor infiltrating lymphocytes (TILs) into a therapeutic population of TILs comprising: (a) culturing autologous T cells by obtaining a first population of TILs from a tumor resected from a mammal, (b) performing a first expansion by culturing the first population of TILs in a cell culture medium comprising IL-2 and one or more TME stimulators to produce a second population of TILs wherein the one or more TME stimulators comprises at least a substance of group B selected from one or more from the group consisting of ipilimumab and tremelimumab; (c) performing a second expansion by supplementing the cell culture medium of the second population of TILs with additional IL-2, anti-CD3 antibodies, and antigen presenting cells (APCs), to produce a third population of TILs, wherein the third population of TILs is a therapeutic population; and (d) after administering nonmyeloablative lymphodepleting chemotherapy, administering to the mammal the therapeutic population of T cells, wherein the T cells administered to the mammal, whereupon the regression of the cancer in the mammal is promoted.

A further aspect of the present invention relates to a method for treating a subject with cancer comprising administering expanded tumor infiltrating lymphocytes (TILs) comprising: (a) culturing autologous T cells by obtaining a first population of TILs from a tumor resected from a mammal, (b) performing a first expansion by culturing the first population of TILs in a cell culture medium comprising IL-2 and one or more TME stimulators to produce a second population of TILs, wherein the one or more TME stimulators comprises at least a substance of group B selected from one or more from the group consisting of ipilimumab and tremelimumab; (c) performing a second expansion by supplementing the cell culture medium of the second population of TILs with additional IL-2, anti-CD3 antibodies, and antigen presenting cells (APCs), to produce a third population of TILs, wherein the third population of TILs is a therapeutic population; and (d) after administering nonmyeloablative lymphodepleting chemotherapy, administering to the mammal the therapeutic population of T cells, wherein the T cells administered to the mammal, whereupon the regression of the cancer in the mammal is promoted.

Another aspect of the present invention relates to a method for expanding tumor infiltrating lymphocytes (TILs) into a therapeutic population of TILs comprising: (a) culturing autologous T cells by obtaining a first population of TILs from a tumor resected from a mammal (b) performing a first expansion by culturing the first population of TILs in a cell culture medium comprising IL-2 and one or more TME stimulators to produce a second population of TILs, wherein the one or more TME stimulators comprises at least a substance of group B selected from one or more from the group consisting of ipilimumab and tremelimumab; and (c) performing a second expansion by supplementing the cell culture medium of the second population of TILs with additional IL-2, anti-CD3 antibodies, and antigen presenting cells (APCs), to produce a third population of TILs, wherein the third population of TILs is a therapeutic population.

In one or more embodiments, the one or more TME stimulators are selected from the groups consisting of: substances that are capable of antagonizing and/or inhibiting receptors expressed on T-cells (or their ligands) known to cause T-cell downregulation, deactivation and/or exhaustion, substances that are capable of agonizing and/or stimulating receptors expressed on T-cells known to cause T-cell upregulation, activation, and/or reinvigoration, substances that are capable of antagonizing and/or inhibiting soluble molecules and cytokines and their receptors known to cause T-cell downregulation, deactivation, and/or exhaustion, and substances that are capable of downregulating and/or depleting regulator T-cells thereby favoring ex-vivo T-cell expansion.

In one or more embodiments, the one or more TME stimulators is/are one or more checkpoint inhibitors or inhibitors of their ligands such as anti-PD1, anti-PD-L1, anti-PD-L2, anti-CTLA-4, anti-LAG3, anti-A2AR, anti-B7-H3, anti B7-H4, anti-BTLA, anti-IDO, anti-HVEM, anti-IDO, anti-TDO, anti-KIR, anti-NOX2 , anti-TIM3, anti-galectin-9, anti-VISTA, anti-SIGLEC7/9, and wherein the one or more checkpoint inhibitors or inhibitors of their ligands optionally also are added to the second expansion.

In one or more embodiments, the substances that are capable of antagonizing and/or inhibiting receptors expressed on T-cells (or their ligands) known to cause T-cell downregulation, deactivation and/or exhaustion are selected from the groups consisting of: A: substances that act through the PD-1 receptor on T-cells, B: substances that act through the CTLA-4 receptor on T-cells, C: substances that act through the LAG-3 receptor on T-cells, D: substances that act through the TIGIT/CD226 receptor on T-cells, E: substances that act through the KIR receptor on T-cells, F: substances that act through the TIM-3 receptor on T-cells, G: substances that act through the BTLA receptor on T-cells, and H: substances that act through the A2aR receptor on T-cells.

In one or more embodiments, the substance of group A is selected from one or more from the group consisting of pembrolizumab, nivolumab, cemiplimab, sym021, atezolizumab, avelumab, and durvalumab.

In one or more embodiments, the substance of group B is selected from one or more from the group consisting of ipilimumab and tremelimumab. In one or more embodiments, the substance of group C is selected from one or more from the group consisting of relatlimab, eftilagimo alpha, and sym022. In one or more embodiments, the substance of group D is tiragolumab. In one or more embodiments, the substance of group E is lirilumab. In one or more embodiments, the substance of group F is sym023. In one or more embodiments, the substance of group G is 40E4 and PJ196.

In one or more embodiments, the substances that are capable of agonizing and/or stimulating receptors expressed on T-cells known to cause T-cell upregulation, activation, and/or reinvigoration are selected from the groups consisting of: I: substances that act through the OX40/CD134 receptor on T-cells, J: substances that act through the 4-1 BB/CD137 receptor on T-cells, K: substances that act through the CD28 receptor on T-cells, L: substances that act through the ICOS receptor on T-cells, M: substances that act through the GITR receptor on T-cells, N: substances that act through the CD40L receptor on T-cells, and O: substances that act through the CD27 receptor on T-cells.

In one or more embodiments, the substance of group J is selected from one or more from the group consisting of urelumab and utomilumab. In one or more embodiments, the substance of group K is theraluzimab. In one or more embodiments, the substance of group O is valilumab.

In one or more embodiments, the substances that are capable of antagonizing and/or inhibiting soluble molecules and cytokines and their receptors known to cause T-cell downregulation, deactivation, and/or exhaustion are selected from the groups consisting of: P: substances that act through the IDO1/2 receptor on T-cells, Q: substances that act through the TGFβ receptor on T-cells, R: substances that act through the IL-10 receptor on T-cells, and S: substances that act through the IL-35 receptor on T-cells.

In one or more embodiments, the substance of group P is epacedostat. In one or more embodiments, the substance of group Q is linrodostat. In one or more embodiments, the substance of group R is galunisertib.

In one or more embodiments, the substances that are capable of downregulating and/or depleting regulatory T-cells thereby favoring ex-vivo T-cell expansion are selected from the groups consisting of: T: cyclophosphamides, U: TKIs, V: substances that act through αCD25, and X: IL2/Diphteria toxin fusions.

In one or more embodiments, the substance of group U is sunitinib. In one or more embodiments, the substance of group V is selected from one or more from the group consisting of sorafenib, imatinib and daclizumab. In one or more embodiments, the substance of group X is dinileukin diftitox.

In one or more embodiments, the concentration of substance in is 0.1 µg/mL to 300 µg/mL, such as 1 µg/mL to 100 µg/mL, such as 10 µg/mL to 100 µg/mL, such as 1 µg/mL to 10 µg/mL.

In one or more embodiments, the therapeutic population of T cells is used to treat a cancer type selected from the groups consisting of: 1: solid tumors, 2: ICI naïve tumors, 3: MSI-H tumors, 4: Hematological tumors, virus-induced tumors, and 5: Hyper-mutated tumors (such as POL-E and POL-D mutated tumors).

In one or more embodiments, the therapeutic population of T cells is used to treat a cancer type selected from the groups consisting of breast cancer, renal cell cancer, bladder cancer, melanoma, cervical cancer, gastric cancer, colorectal cancer, lung cancer, head and neck cancer, ovarian cancer, Hodgkin lymphoma, pancreatic cancer, liver cancer, and sarcomas.

In one or more embodiments, the therapeutic population of T cells is used to treat a breast cancer. In one or more embodiments, the therapeutic population of T cells is used to treat renal cell cancer. In one or more embodiments, the therapeutic population of T cells is used to treat bladder cancer. In one or more embodiments, the therapeutic population of T cells is used to treat melanoma. In one or more embodiments, the therapeutic population of T cells is used to treat cervical cancer. In one or more embodiments, the therapeutic population of T cells is used to treat gastric cancer. In one or more embodiments, the therapeutic population of T cells is used to treat colorectal cancer. In one or more embodiments, the therapeutic population of T cells is used to treat lung cancer. In one or more embodiments, the therapeutic population of T cells is used to treat head and neck cancer. In one or more embodiments, the therapeutic population of T cells is used to treat ovarian cancer. In one or more embodiments, the therapeutic population of T cells is used to treat Hodgkin lymphoma. In one or more embodiments, the therapeutic population of T cells is used to treat pancreatic cancer. In one or more embodiments, the therapeutic population of T cells is used to treat liver cancer. In one or more embodiments, the therapeutic population of T cells is used to treat sarcomas.

In one or more embodiments, steps (a) through (c) or (d) are performed within a period of about 20 days to about 45 days. In one or more embodiments, steps (a) through (c) or (d) are performed within a period of about 20 days to about 40 days. In one or more embodiments, steps (a) through (c) or (d) are performed within a period of about 25 days to about 40 days. In one or more embodiments, steps (a) through (c) or (d) are performed within a period of about 30 days to about 40 days. In one or more embodiments, steps (a) through (b) are performed within a period of about 10 days to about 28 days. In one or more embodiments, steps (a) through (b) are performed within a period of about 10 days to about 20 days.

In one or more embodiments, step (c) is performed within a period of about 12 days to about 18 days. In one or more embodiments, step (c) is performed within a period of about 10 days to about 28 days. In one or more embodiments, step (c) is performed within a period of about 10 days to about 20 days. In one or more embodiments, step (c) is performed within a period of about 12 days to about 18 days.

In one or more embodiments, step (b) results in 1 × 10⁶ to 1× 10⁷ cells, such as 2 × 10⁶ to 5× 10⁶ cells. In one or more embodiments, step (b) results in 5× 10⁶ to 1× 10⁷ cells. In one or more embodiments, step (b) results in 1 × 10⁶ to 5× 10⁷ cells. In one or more embodiments, step (b) results in 1 × 10⁷ to 5× 10⁷ cells. In one or more embodiments, step (c) results in 1 × 10⁷ to 1× 10¹² cells, such as 1 × 10⁸ to 5× 10⁹ cells, such as 1 × 10⁹ to 5× 10⁹ cells, such as 1 × 10⁸ to 5× 10¹⁰ cells, such as 1 × 10⁹ to 5× 10¹¹ cells. In one or more embodiments, step (c) results in 1 × 10⁷ to 1x 10¹⁰ cells. In one or more embodiments, step (c) results in 1 × 10⁷ to 1× 10⁹ cells. In one or more embodiments, step (c) results in 1 × 10⁷ to 1× 10⁸ cells.

In one or more embodiments, the APCs are artificial APCs (aAPCs) or allogeneic feeder cells.

In one or more embodiments, the therapeutic population of TILs are infused into a patient.

In one or more embodiments, the cells are removed from the cell culture and cryopreserved in a storage medium prior to performing step (c).

In one or more embodiments, the method further comprises the step of transducing the first population of TILs with an expression vector comprising a nucleic acid encoding a chimeric antigen receptor (CAR) comprising a single chain variable fragment antibody fused with at least one endodomain of a T-cell signaling molecule.

In one or more embodiments, step (c) further comprises a step of removing the cells from the cell culture medium.

In one or more embodiments, step (a) further comprises processing of the resected tumor into multiple tumor fragments, such as 4 to 50 fragments, such as 20 to 30 fragments.

In one or more embodiments, the fragments have a size of about 5 to 50 mm³, In one or more embodiments, the fragments have a size of about 50 mm³. In one or more embodiments, the fragments have a size of about 0.1 to 10 mm³. In one or more embodiments, the fragments have a size of about 0.1 to 1 mm³. In one or more embodiments, the fragments have a size of about 0.5 to 5 mm³. In one or more embodiments, the fragments have a size of about 1 to 10 mm³. In one or more embodiments, the fragments have a size of about 1 to 3 mm³.

In one or more embodiments, the mammal is a human.

In one or more embodiments, the cell culture medium is provided in a container selected from the group consisting of a G-Rex container and a Xuri cellbag.

An aspect relates to a population of tumor infiltrating lymphocytes (TILs) obtainable by a method of any of the previous claims.

A further aspect relates to expanded tumor infiltrating lymphocytes (TILs) for use in treating a subject with cancer, the treatment comprising the steps of: culturing autologous T cells by obtaining a first population of TILs from a tumor resected from a mammal performing a first expansion by culturing the first population of TILs in a cell culture medium comprising IL-2 and one or more TME stimulators to produce a second population of TILs; performing a second expansion by supplementing the cell culture medium of the second population of TILs with additional IL-2, anti-CD3, and antigen presenting cells (APCs), to produce a third population of TILs, wherein the third population of TILs is a therapeutic population; and after administering nonmyeloablative lymphodepleting chemotherapy, administering to the mammal the therapeutic population of T cells, wherein the T cells administered to the mammal, whereupon the regression of the cancer in the mammal is promoted.

Park Junsik et al., Clinical cancer research, 18 January 2019 discloses the differential status of CD8 tumor-infiltrating lymphocytes (TIL) from patients with primary recurrent glioblastoma (GBM). TILs were ex vivo stimulated with anti-CD3/OKT3 and interleukins (IL-2, IL-7, IL-15) in the presence of anti-PD-1 and/or anti-cytotoxic T-lymphocyte antigen 4 (CTLA-4).

Maclean hall et al., Journal for immunotherapy of cancer, vol. 4, no. 1, 18 October 2016, pages 1-12 describee the expansion of TIL from human pancreatic tumors. PD-1 blockage and 4-1BB stimulation were tested.

WO 2018/075664 shows general knowledge in the field of TIL expansion protocols.

Jessica Ann Cacon et al., Oncoimmunology, vol. 4, no. 12, 1 July 2015 is a review paper on ACT using TILs as a treatment for melanoma.

Friese Christina et al., Scientific reports, vol. 10, no. 1, 3 March 2020 describes that autologous TILs can be expanded directly by manipulating pathways, including the use of CTLA-4.

### DETAILED DESCRIPTION

The present invention is targeted towards reinvigorating exhausted Tumor Infiltrating Lymphocytes (TILs) *in vitro* by co-culturing excised TIL containing tumor fragments with for example checkpoint inhibitors, stimulating the TILs with other interleukins known to revert T cell exhaustion, and/or inhibiting the effect of regulatory T cells secreted factors (such as IL-10) thereby creating a favorable TME where exhausted T-cells can expand faster and to higher numbers than currently established TIL expansion protocols.

This approach is possibly advantageous to systemically administered therapies as the in vitro stimulation can be performed using dose levels that are much higher than would be tolerated in vivo. As an example, current TIL protocols utilizes IL-2 at a concentration at 3-6,000 IU per mL, which is 5-10 higher than the systemically recommended dose.

In addition, as T cells that have a higher affinity to tumor antigens might have an increased tendency to get exhausted, targeted in-vitro reinvigoration might help expand higher affinity T cell clones that more aggressively can target the tumor where they are residing thereby eventually leading to an improved clinical outcome of this novel approach to TIL therapy.

Thus, the present invention relates to a method for promoting regression of a cancer in a mammal by expanding tumor infiltrating lymphocytes (TILs) into a therapeutic population of TILs comprising: (a) culturing autologous T cells by obtaining a first population of TILs from a tumor resected from a mammal, (b) performing a first expansion by culturing the first population of TILs in a cell culture medium comprising IL-2 and one or more TME stimulators to produce a second population of TILs, wherein the one or more TME stimulators comprises at least a substance of group B selected from one or more from the group consisting of ipilimumab and tremelimumab; (c) performing a second expansion by supplementing the cell culture medium of the second population of TILs with additional IL-2, anti-CD3 antibody, and antigen presenting cells (APCs), to produce a third population of TILs, wherein the third population of TILs is a therapeutic population; and (d) after administering nonmyeloablative lymphodepleting chemotherapy, administering to the mammal the therapeutic population of T cells, wherein the T cells administered to the mammal, whereupon the regression of the cancer in the mammal is promoted.

By "tumor infiltrating lymphocytes" or "TILs" herein is meant a population of cells originally obtained as white blood cells that have left the bloodstream of a subject and migrated into a tumor. TILs include, but are not limited to, CD8+ cytotoxic T cells (lymphocytes), Th1 and Th17 CD4+ T cells (CD4+ helper cells), natural killer cells, dendritic cells and Ml macrophages. TILs include both primary and secondary TILs. "Primary TILs" are those that are obtained from patient tissue samples as outlined herein (sometimes referred to as "freshly harvested"), and "secondary TILs" are any TIL cell populations that have been expanded or proliferated as discussed herein. TILs can generally be defined either biochemically, using cell surface markers, or functionally, by their ability to infiltrate tumors and effect treatment. TILs can be generally categorized by expressing one or more of the following biomarkers: CD4, CD8, TCR ab, CD27, CD28, CD56, CCR7, CD45Ra, CD95, PD-1, and CD25. Additionally, and alternatively, TILs can be functionally defined by their ability to infiltrate solid tumors upon reintroduction into a patient. TILs may further be characterized by potency - for example, TILs may be considered potent if, for example, interferon (IFN) release is greater than about 50 pg/mL, greater than about 100 pg/mL, greater than about 150 pg/mL, or greater than about 200 pg/mL.

A further aspect of the present invention relates to a method for treating a subject with cancer comprising administering expanded tumor infiltrating lymphocytes (TILs) comprising: (a) culturing autologous T cells by obtaining a first population of TILs from a tumor resected from a mammal, (b) performing a first expansion by culturing the first population of TILs in a cell culture medium comprising IL-2 and one or more TME stimulators to produce a second population of TILs, wherein the one or more TME stimulators comprises at least a substance of group B selected from one or more from the group consisting of ipilimumab and tremelimumab; (c) performing a second expansion by supplementing the cell culture medium of the second population of TILs with additional IL-2, anti-CD3 antibody, and antigen presenting cells (APCs), to produce a third population of TILs, wherein the third population of TILs is a therapeutic population; and (d) after administering nonmyeloablative lymphodepleting chemotherapy, administering to the mammal the therapeutic population of T cells, wherein the T cells administered to the mammal, whereupon the regression of the cancer in the mammal is promoted.

The terms "treatment", "treating", "treat", and the like, refer to obtaining a desired pharmacologic and/or physiologic effect. The effect may be prophylactic in terms of completely or partially preventing a disease or symptom thereof and/or may be therapeutic in terms of a partial or complete cure for a disease and/or adverse effect attributable to the disease. "Treatment", as used herein, covers any treatment of a disease in a mammal, particularly in a human, and includes: (a) preventing the disease from occurring in a subject which may be predisposed to the disease but has not yet been diagnosed; (b) inhibiting the disease, i.e., arresting its development or progression; and (c) relieving the disease, i.e., causing regression of the disease and/or relieving one or more disease symptoms. "Treatment" is also meant to encompass delivery of an agent in order to provide for a pharmacologic effect, even in the absence of a disease or condition. For example, "treatment" encompasses delivery of a composition that can elicit an immune response or confer immunity in the absence of a disease condition, e.g., in the case of a vaccine.

The term "anti-CD3 antibody" refers to an antibody or variant thereof, e.g, a monoclonal antibody and including human, humanized, chimeric or murine antibodies which are directed against the CD3 receptor in the T cell antigen receptor of mature T cells. Anti-CD3 antibodies include OKT3, also known as muromonab. Anti-CD3 antibodies also include the UHCT1 clone, also known as T3 and CD3e. Other anti-CD3 antibodies include, for example, otelixizumab, teplizumab, and visilizumab. In an embodiment, the cell culture medium comprises OKT3 antibody. In some embodiments, the cell culture medium comprises about 30 ng/mL of OKT3 antibody. In an embodiment, the cell culture medium comprises about 0.1 ng/mL, about 0.5 ng/mL, about 1 ng/mL, about 2.5 ng/mL, about 5 ng/mL, about 7.5 ng/mL, about 10 ng/mL, about 15 ng/mL, about 20 ng/mL, about 25 ng/mL, about 30 ng/mL, about 35 ng/mL, about 40 ng/mL, about 50 ng/mL, about 60 ng/mL, about 70 ng/mL, about 80 ng/mL, about 90 ng/mL, about 100 ng/mL, about 200 ng/mL, about 500 ng/mL, and about 1 pg/mL of OKT3 antibody. In an embodiment, the cell culture medium comprises between 0.1 ng/mL and 1 ng/mL, between 1 ng/mL and 5 ng/mL, between 5 ng/mL and 10 ng/mL, between 10 ng/mL and 20 ng/mL, between 20 ng/mL and 30 ng/mL, between 30 ng/mL and 40 ng/mL, between 40 ng/mL and 50 ng/mL, and between 50 ng/mL and 100 ng/mL of OKT3 antibody. In some embodiments, the cell culture medium does not comprise OKT3 antibody.

The term "IL-2" (also referred to herein as "IL2") refers to the T cell growth factor known as interleukin-2, and includes all forms of IL-2 including human and mammalian forms, conservative amino acid substitutions, glycoforms, biosimilars, and variants thereof.

After preparation of the tumor fragments, the resulting cells (i.e., fragments) are cultured in media containing IL-2 under conditions that favor the growth of TILs over tumor and other cells. In some embodiments, the tumor digests are incubated in 2 mL wells in media comprising inactivated human AB serum (or, in some cases, as outlined herein, in the presence of aAPC cell population) with 6000 IU/mL of IL-2. This primary cell population is cultured for a period of days, generally from 6 to 14 days, resulting in a bulk TIL population, generally about 1 × 10⁶ to 1 × 10⁸ bulk TIL cells. In some embodiments, the growth media during the first expansion comprises IL-2 or a variant thereof. In some embodiments, the IL is recombinant human IL-2 (rhIL-2). In some embodiments the IL-2 stock solution has a specific activity of 20-30 × 10⁶ IU/mg for a 1 mg vial. In some embodiments the IL-2 stock solution has a specific activity of 20 × 10⁶ IU/mg for a 1 mg vial. In some embodiments the IL-2 stock solution has a specific activity of 25 × 10⁶ IU/mg for a 1 mg vial. In some embodiments the IL-2 stock solution has a specific activity of 30× 10⁶ IU/mg for a 1 mg vial. In some embodiments, the IL- 2 stock solution has a final concentration of 4-8 × 10⁶ IU/mg of IL-2. In some embodiments, the IL- 2 stock solution has a final concentration of 5-7× 10⁶ IU/mg of IL-2. In some embodiments, the IL- 2 stock solution has a final concentration of 6 × 10⁶ IU/mg of IL-2. In some embodiments, the IL-2 stock solution is prepare as described in the examples. In some embodiments, the first expansion culture media comprises about 10,000 IU/mL of IL-2, about 9,000 IU/mL of IL-2, about 8,000 IU/mL of IL-2, about 7,000 IU/mL of IL-2, about 6000 IU/mL of IL-2 or about 5,000 IU/mL of IL-2. In some embodiments, the first expansion culture media comprises about 9,000 IU/mL of IL-2 to about 5,000 IU/mL of IL-2. In some embodiments, the first expansion culture media comprises about 8,000 IU/mL of IL-2 to about 6,000 IU/mL of IL-2. In some embodiments, the first expansion culture media comprises about 7,000 IU/mL of IL-2 to about 6,000 IU/mL of IL-2. In some embodiments, the first expansion culture media comprises about 6,000 IU/mL of IL-2. In an embodiment, the cell culture medium further comprises IL-2. In some embodiments, the cell culture medium comprises about 3000 IU/mL of IL-2. In an embodiment, the cell culture medium further comprises IL-2. In a preferred embodiment, the cell culture medium comprises about 3000 IU/mL of IL-2. In an embodiment, the cell culture medium comprises about 1000 IU/mL, about 1500 IU/mL, about 2000 IU/mL, about 2500 IU/mL, about 3000 IU/mL, about 3500 IU/mL, about 4000 IU/mL, about 4500 IU/mL, about 5000 IU/mL, about 5500 IU/mL, about 6000 IU/mL, about 6500 IU/mL, about 7000 IU/mL, about 7500 IU/mL, or about 8000 IU/mL of IL-2. In an embodiment, the cell culture medium comprises between 1000 and 2000 IU/mL, between 2000 and 3000 IU/mL, between 3000 and 4000 IU/mL, between 4000 and 5000 IU/mL, between 5000 and 6000 IU/mL, between 6000 and 7000 IU/mL, between 7000 and 8000 IU/mL, or about 8000 IU/mL of IL-2.

IL-4, IL-7, IL-15 and/or IL-21 can also be added to step (b) and/or (c) of the present methods. The term"IL-4" (also referred to herein as"IL4") refers to the cytokine known as interleukin 4, which is produced by Th2 T cells and by eosinophils, basophils, and mast cells. IL-4 regulates the differentiation of naive helper T cells (ThO cells) to Th2 T cells. The term "IL-7" (also referred to herein as "IL7") refers to a glycosylated tissue-derived cytokine known as interleukin 7, which may be obtained from stromal and epithelial cells, as well as from dendritic cells. The term"IL-15" (also referred to herein as"IL15") refers to the T cell growth factor known as interleukin- 15, and includes all forms of IL-2 including human and mammalian forms, conservative amino acid substitutions, glycoforms, biosimilars, and variants thereof. The term "IL-21" (also referred to herein as "IL21") refers to the pleiotropic cytokine protein known as interleukin-21, and includes all forms of IL-21 including human and mammalian forms, conservative amino acid substitutions, glycoforms, biosimilars, and variants thereof.

Another aspect of the present invention relates to a method for expanding tumor infiltrating lymphocytes (TILs) into a therapeutic population of TILs comprising: (a) culturing autologous T cells by obtaining a first population of TILs from a tumor resected from a mammal (b) performing a first expansion by culturing the first population of TILs in a cell culture medium comprising IL-2 and one or more TME stimulators to produce a second population of TILs; and (c) performing a second expansion by supplementing the cell culture medium of the second population of TILs with additional IL-2, anti-CD3 antibody, and antigen presenting cells (APCs), to produce a third population of TILs, wherein the third population of TILs is a therapeutic population.

Experimental findings indicate that lymphodepletion prior to adoptive transfer of tumor-specific T lymphocytes plays a key role in enhancing treatment efficacy by eliminating regulatory T cells and competing elements of the immune system ("cytokine sinks"). Accordingly, some embodiments of the invention utilize a lymphodepletion step (sometimes also referred to as "immunosuppressive conditioning") on the patient prior to the introduction of the TILs of the invention.

The methods of the present invention, from step (a) to step (c), can be performed in a closed system. The term "closed system" refers to a system that is closed to the outside environment. Any closed system appropriate for cell culture methods can be employed with the methods of the present invention. Closed systems include, for example, but are not limited to closed G-containers. Once a tumor segment is added to the closed system, the system is no opened to the outside environment until the TILs are ready to be administered to the patient.

The term "TME stimulators" relates to substances (or agents) that have the ability to create a favorable microenvironment within the tumor where exhausted T-cells can be reinvigorated in order to expand many fold and restore their anti-tumor functionality. Thus, in one or more embodiments, the one or more TME stimulators are selected from the groups consisting of: (x) one or more substances that are capable of antagonizing and/or inhibiting receptors expressed on T-cells (or their ligands) known to cause T-cell downregulation, deactivation and/or exhaustion, (y) one or more substances that are capable of agonizing and/or stimulating receptors expressed on T-cells known to cause T-cell upregulation, activation, and/or reinvigoration, (z) one or more substances that are capable of antagonizing and/or inhibiting soluble molecules and cytokines and their receptors known to cause T-cell downregulation, deactivation, and/or exhaustion, and (v) one or more substances that are capable of downregulating and/or depleting regulatory T-cells thereby favoring ex-vivo effector T-cell expansion, and (w) one or more substances from the groups (x), (y), (z) and/or (v). Group (w) can be one, two or three of the substances from (x), (y), (z) and/or (v). In one or more embodiments, (w) is one or two of the substances from (x). In one or more embodiments, (w) is one or two of the substances from (y). In one or more embodiments, (w) is one or two of the substances from (z). In one or more embodiments, (w) is one or two of the substances from (v). (w) can also be any of the combinations of substances in Table 1 listed in Tables 2-41 and 43-44.

These may be added in step (b) and/or step (c) of the present methods, and can be removed during the expansions after 2, 4, 6 or more days if they are only need for the initial expansion. They can be removed by washing of the cell culture. The individual TME stimulators can be added together or in time lapse, i.e. one day apart, or such as 2, 3, 4, 5, 6 or 7 days apart.

In one or more embodiments, the one or more TME stimulators is/are one or more checkpoint inhibitors or inhibitors of their ligands such as anti-PD1, anti-PD-L1, anti-PD-L2, anti-CTLA-4, anti-LAG3, anti-A2AR, anti-B7-H3, anti B7-H4, anti-BTLA, anti-IDO, anti-HVEM, anti-IDO, anti-TDO, anti-KIR, anti-NOX2 , anti-TIM3, anti-galectin-9, anti-VISTA, anti-SIGLEC7/9, and wherein the one or more checkpoint inhibitors or inhibitors of their ligands optionally also are added to the second expansion.

In one or more embodiments, the substances that are capable of antagonizing and/or inhibiting receptors expressed on T-cells (or their ligands) known to cause T-cell downregulation, deactivation and/or exhaustion are selected from the groups consisting of: A: substances that act through the PD-1 receptor on T-cells, B: substances that act through the CTLA-4 receptor on T-cells, C: substances that act through the LAG-3 receptor on T-cells, D: substances that act through the TIGIT/CD226 receptor on T-cells, E: substances that act through the KIR receptor on T-cells, F: substances that act through the TIM-3 receptor on T-cells, G: substances that act through the BTLA receptor on T-cells, and H: substances that act through the A2aR receptor on T-cells. It is to be understood that the definition of substances that act through a given receptor also can cover the same receptors ligand. This means e.g. that for the PD-1 receptor can substances that target the PD-L1 or PD-L2 also be covered. Group A can therefore cover substances that act through the PD-1 receptor on T-cells as well as its ligand(s).

In one or more embodiments, the substance of group A is selected from one or more from the group consisting of pembrolizumab, nivolumab, cemiplimab, sym021, atezolizumab, avelumab, and durvalumab. In one or more embodiments, the substance of group A is pembrolizumab. In one or more embodiments, the substance of group A is nivolumab. In one or more embodiments, the substance of group A is cemiplimab. In one or more embodiments, the substance of group A is sym021. In one or more embodiments, the substance of group A is atezolizumab. In one or more embodiments, the substance of group A is avelumab. In one or more embodiments, the substance of group A is durvalumab.

In one or more embodiments, the substance of group B is selected from one or more from the group consisting of ipilimumab and tremelimumab. In one or more embodiments, the substance of group B is ipilimumab. In one or more embodiments, the substance of group B is tremelimumab. In one or more embodiments, the substance of group C is selected from one or more from the group consisting of relatlimab, eftilagimo alpha, and sym022. In one or more embodiments, the substance of group D is tiragolumab. In one or more embodiments, the substance of group E is lirilumab. In one or more embodiments, the substance of group F is sym023. In one or more embodiments, the substance of group G is 40E4 and PJ196.

In one or more embodiments, the substances that are capable of agonizing and/or stimulating receptors expressed on T-cells known to cause T-cell upregulation, activation, and/or reinvigoration are selected from the groups consisting of: I: substances that act through the OX40/CD137 receptor on T-cells, J: substances that act through the 4-1BB/CD137 receptor on T-cells, K: substances that act through the CD28 receptor on T-cells, L: substances that act through the ICOS receptor on T-cells, M: substances that act through the GITR receptor on T-cells, N: substances that act through the CD40L receptor on T-cells, and O: substances that act through the CD27 receptor on T-cells.

In one or more embodiments, the substance of group J is selected from one or more from the group consisting of urelumab and utomilumab. In one or more embodiments, the substance of group J is urelumab. In one or more embodiments, the substance of group J is utomilumab. The group J substances can be used in combination with an anti-CD3 substance such as OKT-3. One combination can therefore be urelumab and OKT-3 (urelumab/OKT-3). Another combination can be utomilumab and OKT-3 (utomilumab /OKT-3). In one or more embodiments, the substance of group K is theralizumab. In one or more embodiments, the substance of group O is valilumab.

In one or more embodiments, one or more of the substances of group A can be combined with one or more of the substances of group B. In one or more embodiments, one or more of the substances of group A can be combined with one or more of the substances of group B, and with one or more of the substances of group J. These combinations are shown to be effective in the examples of the present disclosure. This means that one or more substances of group A selected from one or more from the group consisting of pembrolizumab, nivolumab, cemiplimab, sym021, atezolizumab, avelumab can be combined with one or more of the substances of group B which is selected from one or more from the group consisting of ipilimumab and tremelimumab. These can then be combined with one or more substances of group J which is selected from one or more from the group consisting of urelumab and utomilumab. The group J substances can be used in combination with an anti-CD3 substance such as OKT-3. One combination can therefore be one or more substances of group A selected from one or more from the group consisting of pembrolizumab, nivolumab, cemiplimab, sym021, atezolizumab, avelumab combined with ipilimumab from group B and urelumab from group J. A specific selection can be pembrolizumab combined with ipilimumab from group B and urelumab from group J, with or without an anti-CD3 substance such as OKT-3.

In one or more embodiments, the substances that are capable of antagonizing and/or inhibiting soluble molecules and cytokines and their receptors known to cause T-cell downregulation, deactivation, and/or exhaustion are selected from the groups consisting of: P: substances that act through the IDO1/2 receptor on T-cells, Q: substances that act through the TGFβ receptor on T-cells, R: substances that act through the IL-10 receptor on T-cells, and S: substances that act through the IL-35 receptor on T-cells.

In one or more embodiments, the substance of group P is epacedostat. In one or more embodiments, the substance of group Q is linrodostat. In one or more embodiments, the substance of group R is galunisertib.

In one or more embodiments, the substances that are capable of downregulating and/or depleting regulatory T-cells thereby favoring ex-vivo effector T-cell expansion are selected from the groups consisting of: T: cyclophosphamides, U: TKIs, V: substances that act through αCD25, and X: IL2/Diphteria toxin fusions.

The groups A-X listed in Table 1 can be combined and used as multiple substances as seen in Tables 2-44. Thus, in one or more embodiments is IL2 used in any of the combination with any of the substances (see Table 1) in the first expansion, i.e. step (b) of the methods of the present invention in any of the combinations listed in Tables 2-44.

In one or more embodiments, the substance of group U is sunitinib. In one or more embodiments, the substance of group V is selected from one or more from the group consisting of sorafenib, imatinib and daclizumab. In one or more embodiments, the substance of group X is dinileukin diftitox.

Example 4 demonstrated that the success rate of TIL expansion ex vivo was increased, when TME stimulators were added to the culture medium when TIL cultures were initiated as described in example 2. Example 5 demonstrated that the TIL yield was increased and the culture time of TILs was reduced, when TME stimulators were added to the culture medium as performed in example 2, when TIL cultures were initiated. Example 6 performed as described in example 2 demonstrated that the TIL yield was increased, when TME stimulators were added to the culture medium in different concentrations, when TIL cultures from various tumor types were initiated.

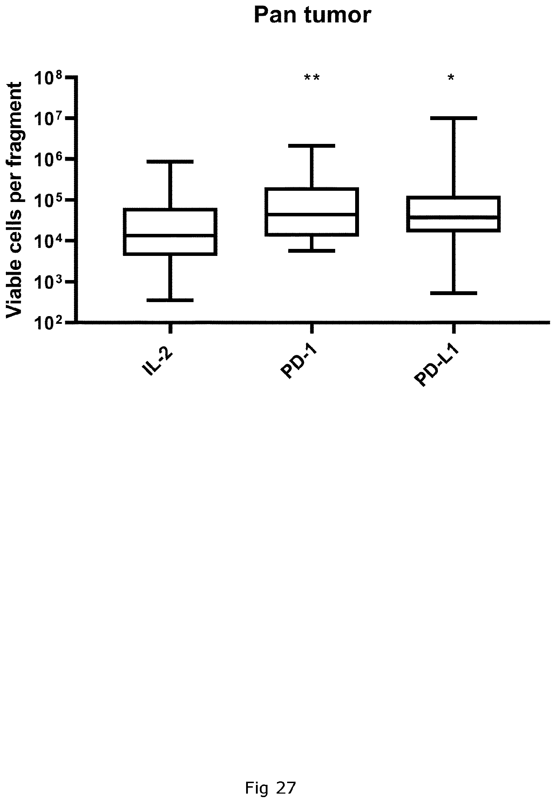

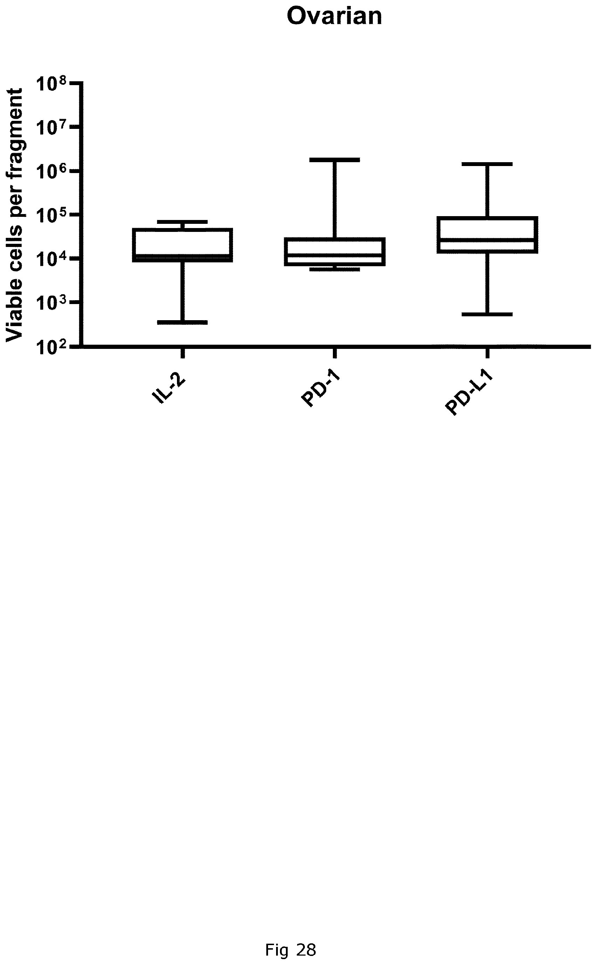

Example 9 illustrated in figure 27 demonstrated that adding TME stimulators to the standard young TIL manufacturing protocol as performed in example 2 significantly enhanced TIL growth which resulted in higher numbers of viable cells per tumor fragment by either antagonizing a receptor expressed on T-cells (in this case PD-1) or its ligand (PD-L1) expressed on tumor cells and other cells in the tumor microenvironment. In figures 28-33, the effect of adding TME stimulators to the initial TIL cultures from the PD-1 group or the PD-L1 group to the standard TIL manufacturing protocol was illustrated in ovarian, melanoma, lung, head and neck, colorectal, and cervical cancer, respectively. Although not significant in all conditions, the effect illustrated a similar pattern between cancers as the pan-tumor PD-1/PD-L1 example in figure 27. The example showed that targeting either the receptor PD-1 or its ligand PD-L1 were interchangeable and could generate a similar effect.

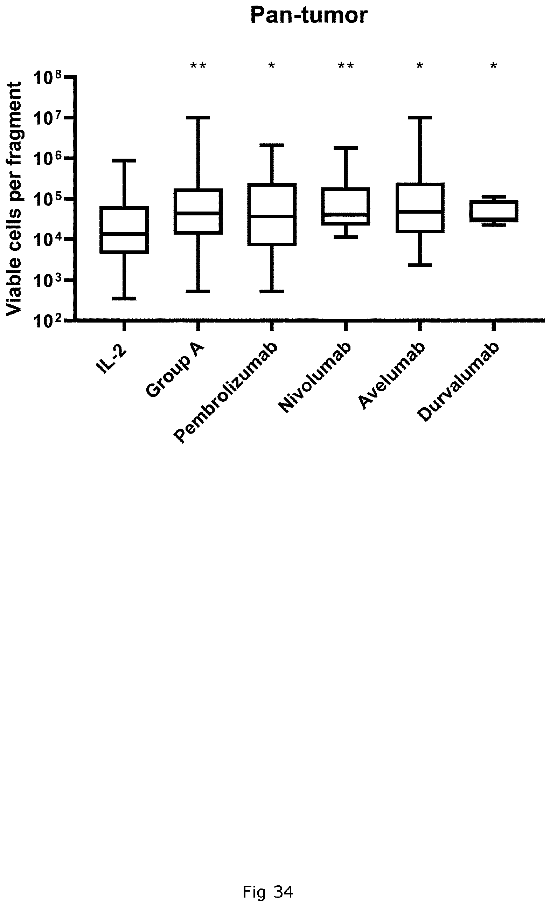

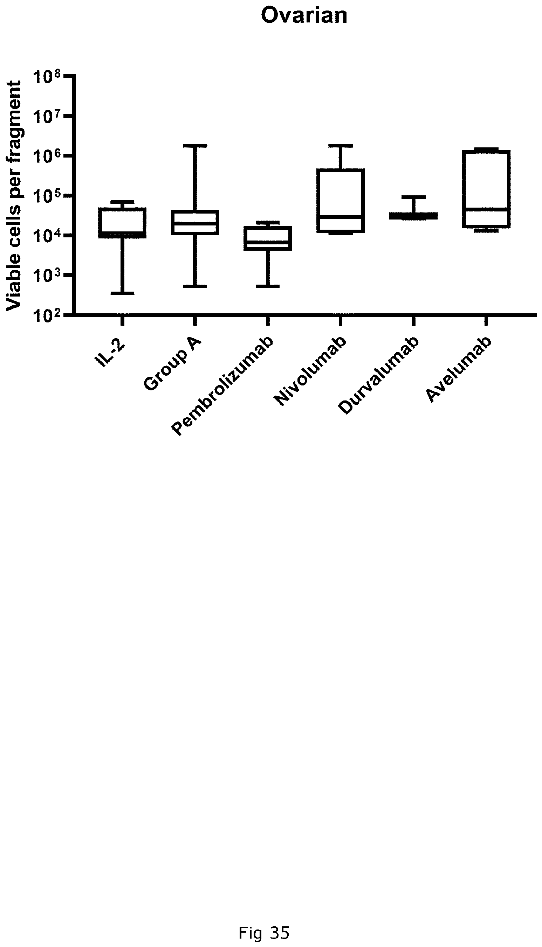

In figures 35-38, the effect of adding inhibitors from group A from different manufacturers to the initial TIL cultures was illustrated in ovarian, melanoma, head and neck, and cervical cancer, respectively. The effect illustrated a similar pattern between cancers as the pan-tumor example in figure 34 and showed the TME stimulator from different manufacturers could be used to obtain a similar effect.

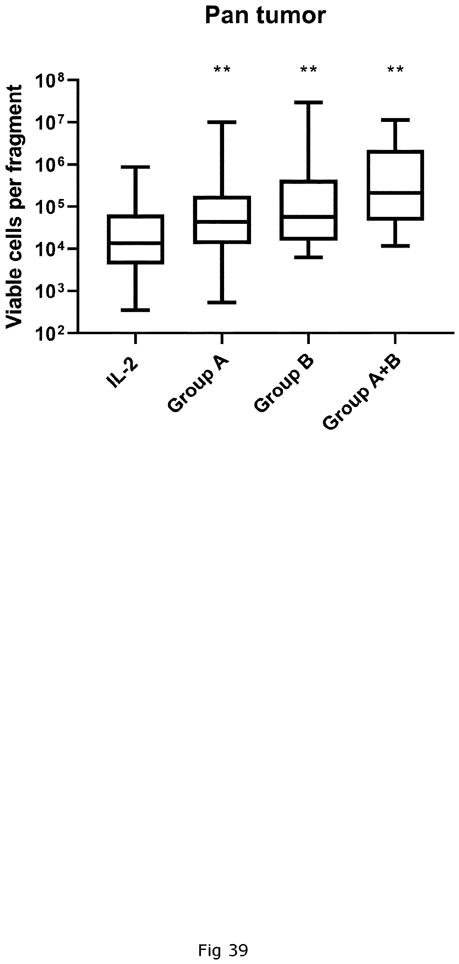

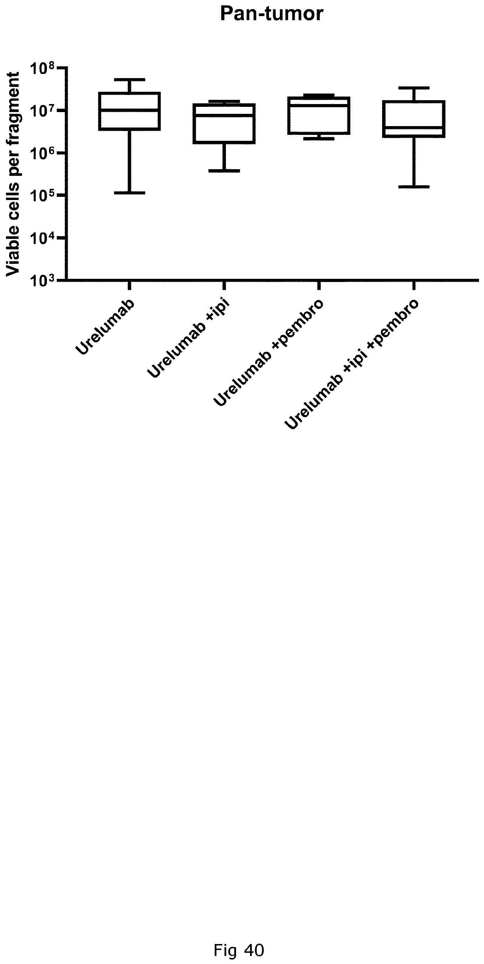

Figure 39 shows that TIL expansion increased significantly when adding TME stimulators from group A (PD-1 inhibitor or its ligands), group B (CTLA-4 inhibitor or its ligand), or when adding both group A and B as compared to the standard young TIL manufacturing protocol. There is a tendency that co-adding TME stimulators from group A and B further improved TIL growth rates. Figure 40 shows that a 4-1 BB agonist and an anti-CD3, urelumab/OKT3 (group J) either alone, or in combination with a CTLA-4 inhibitor, ipilimumab (group B), or in combination with a PD-1 inhibitor, pembrolizumab (group A), or in a triple combination of both ipilimumab and pembrolizumab all showed a very strong TIL growth in viable cells per tumor fragment from various cancers. Thus, one embodiment of the present invention relates to the use of one or more TME stimulators from group A for the methods of the present invention. One embodiment of the present invention relates to the use of one or more TME stimulators from group B for the methods of the present invention. One embodiment of the present invention relates to the use of one or more TME stimulators from group J for the methods of the present invention. A further embodiment of the present invention relates to a combination where one or more substances from group A and B are used together as TME stimulators. A further embodiment of the present invention relates to a combination where one or more substances from group A, B and J are used together as TME stimulators.

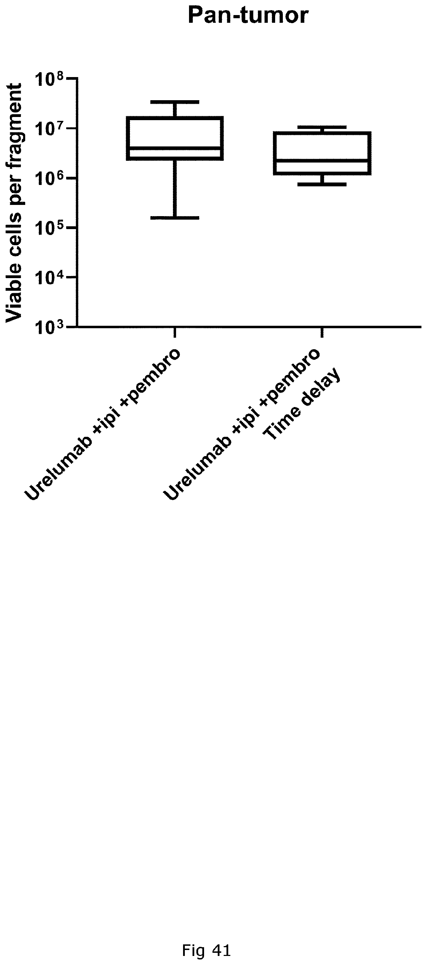

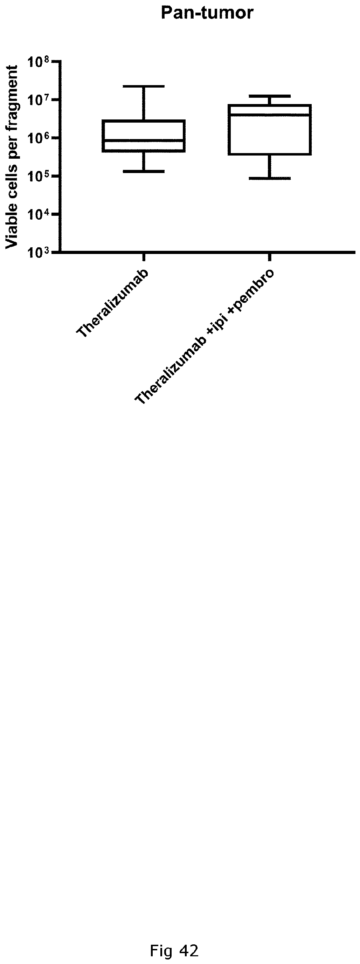

In figure 41 the effect of a time-delay for adding urelumab/OKT3 to the manufacturing process of young TIL culture was investigated. Here, the triple combination of urelumab/OKT3, ipilimumab and pembrolizumab added to the initial young TIL culture on day zero (left side of the figure) was compared to adding ipilimumab and pembrolizumab and waiting 2 days to add urelumab/OKT3 in a time-delay (right side of the figure). There was no significant difference between the two conditions and both conditions showed very strong TIL growth. In Figure 42, the effect of adding theralizumab alone (left side of the figure) or in combination with both ipilimumab and pembrolizumab to the initial young TIL culture was investigated. Although not significant, there was a tendency that the triple combination induced a faster growth rate in young TILs as compared to theralizumab alone.

Example 12 illustrates that the success rate of TIL expansion ex vivo was increased, when TME stimulators alone or in combinations were added to the culture medium when TIL cultures were initiated. Thus, one embodiment of the present invention relates to the use of TME stimulators in the methods according to the present invention resulting in increased ex vivo expansion relative to without the use of one or more TME stimulators. The methods of the present invention are ex vivo and are not performed on or in the body. They represent expansion of patient cells in a laboratory which therefore does not require a medical doctor in the production.

Example 13 illustrates that adding TME stimulators to the young TIL processing step provided a novel improvement over the existing standard TIL manufacturing protocol that allowed for generation of a TIL product containing an increased frequency of T cells and, an increased number of viable T cells. One embodiment of the present invention relates to the use of TME stimulators in the methods according to the present invention resulting in the generation of a TIL product containing an increased frequency of T cells and an increased number of viable T cells relative to without the use of one or more TME stimulators.

Example 14 illustrates that adding TME stimulators to the young TIL manufacturing step provided a novel improvement over the existing standard TIL protocol that allowed for generation of a TIL product containing a comparable frequency of effector memory T cells. One embodiment of the present invention relates to the use of TME stimulators in the methods according to the present invention resulting in the generation of a TIL product containing a comparable frequency of effector memory T cells relative to without the use of one or more TME stimulators.

Example 15 illustrates that adding TME stimulators alone and in combinations to the young TIL manufaturing step provided a novel improvement over the existing standard TIL protocol that allowed for generation of a TIL product containing an increased frequency of CD8+ T cells. Thus, one embodiment of the present invention relates to the use of TME stimulators in the methods according to the present invention resulting in the generation of a TIL product with an increased frequency of CD8+ T cells relative to without the use of one or more TME stimulators.

Example 16 illustrates that adding TME stimulators to the young TIL manufacturing step provided a novel improvement over the existing standard TIL protocol that allowed for generation of a TIL product containing a reduced frequency of CD4+ T cells. One embodiment of the present invention relates to the use of TME stimulators in the methods according to the present invention resulting in the generation of a TIL product containing a reduced frequency of CD4+ T cells relative to without the use of one or more TME stimulators.

Example 17 illustrates that adding TME stimulators to the young TIL manufacturing step provided a novel improvement over the existing standard TIL protocol that allowed for generation of a TIL product containing a reduced frequency of NK cells. Thus, one embodiment of the present invention relates to the use of TME stimulators in the methods according to the present invention resulting in the generation of a TIL product containing a reduced frequency of NK cells relative to without the use of one or more TME stimulators.

Example 18 illustrates that adding TME stimulators to the young TIL processing step provided a novel improvement over the existing standard TIL manufacturing protocol that allowed for generation of a TIL product containing a reduced frequency of NK cells but an increased frequency of CD8+ T cells. Thus, one embodiment of the present invention relates to the use of TME stimulators in the methods according to the present invention resulting in the generation of a TIL product containing a reduced frequency of NK cells but an increased frequency of CD8+ T cells relative to without the use of one or more TME stimulators.

Example 19 illustrates that adding TME stimulators with a time delay to the young TIL processing step provided a novel improvement over the existing standard TIL manufacturing protocol that allowed for generation of a TIL product containing an increased frequency of T cells in total, CD8+ T cells and a reduced frequency of NK cells and CD4+ T cells. One embodiment of the present invention relates to the use of TME stimulators in the methods according to the present invention resulting in the generation of a TIL product containing an increased frequency of T cells in total, CD8+ T cells and a reduced frequency of NK cells and CD4+ T cells relative to without the use of one or more TME stimulators.

Example 20 illustrates that adding TME stimulators to the young TIL manufacuring step provided a novel improvement over the existing standard TIL protocol that allowed for generation of a TIL product containing an increased frequency of tumor-specific LAG-3+ T cells. As LAG-3 is known to be a marker for T-cell exhaustion and that T cells that have a higher affinity to tumor antigens generally have an increased tendency to get exhausted, expansion of CD8+ LAG-3+ T cell clones can lead to a higher proportion of tumor-reactive T-cells possibly leading to an improved clinical outcome of this novel approach to TIL therapy. Thus, one embodiment of the present invention relates to the use of TME stimulators in the methods according to the present invention resulting in increased frequency of tumor-specific LAG-3+ T cells relative to without the use of one or more TME stimulators.

Example 21 illustrates that adding TME stimulators to the young TIL manufacturing step provided a novel improvement over the existing standard TIL protocol that allowed for generation of a TIL product containing an increased frequency of CD8+ T cells with a younger phenotype expressing CD28. One embodiment of the present invention relates to the use of TME stimulators in the methods according to the present invention resulting in increased frequency of CD8+ T cells with a younger phenotype expressing CD28 relative to without the use of one or more TME stimulators.

Using the approaches presented herein allows for dose levels that are much higher than would be tolerated in vivo. The concentrations can therefore be at least twice as high as the maximum allowed dose tolerated in vivo. The concentration can be even higher such as 5-10 as high as the maximum allowed dose tolerated in vivo. Thus, in one or more embodiments, the concentration of substance in is 0.1 µg/mL to 300 µg/mL. The concentration can also be 1 µg/mL to 100 µg/mL. The concentration can also be 10 µg/mL to 100 µg/mL. The concentration can also be 1 µg/mL to 10 µg/mL.

In one or more embodiments, the therapeutic population of T cells is used to treat a cancer type selected from the groups consisting of: 1: solid tumors, 2: ICI naïve tumors, 3: MSI-H tumors, 4: Hematological tumors, 5: Hyper-mutated tumors (such as POL-E and POL-D mutated tumors), and 6: virus-induced tumors.

In one or more embodiments, the therapeutic population of T cells is used to treat a cancer type selected from the groups consisting of breast cancer, renal cell cancer, bladder cancer, melanoma, cervical cancer, gastric cancer, colorectal cancer, lung cancer, head and neck cancer, ovarian cancer, Hodgkin lymphoma, pancreatic cancer, liver cancer, and sarcomas.

In one or more embodiments, the therapeutic population of T cells is used to treat a breast cancer. In one or more embodiments, the therapeutic population of T cells is used to treat renal cell cancer. In one or more embodiments, the therapeutic population of T cells is used to treat bladder cancer. In one or more embodiments, the therapeutic population of T cells is used to treat melanoma. In one or more embodiments, the therapeutic population of T cells is used to treat cervical cancer. In one or more embodiments, the therapeutic population of T cells is used to treat gastric cancer. In one or more embodiments, the therapeutic population of T cells is used to treat colorectal cancer. In one or more embodiments, the therapeutic population of T cells is used to treat lung cancer. In one or more embodiments, the therapeutic population of T cells is used to treat head and neck cancer. In one or more embodiments, the therapeutic population of T cells is used to treat ovarian cancer. In one or more embodiments, the therapeutic population of T cells is used to treat Hodgkin lymphoma. In one or more embodiments, the therapeutic population of T cells is used to treat pancreatic cancer. In one or more embodiments, the therapeutic population of T cells is used to treat liver cancer. In one or more embodiments, the therapeutic population of T cells is used to treat sarcomas.

In one or more embodiments, steps (a) through (c) or (d) are performed within a period of about 20 days to about 45 days. In one or more embodiments, steps (a) through (c) or (d) are performed within a period of about 20 days to about 40 days. In one or more embodiments, steps (a) through (c) or (d) are performed within a period of about 25 days to about 40 days. In one or more embodiments, steps (a) through (c) or (d) are performed within a period of about 30 days to about 40 days. In one or more embodiments, steps (a) through (b) are performed within a period of about 10 days to about 28 days. In one or more embodiments, steps (a) through (b) are performed within a period of about 10 days to about 20 days.

In some embodiments, the first TIL expansion (step (a)) can proceed for 1 day, 2 days, 3 days, 4 days, 5 days, 6 days, 7 days, 8 days, 9 days, 10 days, 11 days, 12 days, 13 days, or 14 days. In some embodiments, the first TIL expansion can proceed for 1 day to 14 days. In some embodiments, the first TIL expansion can proceed for 2 days to 14 days. In some embodiments, the first TIL expansion can proceed for 3 days to 14 days. In some embodiments, the first TIL expansion can proceed for 4 days to 14 days. In some embodiments, the first TIL expansion can proceed for 5 days to 14 days. In some embodiments, the first TIL expansion can proceed for 6 days to 14 days. In some embodiments, the first TIL expansion can proceed for 7 days to 14 days. In some embodiments, the first TIL expansion can proceed for 8 days to 14 days. In some embodiments, the first TIL expansion can proceed for 9 days to 14 days. In some embodiments, the first TIL expansion can proceed for 10 days to 14 days. In some embodiments, the first TIL expansion can proceed for 11 days to 14 days. In some embodiments, the first TIL expansion can proceed for 12 days to 14 days. In some embodiments, the first TIL expansion can proceed for 13 days to 14 days. In some embodiments, the first TIL expansion can proceed for 14 days. In some embodiments, the first TIL expansion can proceed for 1 day to 11 days. In some embodiments, the first TIL expansion can proceed for 2 days to 11 days. In some embodiments, the first TIL expansion can proceed for 3 days to 11 days. In some embodiments, the first TIL expansion can proceed for 4 days to 11 days. In some embodiments, the first TIL expansion can proceed for 5 days to 11 days. In some embodiments, the first TIL expansion can proceed for 6 days to 11 days. In some embodiments, the first TIL expansion can proceed for 7 days to 11 days. In some embodiments, the first TIL expansion can proceed for 8 days to 11 days. In some embodiments, the first TIL expansion can proceed for 9 days to 11 days. In some embodiments, the first TIL expansion can proceed for 10 days to 11 days. In some embodiments, the first TIL expansion can proceed for 11 days.

In one or more embodiments, step (b) is performed within a period of about 6 days to about 18 days. In one or more embodiments, step (b) is performed within a period of about 7 days to about 14 days. In one or more embodiments, step (b) is performed within a period of about 7 days to about 10 days. In one or more embodiments, step (b) is performed within a period of about 6 days to about 12 days.

In one or more embodiments, step (c) is performed within a period of about 12 days to about 18 days. In one or more embodiments, step (c) is performed within a period of about 10 days to about 28 days. In one or more embodiments, step (c) is performed within a period of about 10 days to about 20 days. In one or more embodiments, step (c) is performed within a period of about 12 days to about 18 days.

In some embodiments, the transition from the first expansion to the second expansion occurs at 1 day, 2 days, 3 days, 4 days, 5 days, 6 days, 7 days, 8 days, 9 days, 10 days, 11 days, 12 days, 13 days, or 14 days from when fragmentation occurs. In some embodiments, the transition from the first expansion to the second expansion occurs 1 day to 14 days from when fragmentation occurs. In some embodiments, the first TIL expansion can proceed for 2 days to 14 days. In some embodiments, the transition from the first expansion to the second expansion occurs 3 days to 14 days from when fragmentation occurs. In some embodiments, the transition from the first expansion to the second expansion occurs 4 days to 14 days from when fragmentation occurs. In some embodiments, the transition from the first expansion to the second expansion occurs 5 days to 14 days from when fragmentation occurs. In some embodiments, the transition from the first expansion to the second expansion occurs 6 days to 14 days from when fragmentation occurs. In some embodiments, the transition from the first expansion to the second expansion occurs 7 days to 14 days from when fragmentation occurs. In some embodiments, the transition from the first expansion to the second expansion occurs 8 days to 14 days from when fragmentation occurs. In some embodiments, the transition from the first expansion to the second expansion occurs 9 days to 14 days from when fragmentation occurs. In some embodiments, the transition from the first expansion to the second expansion occurs 10 days to 14 days from when fragmentation occurs. In some embodiments, the transition from the first expansion to the second expansion occurs 11 days to 14 days from when fragmentation occurs. In some embodiments, the transition from the first expansion to the second expansion occurs 12 days to 14 days from when fragmentation occurs. In some embodiments, the transition from the first expansion to the second expansion occurs 13 days to 14 days from when fragmentation occurs. In some embodiments, the transition from the first expansion to the second expansion occurs 14 days from when fragmentation occurs. In some embodiments, the transition from the first expansion to the second expansion occurs 1 day to 11 days from when fragmentation occurs. In some embodiments, the transition from the first expansion to the second expansion occurs 2 days to 11 days from when fragmentation occurs. In some embodiments, the transition from the first expansion to the second expansion occurs 3 days to 11 days from when fragmentation occurs. In some embodiments, the transition from the first expansion to the second expansion occurs 4 days to 11 days from when fragmentation occurs. In some embodiments, the transition from the first expansion to the second expansion occurs 5 days to 11 days from when fragmentation occurs. In some embodiments, the transition from the first expansion to the second expansion occurs 6 days to 11 days from when fragmentation occurs. In some embodiments, the transition from the first expansion to the second expansion occurs 7 days to 11 days from when fragmentation occurs. In some embodiments, the transition from the first expansion to the second expansion occurs 8 days to 11 days from when fragmentation occurs. In some embodiments, the transition from the first expansion to the second expansion occurs 9 days to 11 days from when fragmentation occurs. In some embodiments, the transition from the first expansion to the second expansion occurs 10 days to 11 days from when fragmentation occurs. In some embodiments, the transition from the first expansion to the second expansion occurs 11 days from when fragmentation occurs.

One of the key findings has been that more TILs can be reached faster. This has high value because there is a certain amount of cells that are needed in order to be relevant for medical treatment. More cells faster will drive down the costs for production and also provide treatment to the patient faster. In one or more embodiments, step (b) results in 1 × 10⁶ to 1× 10⁷ cells, such as 2 × 10⁶ to 5× 10⁶ cells. In one or more embodiments, step (b) results in 5 × 10⁶ to 1× 10⁷ cells. In one or more embodiments, step (b) results in 1 × 10⁶ to 5× 10⁷ cells. In one or more embodiments, step (b) results in 1 × 10⁷ to 5× 10⁷ cells. In one or more embodiments, step (c) results in 1 × 10⁷ to 1x 10¹² cells, such as 1 × 10⁸ to 5× 10⁹ cells, such as 1 × 10⁹ to 5× 10⁹ cells, such as 1 × 10⁸ to 5x 10¹⁰ cells, such as 1 × 10⁹ to 5× 10¹¹ cells. In one or more embodiments, step (c) results in an at least 10⁴ fold increase as compared to the number of cells after the expansion in step (b), such as at least 10³ fold increase, such as at least 10² fold increase, such as at least 10 fold increase. In one or more embodiments, step (c) results in 1 × 10⁷ to 1x 10¹⁰ cells. In one or more embodiments, step (c) results in 1 × 10⁷ to 1× 10⁹ cells. In one or more embodiments, step (c) results in 1 × 10⁷ to 1× 10⁸ cells.

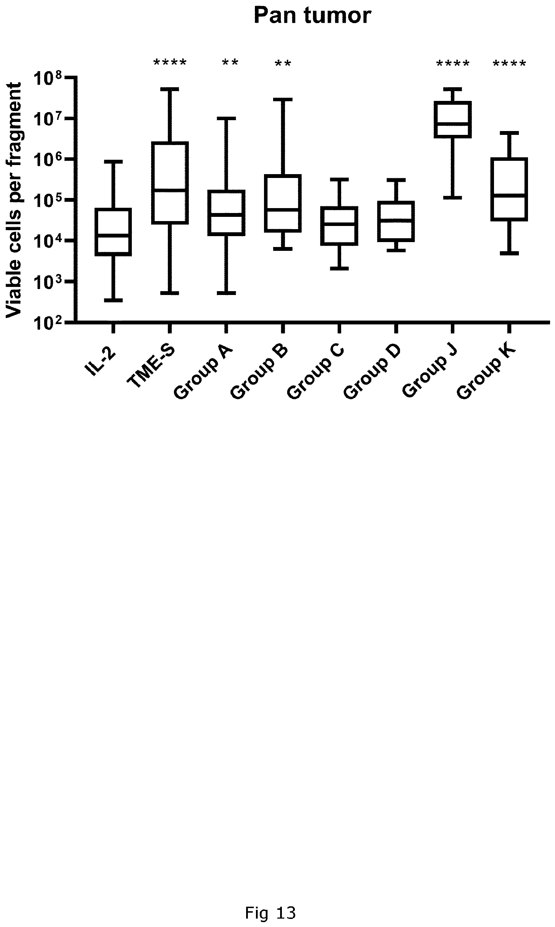

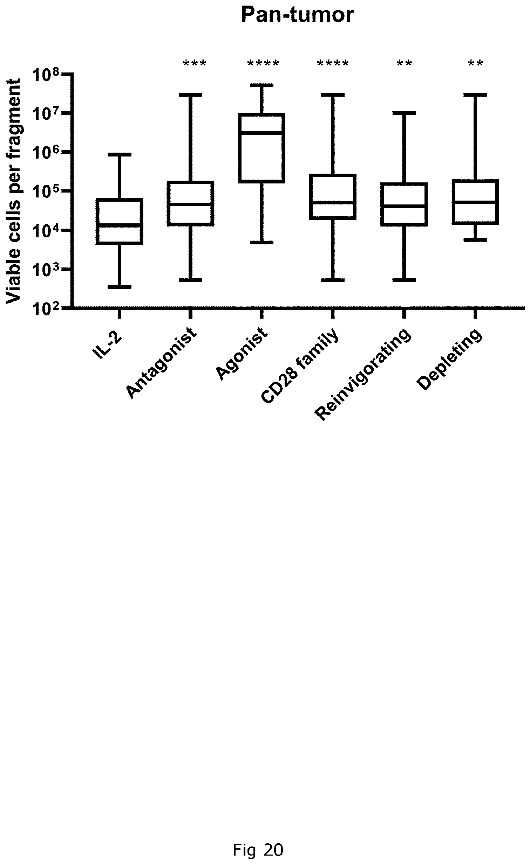

Example 7 illustrated in figure 13 demonstrated that adding TME-stimulators to the standard young TIL manufacturing protocol performed as described in example 2 significantly enhanced TIL growth which resulted in higher numbers of viable cells per tumor fragment. Example 8 illustrated in figure 20 demonstrate that adding TME stimulators that were either antagonizing receptors expressed on T-cells (or their ligands), agonizing receptors expressed on T-cells (or their ligands), reinvigorating exhausted T-cells (or their ligands), depleting regulatory T-cells and/or targeting receptors expressed on T-cells originating from the CD28 family (or their ligands originating from the B7 family of proteins) to the young TIL processing step provided a novel improvement over the existing standard TIL protocol that allowed for a faster TIL therapy manufacturing protocol.In some embodiments, the antigen-presenting feeder cells (APCs) are PBMCs. In some embodiments, the antigen-presenting feeder cells (APCs) are allogeneic feeder cells. In some embodiments, the antigen-presenting feeder cells are artificial antigen-presenting feeder cells. In an embodiment, the ratio of TILs to antigen-presenting feeder cells in the second expansion is about 1 to 25, about 1 to 50, about 1 to 100, about 1 to 125, about 1 to 150, about 1 to 175, about 1 to 200, about 1 to 225, about 1 to 250, about 1 to 275, about 1 to 300, about 1 to 325, about 1 to 350, about 1 to 375, about 1 to 400, or about 1 to 500. In an embodiment, the ratio of TILs to antigen-presenting feeder cells in the second expansion is between 1 to 50 and 1 to 300. In an embodiment, the ratio of TILs to antigen-presenting feeder cells in the second expansion is between 1 to 100 and 1 to 200. In one or more embodiments, the APCs are artificial APCs (aAPCs).

In an embodiment, TILs expanded using APCs of the present disclosure are administered to a patient as a pharmaceutical composition. In an embodiment, the pharmaceutical composition is a suspension of TILs in a sterile buffer. TILs expanded using PBMCs of the present disclosure may be administered by any suitable route as known in the art. In some embodiments, the T-cells are administered as a single intra-arterial or intravenous infusion, which preferably lasts approximately 30 to 60 minutes. Other suitable routes of administration include intraperitoneal, intrathecal, and intralymphatic. In one or more embodiments, the therapeutic population of TILs are infused into a patient.

In one or more embodiments, the cells are removed from the cell culture and cryopreserved in a storage medium prior to performing step (c).

In one or more embodiments, the method further comprises the step of transducing the first population of TILs with an expression vector comprising a nucleic acid encoding a chimeric antigen receptor (CAR) comprising a single chain variable fragment antibody fused with at least one endodomain of a T-cell signaling molecule.

In one or more embodiments, step (c) further comprises a step of removing the cells from the cell culture medium.

In one or more embodiments, step (a) further comprises processing of the resected tumor into multiple tumor fragments, such as 4 to 50 fragments, such as 20 to 30 fragments. In one or more embodiments, the fragments have a size of about 1 to 50 mm³. In one or more embodiments, the fragments have a size of about 5 to 50 mm³. In one or more embodiments, the fragments have a size of about 0.1 to 10 mm³. In one or more embodiments, the fragments have a size of about 0.1 to 1 mm³. In one or more embodiments, the fragments have a size of about 0.5 to 5 mm³. In one or more embodiments, the fragments have a size of about 1 to 10 mm³. In one or more embodiments, the fragments have a size of about 1 to 3 mm³.The terms "fragmenting", "fragment," and "fragmented", as used herein to describe processes for disrupting a tumor, includes mechanical fragmentation methods such as crushing, slicing, dividing, and morcellating tumor tissue as well as any other method for disrupting the physical structure of tumor tissue.

In one or more embodiments, the mammal is a human. In some embodiments, the TILs are obtained from tumor fragments. In some embodiments, the tumor fragment is obtained by sharp dissection. In some embodiments, the tumor fragment is between about 0.1 mm³ and 10 mm³. In some embodiments, the tumor fragment is between about 1 mm³ and 10 mm³. In some embodiments, the tumor fragment is between about 1 mm³ and 8 mm³. In some embodiments, the tumor fragment is about 1 mm³. In some embodiments, the tumor fragment is about 2 mm³. In some embodiments, the tumor fragment is about 3 mm³. In some embodiments, the tumor fragment is about 4 mm³. In some embodiments, the tumor fragment is about 5 mm³. In some embodiments, the tumor fragment is about 6 mm³. In some embodiments, the tumor fragment is about 7 mm³. In some embodiments, the tumor fragment is about 8 mm³. In some embodiments, the tumor fragment is about 9 mm³. In some embodiments, the tumor fragment is about 10 mm³. In some embodiments, the tumors are 1-4 mm × 1-4 mm × 1-4 mm. In some embodiments, the tumors are 1 mm × 1 mm × 1 mm. In some embodiments, the tumors are 2 mm × 2 mm × 2 mm. In some embodiments, the tumors are 3 mm × 3 mm × 3 mm. In some embodiments, the tumors are 4 mm × 4 mm × 4 mm. Currently fairly large fragment sizes are needed (more than 5 mm³). The present invention allows for the use of smaller fragments because the cells grow in a more optimized way reaching the cell count needed for treatment faster. The use of smaller fragments means that patients that until now have not been treatable because e.g. because their tumor has been too small or because it only has been possible to obtain a small tumor sample, now can be treated. The size of the fragments used in the methods of the present invention can therefore be important.

In some embodiments, the tumor fragmentation is performed in order to maintain the tumor internal structure. In some embodiments, the tumor fragmentation is performed without preforming a sawing motion with a scapel. In some embodiments, the TILs are obtained from tumor digests. In some embodiments, tumor digests were generated by incubation in enzyme media, for example but not limited to RPMI 1640, 2 mM GlutaMAX,10 mg/mL gentamicin, 30 U/mL DNase, and 1.0 mg/mL collagenase, followed by mechanical dissociation (GentleMACS, Miltenyi Biotec, Auburn, CA). After placing the tumor in enzyme media, the tumor can be mechanically dissociated for approximately 1 minute. The solution can then be incubated for 30 minutes at 37 °C in 5% CO₂ and it then mechanically disrupted again for approximately 1 minute. After being incubated again for 30 minutes at 37 °C in 5% CO₂, the tumor can be mechanically disrupted a third time for approximately 1 minute. In some embodiments, after the third mechanical disruption if large pieces of tissue were present, 1 or 2 additional mechanical dissociations were applied to the sample, with or without 30 additional minutes of incubation at 37 °C in 5% CO₂. In some embodiments, at the end of the final incubation if the cell suspension contained a large number of red blood cells or dead cells, a density gradient separation using Ficoll can be performed to remove these cells.

In one or more embodiments, the cell culture medium is provided in a container selected from the group consisting of a G-Rex container and a Xuri cellbag.

An aspect relates to a population of tumor infiltrating lymphocytes (TILs) obtainable by a method of any of the previous claims.

A further aspect relates to expanded tumor infiltrating lymphocytes (TILs) for use in treating a subject with cancer, the treatment comprising the steps of: culturing autologous T cells by obtaining a first population of TILs from a tumor resected from a mammal performing a first expansion by culturing the first population of TILs in a cell culture medium comprising IL-2 and one or more TME stimulators to produce a second population of TILs, wherein the one or more TME stimulators comprise at least a substance of group B is selected from one or more from the group consisting of ipilimumab and tremelimumab; performing a second expansion by supplementing the cell culture medium of the second population of TILs with additional IL-2, anti-CD3, and antigen presenting cells (APCs), to produce a third population of TILs, wherein the third population of TILs is a therapeutic population; and after administering nonmyeloablative lymphodepleting chemotherapy, administering to the mammal the therapeutic population of T cells, wherein the T cells administered to the mammal, whereupon the regression of the cancer in the mammal is promoted.

In an embodiment, the non-myeloablative chemotherapy is cyclophosphamide 60 mg/kg/d for 2 days (days 7 and 2 prior to TIL infusion) and fludarabine 25 mg/m2/d for 5 days (days 5 to 1 prior to TIL infusion). In an embodiment, after non-myeloablative chemotherapy and TIL infusion (at day 0) according to the present disclosure, the patient receives an intravenous infusion of IL-2 intravenously at 720,000 IU/kg every 8 hours to physiologic tolerance.

In some embodiments, the present disclosure a population of tumor infiltrating lymphocytes (TILs) for use in treating cancer, wherein the population of TILs are obtainable by a method comprising the steps of (b) performing an initial expansion of a first population of TILs obtained from a tumor resected from a patient in a first cell culture medium to obtain a second population of TILs, wherein the second population of TILs is at least 5-fold greater in number than the first population of TILs, and wherein the first cell culture medium comprises IL-2; (c) performing a rapid expansion of the second population of TILs using a population of myeloid artificial antigen presenting cells (myeloid aAPCs) in a second cell culture medium to obtain a third population of TILs, wherein the third population of TILs is at least 50-fold greater in number than the second population of TILs after 7 days from the start of the rapid expansion; and wherein the second cell culture medium comprises IL-2 and anti-CD3; (d) administering a therapeutically effective portion of the third population of TILs to a patient with the cancer. In some embodiments, the method comprises a first step (a) of obtaining the first population of TILs from a tumor resected from a patient. In some embodiments, the IL-2 is present at an initial concentration of about 3000 IU/mL and anti-CD3antibody is present at an initial concentration of about 30 ng/mL in the second cell culture medium. In some embodiments, first expansion is performed over a period not greater than 14 days. In some embodiments, the first expansion is performed using a gas permeable container. In some embodiments, the second expansion is performed using a gas permeable container. In some embodiments, the ratio of the second population of TILs to the population of aAPCs in the rapid expansion is between 1 to 80 and 1 to 400. In some embodiments, the ratio of the second population of TILs to the population of aAPCs in the rapid expansion is about 1 to 300.

The following figures and examples are provided below to illustrate the present invention. They are intended to be illustrative and are not to be construed as limiting in any way.
Tables

**Table 1**

| ***Group*** | ***Receptor on T-cell*** | ***Ligand*** | |
|---|---|---|---|
| A | PD-1 | PD-L1/PD-L2 | antagonist |
| B | CTLA-4 | CD80/CD86 | antagonist |
| C | LAG-3 | MHC I/II LAG-3 binding site(s) | antagonist |
| D | TIGIT/CD226 | CD155/CD112 | antagonist |
| E | KIR | MHC I KIR binding site(s) | antagonist |
| F | TIM-3 | Galectin 9 | antagonist |
| G | BTLA | HVEM | antagonist |
| H | A2aR | Adenosine | antagonist |

| ***Group*** | ***Receptor on T-cell*** | ***Ligand*** | |
|---|---|---|---|
| I | OX40/CD134 | OX40L | agonist |
| J | 4-1BB/CD137 | 4-1BBL | agonist |
| K | CD28 | CD80/CD86 | agonist |
| L | ICOS | ICOSL/B7RP1 | agonist |
| M | GITR | GITRL | agonist |
| N | CD40L | CD40 | agonist |
| O | CD27 | CD70 | agonist |

| ***Group*** | ***Soluble factor*/*cytokine*** | ***Receptor*** | |
|---|---|---|---|
| P | IDO1/2 | | antagonist |
| Q | TGFβ | TGFβ receptor type I/II/III | antagonist |
| R | IL-10 | IL10Rα | antagonist |
| S | IL-35 | IL-35R | antagonist |

| ***Group*** | ***Soluble factor*/*cytokine*** | | |
|---|---|---|---|
| T | cyclophosphamide | | |
| U | TKIs | | |
| V | αCD25 | | |
| X | IL2/Diphteria toxin fusion | | |

**Table 2**

| ***Single substance:*** | |
|---|---|
| IL2 | A |
| IL2 | B |
| IL2 | C |
| IL2 | D |
| IL2 | E |
| IL2 | F |
| IL2 | G |
| IL2 | H |
| IL2 | I |
| IL2 | J |
| IL2 | K |
| IL2 | L |
| IL2 | M |
| IL2 | N |
| IL2 | O |
| IL2 | P |
| IL2 | Q |
| IL2 | R |
| IL2 | S |
| IL2 | T |
| IL2 | U |
| IL2 | V |
| IL2 | X |

**Table 3**

| ***PD1 group dual substance:*** | | |
|---|---|---|
| IL2 | A | B |
| IL2 | A | C |
| IL2 | A | D |
| IL2 | A | E |
| IL2 | A | F |
| IL2 | A | G |
| IL2 | A | H |
| IL2 | A | I |
| IL2 | A | J |
| IL2 | A | K |
| IL2 | A | L |
| IL2 | A | M |
| IL2 | A | N |
| IL2 | A | O |
| IL2 | A | P |
| IL2 | A | Q |
| IL2 | A | R |
| IL2 | A | S |
| IL2 | A | T |
| IL2 | A | U |
| IL2 | A | V |
| IL2 | A | X |

**Table 4**

| ***PD1*/*CTLA-4 group triple substance:*** | | | |
|---|---|---|---|
| IL2 | A | B | C |
| IL2 | A | B | D |
| IL2 | A | B | E |
| IL2 | A | B | F |
| IL2 | A | B | G |
| IL2 | A | B | H |
| IL2 | A | B | I |
| IL2 | A | B | J |
| IL2 | A | B | K |
| IL2 | A | B | L |
| IL2 | A | B | M |
| IL2 | A | B | N |
| IL2 | A | B | O |
| IL2 | A | B | P |
| IL2 | A | B | Q |
| IL2 | A | B | R |
| IL2 | A | B | S |
| IL2 | A | B | T |
| IL2 | A | B | U |
| IL2 | A | B | V |
| IL2 | A | B | X |

**Table 5**

| ***PD1*/*LAG-3 group triple substance:*** | | | |
|---|---|---|---|
| IL2 | A | C | D |
| IL2 | A | C | E |
| IL2 | A | C | F |
| IL2 | A | C | G |
| IL2 | A | C | H |
| IL2 | A | C | I |
| IL2 | A | C | J |
| IL2 | A | C | K |
| IL2 | A | C | L |
| IL2 | A | C | M |
| IL2 | A | C | N |
| IL2 | A | C | O |
| IL2 | A | C | P |
| IL2 | A | C | Q |
| IL2 | A | C | R |
| IL2 | A | C | S |
| IL2 | A | C | T |
| IL2 | A | C | U |
| IL2 | A | C | V |
| IL2 | A | C | X |

**Table 6**

| ***PD1*/*TIGIT group triple substance:*** | | | |
|---|---|---|---|
| IL2 | A | D | E |
| IL2 | A | D | F |
| IL2 | A | D | G |
| IL2 | A | D | H |
| IL2 | A | D | I |
| IL2 | A | D | J |
| IL2 | A | D | K |
| IL2 | A | D | L |
| IL2 | A | D | M |
| IL2 | A | D | N |
| IL2 | A | D | O |
| IL2 | A | D | P |
| IL2 | A | D | Q |
| IL2 | A | D | R |
| IL2 | A | D | S |
| IL2 | A | D | T |
| IL2 | A | D | U |
| IL2 | A | D | V |
| IL2 | A | D | X |

**Table 7**

| ***PD1*/*KIR group triple substance:*** | | | |
|---|---|---|---|
| IL2 | A | E | F |
| IL2 | A | E | G |
| IL2 | A | E | H |
| IL2 | A | E | I |
| IL2 | A | E | J |
| IL2 | A | E | K |
| IL2 | A | E | L |
| IL2 | A | E | M |
| IL2 | A | E | N |
| IL2 | A | E | O |
| IL2 | A | E | P |
| IL2 | A | E | Q |
| IL2 | A | E | R |
| IL2 | A | E | S |
| IL2 | A | E | T |
| IL2 | A | E | U |
| IL2 | A | E | V |
| IL2 | A | E | X |

**Table 8**

| ***PD1*/*TIM-3 group triple substance:*** | | | |
|---|---|---|---|
| IL2 | A | F | G |
| IL2 | A | F | H |
| IL2 | A | F | I |
| IL2 | A | F | J |
| IL2 | A | F | K |
| IL2 | A | F | L |
| IL2 | A | F | M |
| IL2 | A | F | N |
| IL2 | A | F | O |
| IL2 | A | F | P |
| IL2 | A | F | Q |
| IL2 | A | F | R |
| IL2 | A | F | S |
| IL2 | A | F | T |
| IL2 | A | F | U |
| IL2 | A | F | V |
| IL2 | A | F | X |

**Table 9**

| ***PD1*/*BTLA group triple substance:*** | | | |
|---|---|---|---|
| IL2 | A | G | H |
| IL2 | A | G | I |
| IL2 | A | G | J |
| IL2 | A | G | K |
| IL2 | A | G | L |
| IL2 | A | G | M |
| IL2 | A | G | N |
| IL2 | A | G | O |
| IL2 | A | G | P |
| IL2 | A | G | Q |
| IL2 | A | G | R |
| IL2 | A | G | S |
| IL2 | A | G | T |
| IL2 | A | G | U |
| IL2 | A | G | V |
| IL2 | A | G | X |

**Table 10**

| ***PD1*/*A2aR group triple substance:*** | | | |
|---|---|---|---|
| IL2 | A | H | I |
| IL2 | A | H | J |
| IL2 | A | H | K |
| IL2 | A | H | L |
| IL2 | A | H | M |
| IL2 | A | H | N |
| IL2 | A | H | O |
| IL2 | A | H | P |
| IL2 | A | H | Q |
| IL2 | A | H | R |
| IL2 | A | H | S |
| IL2 | A | H | T |
| IL2 | A | H | U |
| IL2 | A | H | V |
| IL2 | A | H | X |

**Table 11**

| ***PD1*/*OX40 group triple substance:*** | | | |
|---|---|---|---|
| IL2 | A | I | J |
| IL2 | A | I | K |
| IL2 | A | I | L |
| IL2 | A | I | M |
| IL2 | A | I | N |
| IL2 | A | I | O |
| IL2 | A | I | P |
| IL2 | A | I | Q |
| IL2 | A | I | R |
| IL2 | A | I | S |
| IL2 | A | I | T |
| IL2 | A | I | U |
| IL2 | A | I | V |
| IL2 | A | I | X |

**Table 12**

| ***PD1*/*4-1BB group triple substance:*** | | | |
|---|---|---|---|
| IL2 | A | J | K |
| IL2 | A | J | L |
| IL2 | A | J | M |
| IL2 | A | J | N |
| IL2 | A | J | O |
| IL2 | A | J | P |
| IL2 | A | J | Q |
| IL2 | A | J | R |
| IL2 | A | J | S |
| IL2 | A | J | T |
| IL2 | A | J | U |
| IL2 | A | J | V |
| IL2 | A | J | X |

**Table 13**

| ***PD1*/*CD28 group triple substance:*** | | | |
|---|---|---|---|
| IL2 | A | K | L |
| IL2 | A | K | M |
| IL2 | A | K | N |
| IL2 | A | K | O |
| IL2 | A | K | P |
| IL2 | A | K | Q |
| IL2 | A | K | R |
| IL2 | A | K | S |
| IL2 | A | K | T |
| IL2 | A | K | U |
| IL2 | A | K | V |
| IL2 | A | K | X |

**Table 14**

| ***PD1*/*ICOS group triple substance:*** | | | |
|---|---|---|---|
| IL2 | A | L | M |
| IL2 | A | L | N |
| IL2 | A | L | O |
| IL2 | A | L | P |
| IL2 | A | L | Q |
| IL2 | A | L | R |
| IL2 | A | L | S |
| IL2 | A | L | T |
| IL2 | A | L | U |
| IL2 | A | L | V |
| IL2 | A | L | X |

**Table 15**

| ***PD1*/*GITR group triple substance:*** | | | |
|---|---|---|---|
| IL2 | A | M | N |
| IL2 | A | M | O |
| IL2 | A | M | P |
| IL2 | A | M | Q |
| IL2 | A | M | R |
| IL2 | A | M | S |
| IL2 | A | M | T |
| IL2 | A | M | U |
| IL2 | A | M | V |
| IL2 | A | M | X |

**Table 16**

| ***PD1*/*CD40 group triple substance:*** | | | |
|---|---|---|---|
| IL2 | A | N | O |
| IL2 | A | N | P |
| IL2 | A | N | Q |
| IL2 | A | N | R |
| IL2 | A | N | S |
| IL2 | A | N | T |
| IL2 | A | N | U |
| IL2 | A | N | V |
| IL2 | A | N | X |

**Table 17**

| ***PD1*/*CD27 group triple substance:*** | | | |
|---|---|---|---|
| IL2 | A | O | P |
| IL2 | A | O | Q |
| IL2 | A | O | R |
| IL2 | A | O | S |
| IL2 | A | O | T |
| IL2 | A | O | U |
| IL2 | A | O | V |
| IL2 | A | O | X |

**Table 18**

| ***PD1*/*IDO group triple substance:*** | | | |
|---|---|---|---|
| IL2 | A | P | Q |
| IL2 | A | P | R |
| IL2 | A | P | S |
| IL2 | A | P | T |
| IL2 | A | P | U |
| IL2 | A | P | V |
| IL2 | A | P | X |

**Table 19**

| ***PD1*/*TGF*β *group triple substance:*** | | | |
|---|---|---|---|
| IL2 | A | Q | R |
| IL2 | A | P | S |
| IL2 | A | P | T |
| IL2 | A | P | U |
| IL2 | A | P | V |
| IL2 | A | P | X |

**Table 20**

| ***PD1*/*IL10 group triple agent:*** | | | |
|---|---|---|---|
| IL2 | A | R | S |
| IL2 | A | R | T |
| IL2 | A | R | U |
| IL2 | A | R | V |
| IL2 | A | R | X |

**Table 21**

| ***PD1*/*Adenosine group triple agent:*** | | | |
|---|---|---|---|
| IL2 | A | S | T |
| IL2 | A | S | U |
| IL2 | A | S | V |
| IL2 | A | S | X |

**Table 22**

| ***CTLA-4 group dual substance:*** | | |
|---|---|---|
| IL2 | B | C |
| IL2 | B | D |
| IL2 | B | E |
| IL2 | B | F |
| IL2 | B | G |
| IL2 | B | H |
| IL2 | B | I |
| IL2 | B | J |
| IL2 | B | K |
| IL2 | B | L |
| IL2 | B | M |
| IL2 | B | N |
| IL2 | B | O |
| IL2 | B | P |
| IL2 | B | Q |
| IL2 | B | R |
| IL2 | B | S |
| IL2 | B | T |
| IL2 | B | U |
| IL2 | B | V |
| IL2 | B | X |

**Table 23**

| ***CTLA-4*/*LAG-3 group triple substance:*** | | | |
|---|---|---|---|
| IL2 | B | C | D |
| IL2 | B | C | E |
| IL2 | B | C | F |
| IL2 | B | C | G |
| IL2 | B | C | H |
| IL2 | B | C | I |
| IL2 | B | C | J |
| IL2 | B | C | K |
| IL2 | B | C | L |
| IL2 | B | C | M |
| IL2 | B | C | N |
| IL2 | B | C | O |
| IL2 | B | C | P |
| IL2 | B | C | Q |
| IL2 | B | C | R |
| IL2 | B | C | S |
| IL2 | B | C | T |
| IL2 | B | C | U |
| IL2 | B | C | V |
| IL2 | B | C | X |

**Table 24**

| ***CTLA-4*/*TIGIT group triple substance:*** | | | |
|---|---|---|---|
| IL2 | B | D | E |
| IL2 | B | D | F |
| IL2 | B | D | G |
| IL2 | B | D | H |
| IL2 | B | D | I |
| IL2 | B | D | J |
| IL2 | B | D | K |
| IL2 | B | D | L |
| IL2 | B | D | M |
| IL2 | B | D | N |
| IL2 | B | D | O |
| IL2 | B | D | P |
| IL2 | B | D | Q |
| IL2 | B | D | R |
| IL2 | B | D | S |
| IL2 | B | D | T |
| IL2 | B | D | U |
| IL2 | B | D | V |
| IL2 | B | D | X |

**Table 25**

| ***CTLA-4*/*KIR group triple substance:*** | | | |
|---|---|---|---|
| IL2 | B | E | F |
| IL2 | B | E | G |
| IL2 | B | E | H |
| IL2 | B | E | I |
| IL2 | B | E | J |
| IL2 | B | E | K |
| IL2 | B | E | L |
| IL2 | B | E | M |
| IL2 | B | E | N |
| IL2 | B | E | O |
| IL2 | B | E | P |
| IL2 | B | E | Q |
| IL2 | B | E | R |
| IL2 | B | E | S |
| IL2 | B | E | T |
| IL2 | B | E | U |
| IL2 | B | E | V |
| IL2 | B | E | X |

**Table 26**

| ***CTLA-4*/*TIM-3 group triple substance:*** | | | |
|---|---|---|---|
| IL2 | B | F | G |
| IL2 | B | F | H |
| IL2 | B | F | I |
| IL2 | B | F | J |
| IL2 | B | F | K |
| IL2 | B | F | L |
| IL2 | B | F | M |
| IL2 | B | F | N |
| IL2 | B | F | O |
| IL2 | B | F | P |
| IL2 | B | F | Q |
| IL2 | B | F | R |
| IL2 | B | F | S |
| IL2 | B | F | T |
| IL2 | B | F | U |
| IL2 | B | F | V |
| IL2 | B | F | X |
| IL2 | B | F | Y |

**Table 27**

| ***CTLA-4*/*BTLA group triple substance:*** | | | |
|---|---|---|---|
| IL2 | B | G | H |
| IL2 | B | G | I |
| IL2 | B | G | J |
| IL2 | B | G | K |
| IL2 | B | G | L |
| IL2 | B | G | M |
| IL2 | B | G | N |
| IL2 | B | G | O |
| IL2 | B | G | P |
| IL2 | B | G | Q |
| IL2 | B | G | R |
| IL2 | B | G | S |
| IL2 | B | G | T |
| IL2 | B | G | U |
| IL2 | B | G | V |
| IL2 | B | G | X |
| IL2 | B | G | Y |

**Table 28**

| ***CTLA-4*/*A2aR group triple substance:*** | | | |
|---|---|---|---|
| IL2 | B | H | I |
| IL2 | B | H | J |
| IL2 | B | H | K |
| IL2 | B | H | L |
| IL2 | B | H | M |
| IL2 | B | H | N |
| IL2 | B | H | O |
| IL2 | B | H | P |
| IL2 | B | H | Q |
| IL2 | B | H | R |
| IL2 | B | H | S |
| IL2 | B | H | T |
| IL2 | B | H | U |
| IL2 | B | H | V |
| IL2 | B | H | X |
| IL2 | B | H | Y |

**Table 29**

| ***CTLA-4*/*OX40 group triple substance:*** | | | |
|---|---|---|---|
| IL2 | B | I | J |
| IL2 | B | I | K |
| IL2 | B | I | L |
| IL2 | B | I | M |
| IL2 | B | I | N |
| IL2 | B | I | O |
| IL2 | B | I | P |
| IL2 | B | I | Q |
| IL2 | B | I | R |
| IL2 | B | I | S |
| IL2 | B | I | T |
| IL2 | B | I | U |
| IL2 | B | I | V |
| IL2 | B | I | X |
| IL2 | B | I | Y |

**Table 30**

| ***CTLA-4*/*4-1BB group triple substance:*** | | | |
|---|---|---|---|
| IL2 | B | J | K |
| IL2 | B | J | L |
| IL2 | B | J | M |
| IL2 | B | J | N |
| IL2 | B | J | O |
| IL2 | B | J | P |
| IL2 | B | J | Q |
| IL2 | B | J | R |
| IL2 | B | J | S |
| IL2 | B | J | T |
| IL2 | B | J | U |
| IL2 | B | J | V |
| IL2 | B | J | X |
| IL2 | B | J | Y |

**Table 31**

| ***CTLA-4*/*CD28 group triple substance:*** | | | |
|---|---|---|---|
| IL2 | B | K | L |
| IL2 | B | K | M |
| IL2 | B | K | N |
| IL2 | B | K | O |
| IL2 | B | K | P |
| IL2 | B | K | Q |
| IL2 | B | K | R |
| IL2 | B | K | S |
| IL2 | B | K | T |
| IL2 | B | K | U |
| IL2 | B | K | V |
| IL2 | B | K | X |
| IL2 | B | K | Y |

**Table 32**

| ***CTLA-4*/*ICOS group triple substance:*** | | | |
|---|---|---|---|
| IL2 | B | L | M |
| IL2 | B | L | N |
| IL2 | B | L | O |
| IL2 | B | L | P |
| IL2 | B | L | Q |
| IL2 | B | L | R |
| IL2 | B | L | S |
| IL2 | B | L | T |
| IL2 | B | L | U |
| IL2 | B | L | V |
| IL2 | B | L | X |
| IL2 | B | L | Y |

**Table 33**

| ***CTLA-4*/*GITR group triple substance:*** | | | |
|---|---|---|---|
| IL2 | B | M | N |
| IL2 | B | M | O |
| IL2 | B | M | P |
| IL2 | B | M | Q |
| IL2 | B | M | R |
| IL2 | B | M | S |
| IL2 | B | M | T |
| IL2 | B | M | U |
| IL2 | B | M | V |
| IL2 | B | M | X |
| IL2 | B | M | Y |

**Table 34**

| ***CTLA-4*/*CD40 group triple substance:*** | | | |
|---|---|---|---|
| IL2 | B | N | O |
| IL2 | B | N | P |
| IL2 | B | N | Q |
| IL2 | B | N | R |
| IL2 | B | N | S |
| IL2 | B | N | T |
| IL2 | B | N | U |
| IL2 | B | N | V |
| IL2 | B | N | X |
| IL2 | B | N | Y |

**Table 35**

| ***CTLA-4*/*CD27 group triple substance:*** | | | |
|---|---|---|---|
| IL2 | B | O | P |
| IL2 | B | O | Q |
| IL2 | B | O | R |
| IL2 | B | O | S |
| IL2 | B | O | T |
| IL2 | B | O | U |
| IL2 | B | O | V |
| IL2 | B | O | X |
| IL2 | B | O | Y |

**Table 37**

| ***CTLA-4*/*IDO group triple substance:*** | | | |
|---|---|---|---|
| IL2 | B | P | Q |
| IL2 | B | P | R |
| IL2 | B | P | S |
| IL2 | B | P | T |
| IL2 | B | P | U |
| IL2 | B | P | V |
| IL2 | B | P | X |
| IL2 | B | P | Y |

**Table 38**

| ***CTLA-4*/*TGFβ group triple substance:*** | | | |
|---|---|---|---|
| IL2 | B | Q | R |
| IL2 | B | P | S |
| IL2 | B | P | T |
| IL2 | B | P | U |
| IL2 | B | P | V |
| IL2 | B | P | X |
| IL2 | B | P | Y |

**Table 39**

| ***CTLA-4*/*IL10 group triple substance:*** | | | |
|---|---|---|---|
| IL2 | B | R | S |
| IL2 | B | R | T |
| IL2 | B | R | U |
| IL2 | B | R | V |
| IL2 | B | R | X |
| IL2 | B | R | Y |

**Table 40**

| ***CTLA-4*/*Adenosine group triple agent:*** | | | |
|---|---|---|---|
| IL2 | B | S | T |
| IL2 | B | S | U |
| IL2 | B | S | V |
| IL2 | B | S | X |
| IL2 | B | S | Y |

**Table 41**

| ***CTLA-4*/*IL35 group triple agent:*** | | | |
|---|---|---|---|
| IL2 | B | T | U |
| IL2 | B | T | V |
| IL2 | B | T | X |
| IL2 | B | T | Y |

**Table 42**

| **Name** | **Diagnosis** | **Gender** | **Age** | **Ethnicity** | **Stage** | **Prior Treatment** |
|---|---|---|---|---|---|---|
| MM1 | Melanoma | Female | 76 | Caucasian | II | treatment naive |
| MM2 | Melanoma | Female | 48 | Caucasian | IIIB | treatment naive |
| MM3 | Melanoma | Male | 56 | Caucasian | IIB | treatment naive |
| HN1 | Head and neck cancer | Male | 64 | Caucasian | III | treatment naive |
| HN2 | Head and neck cancer | Male | 69 | Caucasian | III | treatment naive |
| HN3 | Head and neck cancer | Male | 67 | Caucasian | IV | treatment naive |
| CC1 | Colorectal Carcinoma | Female | 88 | Caucasian | IIA | treatment naive |
| CC2 | Colorectal Carcinoma | Male | 81 | Caucasian | I | treatment naive |
| OC1 | Ovarian carcinoma | Female | 56 | Caucasian | IIIC | treatment naive |
| OC2 | Ovarian carcinoma | Female | 52 | Caucasian | IV | treatment naive |
| OC3 | Ovarian carcinoma | Female | 54 | Caucasian | IIIA | treatment naive |
| LC1 | NSCLC | Male | 72 | Caucasian | IB | treatment naive |
| LC2 | NSCLC | Male | 62 | Caucasian | IB | treatment naive |
| CE1 | Cervical cancer | Female | 64 | Caucasian | IB | treatment naive |

**Table 43**

| | **Group** | **Stimulant** | **Target** | **Manufacturer** |
|---|---|---|---|---|
| TME-S | Group A | pembrolizumab | PD-1 | Merck |
| | | nivolumab | | Bristol-Meyers Squibb |
| | | avelumab | PD-L1 | Pfizer |
| | | durvalumab | | AstraZeneca |
| | Group B | ipilimumab | CTLA-4 | Bristol-Meyers Squibb |
| | Group C | relatlimab | LAG-3 | Creative Biolabs |
| | Group D | tiragolumab | TIGIT | Creative Biolabs |
| | Group J | urelumab/OKT3 | 4-1BB (CD137) and CD3 | Creative Biolabs/Miltenyi |
| | Group K | theralizumab | CD28 | Creative Biolabs |

**Table 44**

| **Group** | **Stimulant** | **Manufacturer** |
|---|---|---|
| **Antagonist** | pembrolizumab | Merck |
| | nivolumab | Bristol-Meyers Squibb |
| | avelumab | Pfizer |
| | durvalumab | AstraZeneca |
| | ipilimumab | Bristol-Meyers Squibb |
| | relatlimab | Creative Biolabs |
| | tiragolumab | Creative Biolabs |
| **Agonist** | urelumab/OKT3 | Creative Biolabs/Miltenyi |
| | theralizumab | Creative Biolabs |
| **CD28 family** | pembrolizumab | Merck |
| | nivolumab | Bristol-Meyers Squibb |
| | avelumab | Pfizer |
| | durvalumab | AstraZeneca |
| | ipilimumab | Bristol-Meyers Squibb |
| | theralizumab | Creative Biolabs |
| **Reinvigorating** | pembrolizumab | Merck |
| | nivolumab | Bristol-Meyers Squibb |
| | avelumab | Pfizer |
| | durvalumab | AstraZeneca |
| | relatlimab | Creative Biolabs |
| **Depleting** | ipilimumab | Bristol-Meyers Squibb |
| | tiragolumab | Creative Biolabs |

**Table 45**

| **Marker** | **clone** | **company** |
|---|---|---|
| **CD3** | UCHT1 | BD Biosciences |
| **CD4** | SK3 | BD Biosciences |
| **CD8** | RPA-T8 | BD Biosciences |
| **CD27** | L128 | BD Biosciences |
| **CD28** | CD28.2 | BD Biosciences |
| **CD45RA** | H1100 | BD Biosciences |
| **CD56** | B159 | BD Biosciences |
| **CD57** | NK-1 | BD Biosciences |
| **CD69** | FN50 | BD Biosciences |
| **BTLA** | J168-540 | BD Biosciences |
| **CCR7** | 2-L1-A | BD Biosciences |
| **LAG-3** | T47-530 | BD Biosciences |
| **PD-1** | MIH4 | BD Biosciences |
| **TIM-3** | 7D3 | BD Biosciences |
| **FVS780** | | BD Biosciences |

### BRIEF DESCRIPTION OF THE FIGURES

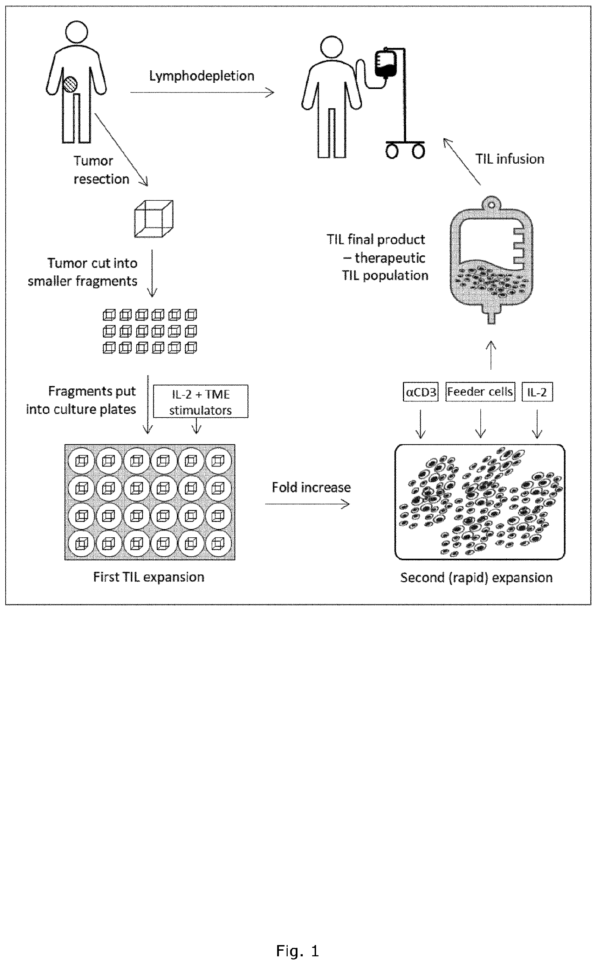

Figure 1 shows Tumor containing Tumor Infiltrating Lymphocytes (TILs) resected from a mammal is cut into smaller fragments and put into one or more multi-well cell culture plates. Here the fragments are incubated in cell culture medium containing interleukin 2 (IL-2) and tumor microenvironment (TME) stimulators during a first expansion in order to produce a second population of TILs. Hereafter, the second population of TILs is further expanded in a second (often called rapid) expansion in cell culture medium containing feeder cells, anti-CD3 antibody and IL-2. The second expansion can dependent on protocol and cell culture equipment be performed in one or more steps using on or more containers in further sub-steps but is for simplicity reasons only illustrated as one step in the figure. Lastly, the TILs are harvested to produce the third and therapeutic population of TILs and resuspended into the final TIL product, which is infused back to the mammal promoting regression of the cancer.

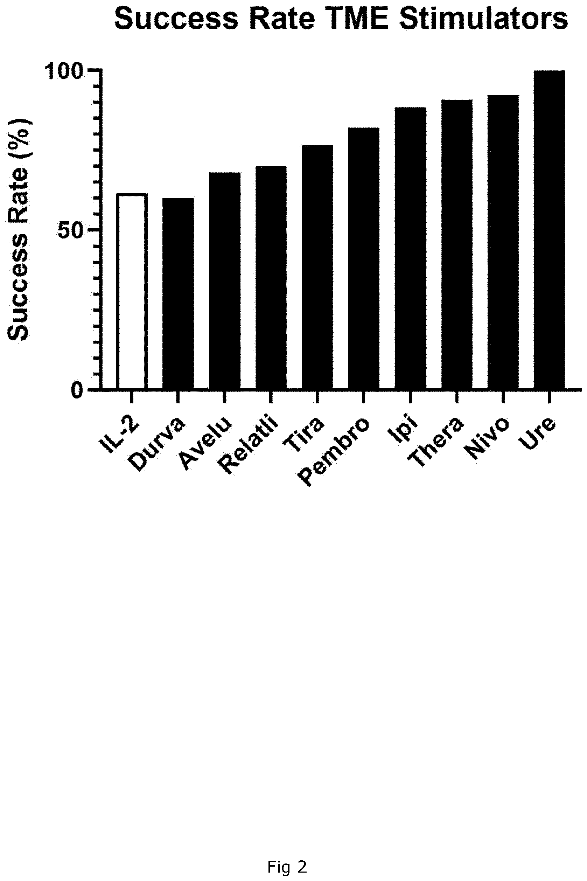

Figure 2 shows percentage of successful cell expansions (> 50,000 cells/fragment) using IL-2, durvalumab (Durva), avelumab (Avelu), relatlimab (Relatli), tiragolumab (Tira), Pembrolizumab (Pembro), ipilimumab (Ipi), theralizumab (Thera), nivolumab (Nivo), or urelumab/OKT3 (Ure).

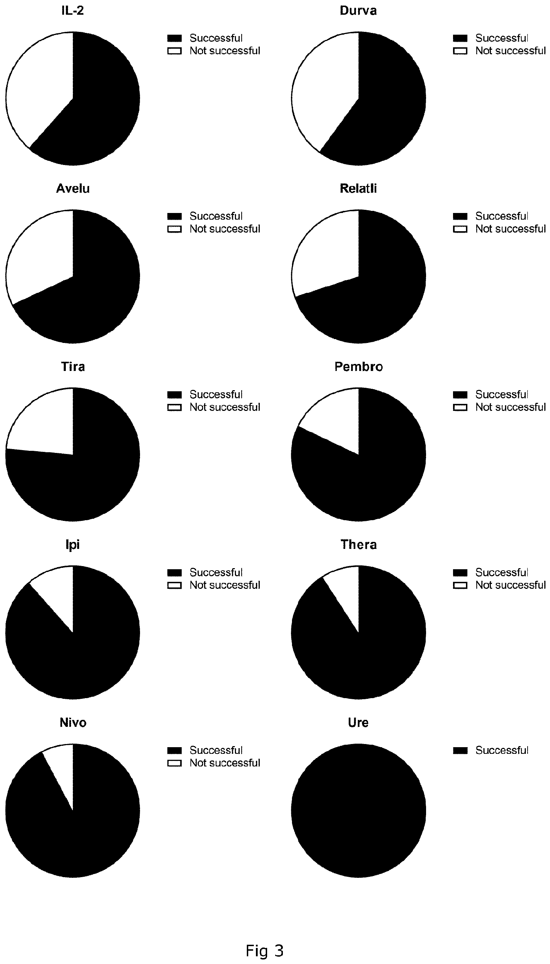

Figure 3 shows percentage of successful (black) and not successful (white) cell expansions (> 50,000 cells/fragment) using IL-2, durvalumab (Durva), avelumab (Avelu), relatlimab (Relatli), tiragolumab (Tira), Pembrolizumab (Pembro), ipilimumab (Ipi), theralizumab (Thera), nivolumab (Nivo), or urelumab/OKT3 (Ure).

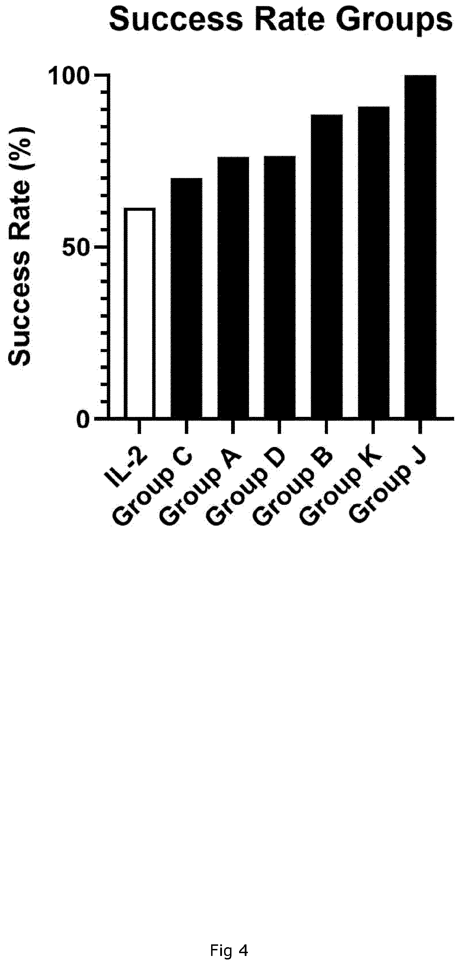

Figure 4 shows percentage of successful cell expansions (> 50,000 cells/fragment) using IL-2 +/-TME-S. Refer to table 43 for the specific stimulator of each group.

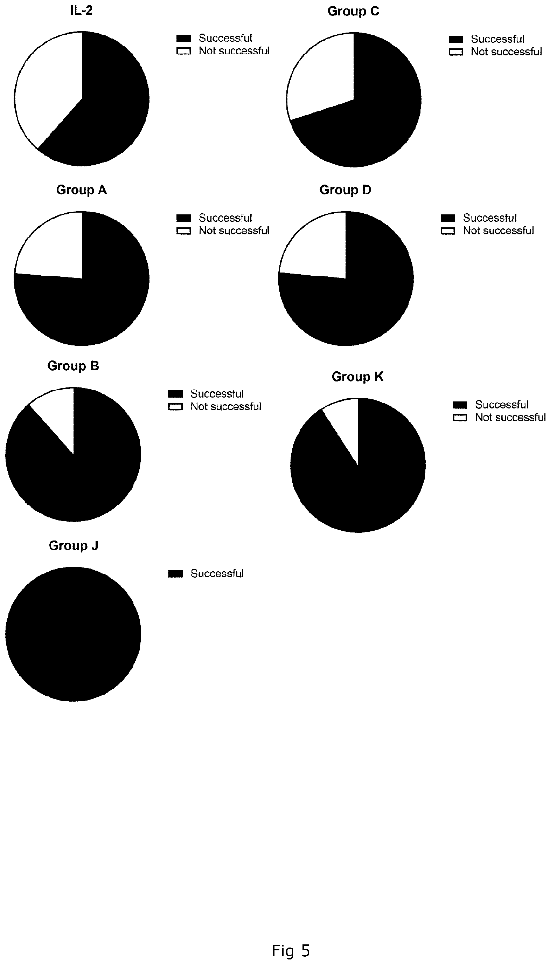

Figure 5 shows percentage of successful (black) and not successful (white) cell expansions (> 50,000 cells/fragment) using IL-2 +/- TME-S. Refer to table 43 for the specific stimulator of each group.

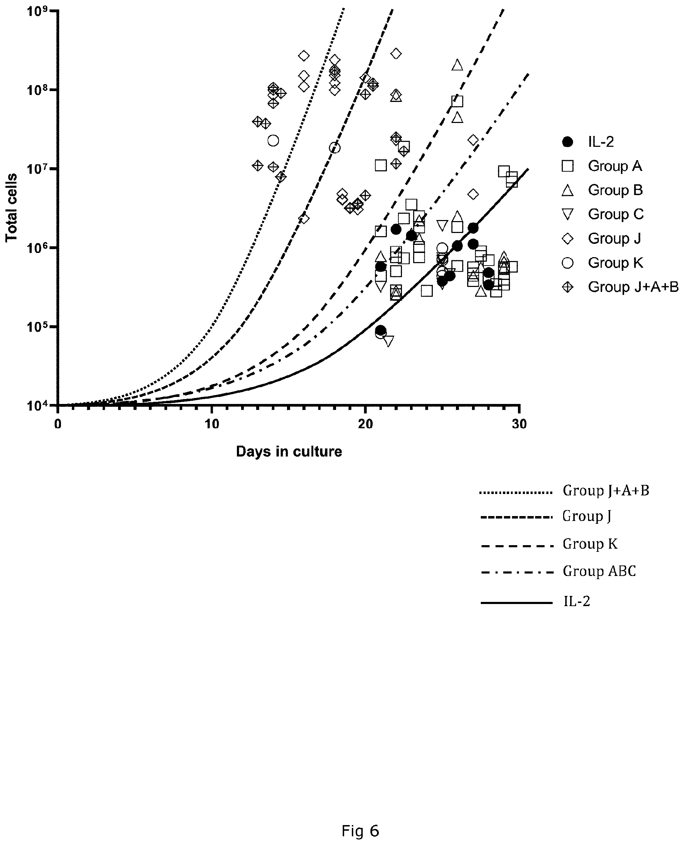

Figure 6 shows total cell number and expansion time for cell cultures in a G-Rex flask containing IL-2 +/- TME-S. Refer to table 43 for the specific stimulator of each group. Visual growth indication lines were manually added for simplicity.

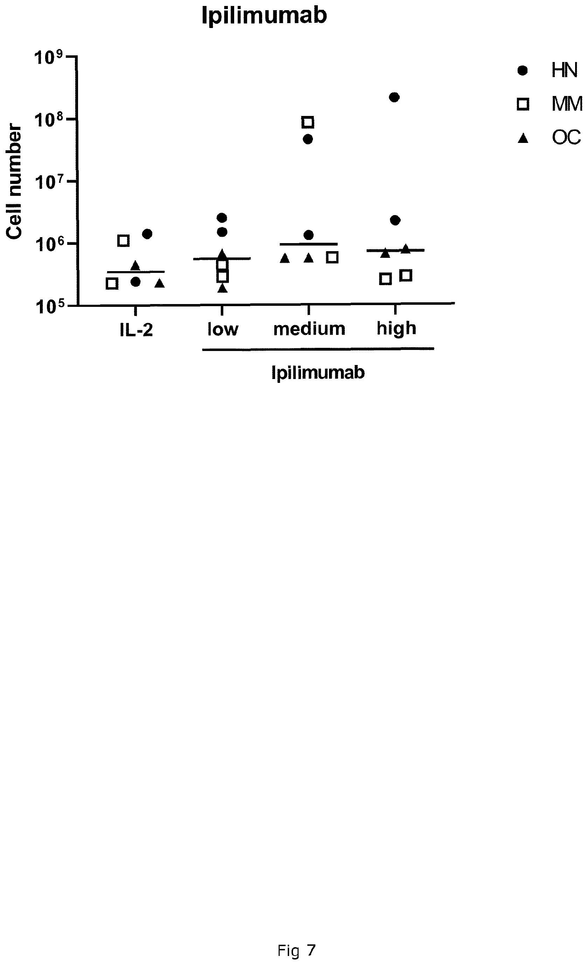

Figure 7 shows total cell number for cell expansion from head and neck cancer (HN), metastatic melanoma (MM) and ovarian cancer (OC) using anti-CTLA-4 (ipilimumab) at low (1 µg/ml), medium (5 µg/ml) and high (25 µg/ml) concentration.

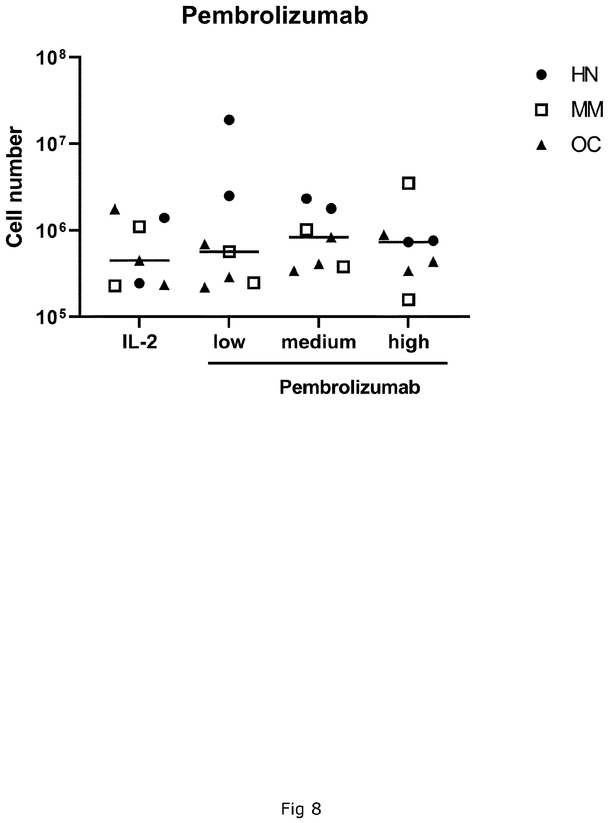

Figure 8 shows total cell number for cell expansion from head and neck cancer (HN), metastatic melanoma (MM) and ovarian cancer (OC) using anti-PD1 (pembrolizumab) at low (1 µg/ml), medium (5 µg/ml) and high (25 µg/ml) concentration.

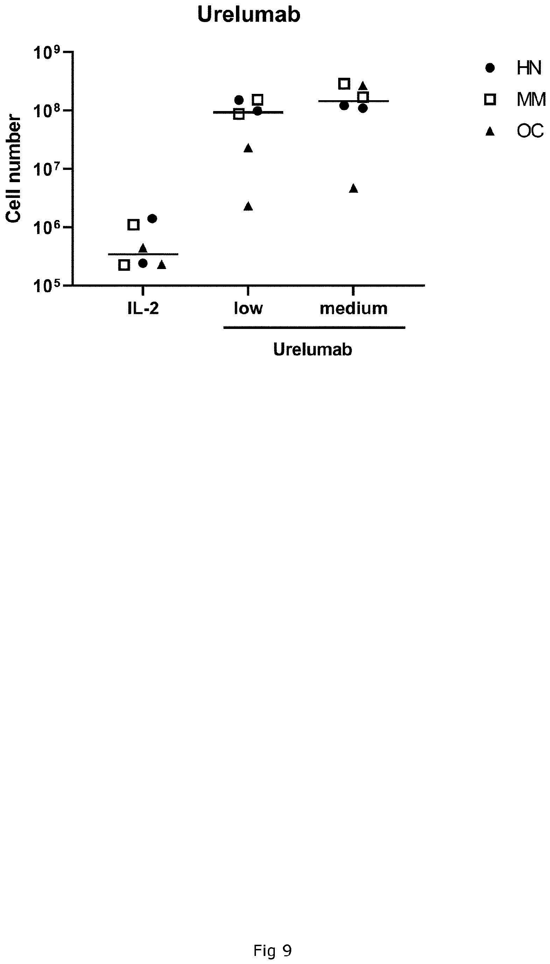

Figure 9 shows total cell number for cell expansion from head and neck cancer (HN), metastatic melanoma (MM) and ovarian cancer (OC) using anti-4-1BB (urelumab) at low (2 µg/ml) and medium (10 µg/ml) concentration together with OKT3 (30 ng/ml).

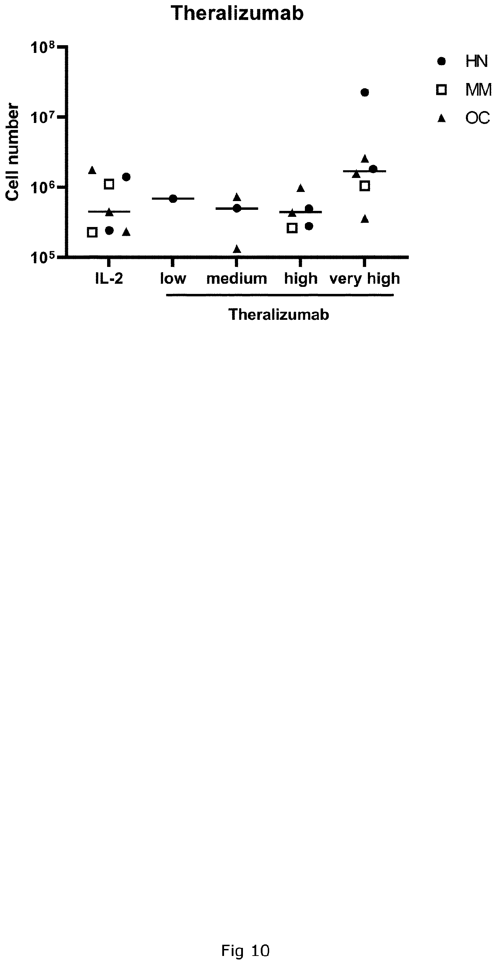

Figure 10 shows total cell number for cell expansion from head and neck cancer (HN), metastatic melanoma (MM) and ovarian cancer (OC) using anti-CD28 (theralizumab) at low (0.02 µg/ml) medium (0.1 µg/ml), high (2 µg/ml), and very high (2 µg/ml) concentration.

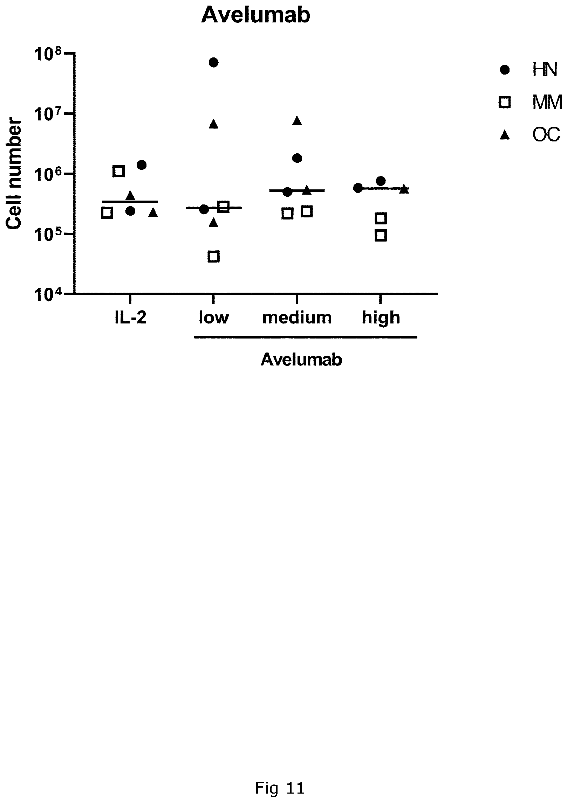

Figure 11 shows total cell number for cell expansion from head and neck cancer (HN), metastatic melanoma (MM) and ovarian cancer (OC) using anti-PD-L1 (avelumab) at low (2 µg/ml), medium (10 µg/ml) and high (50 µg/ml) concentration.

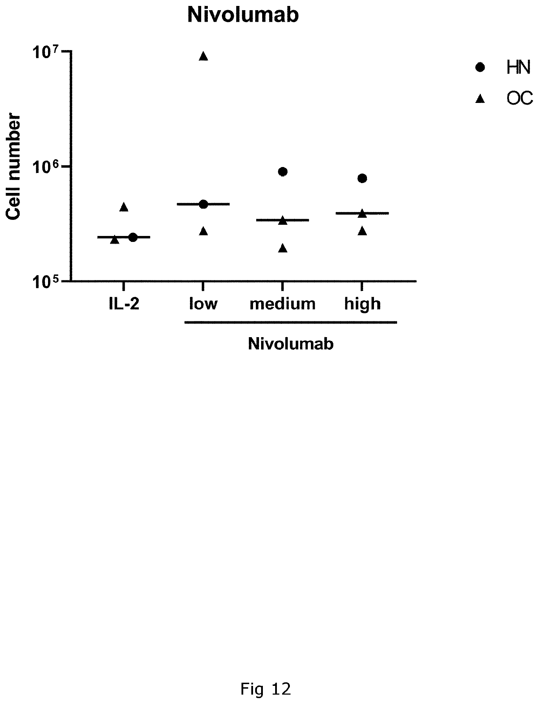

Figure 12 shows total cell number for cell expansion from head and neck cancer (HN), metastatic melanoma (MM) and ovarian cancer (OC) using anti-PD-1 (nivolumab) at low (2 µg/ml), medium (10 µg/ml) and high (50 µg/ml) concentration.
Figure 13 shows number of viable cells per fragment after incubation of tumor tissue from any cancer type in a G-Rex flask containing IL-2 +/- TME-S. Refer to table 43 for the specific stimulator of each group. Statistics performed by two-tailed Mann-Whitney U test comparing each group to controls (IL-2). p>0.05 was considered non-significant, *p<0.05, **p<0.01, ***p<0.001, ****p<0.0001.

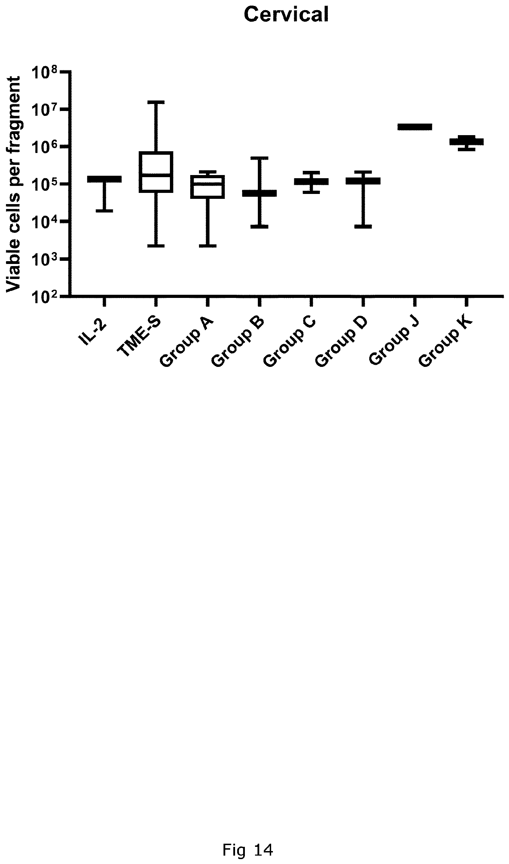

Figure 14 shows number of viable cells per fragment after incubation of tumor tissue from cervical cancer in a G-Rex flask containing IL-2 +/- TME-S. Refer to table 43 for the specific stimulator of each group. Statistics performed by two-tailed Mann-Whitney Utest comparing each group to controls (IL-2). p>0.05 was considered non-significant, *p<0.05, **p<0.01, ***p<0.001, ****p<0.0001.

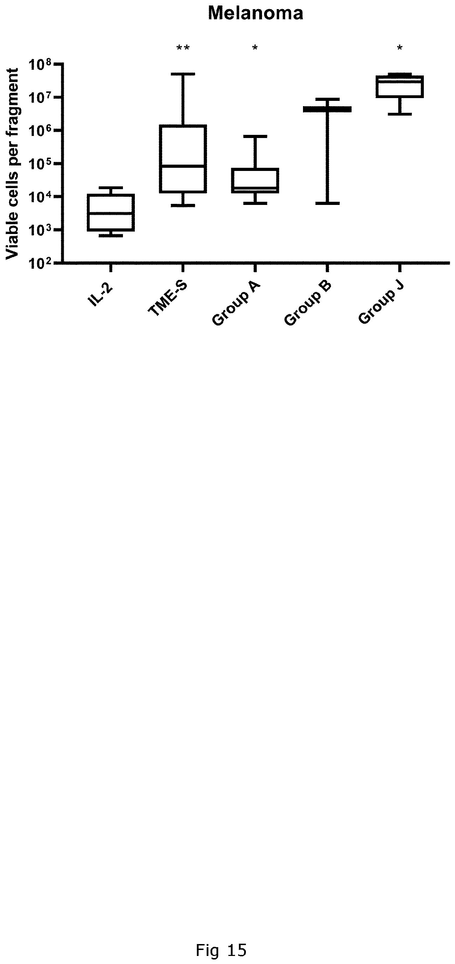

Figure 15 shows number of viable cells per fragment after incubation of tumor tissue from melanoma in a G-Rex flask containing IL-2 +/- TME-S. Refer to table 43 for the specific stimulator of each group. Statistics performed by two-tailed Mann-Whitney U test comparing each group to controls (IL-2). p>0.05 was considered non-significant, *p<0.05, **p<0.01, ***p<0.001, ****p<0.0001.

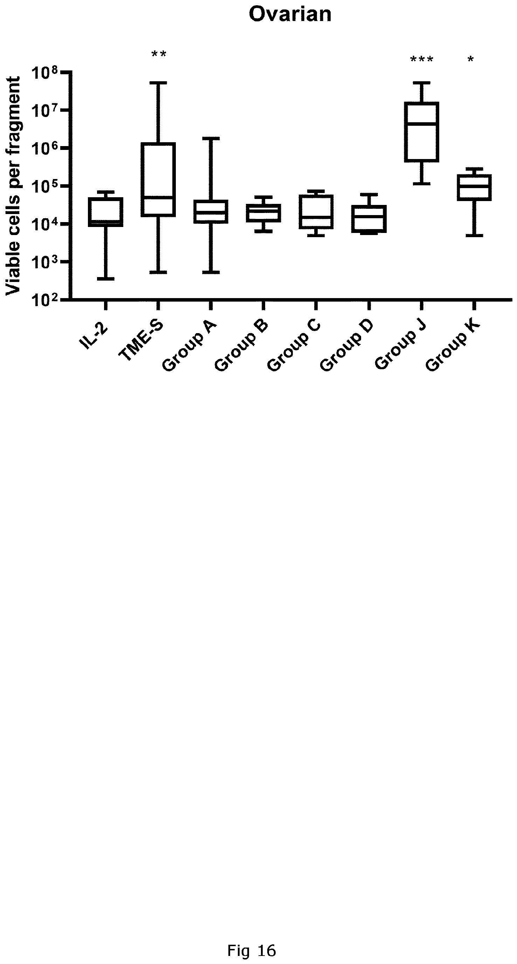

Figure 16 shows number of viable cells per fragment after incubation of tumor tissue from ovarian cancer in a G-Rex flask containing IL-2 +/- TME-S. Refer to table 43 for the specific stimulator of each group. Statistics performed by two-tailed Mann-Whitney U test comparing each group to controls (IL-2). p>0.05 was considered non-significant, *p<0.05, **p<0.01, ***p<0.001, ****p<0.0001.

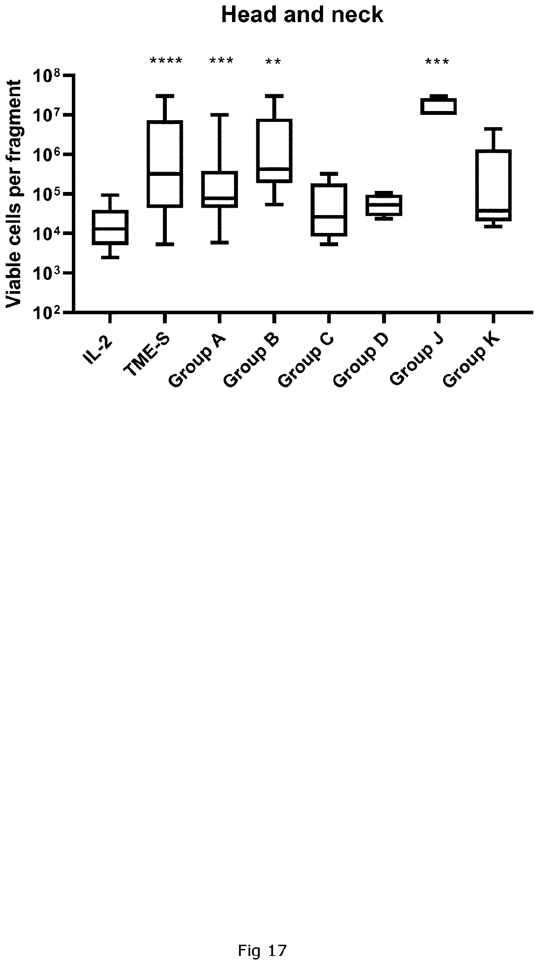

Figure 17 shows number of viable cells per fragment after incubation of tumor tissue from head and neck cancer in a G-Rex flask containing IL-2 +/- TME-S. Refer to table 43 for the specific stimulator of each group. Statistics performed by two-tailed Mann-Whitney U test comparing each group to controls (IL-2). p>0.05 was considered non-significant, *p<0.05, **p<0.01, ***p<0.001, ****p<0.0001.

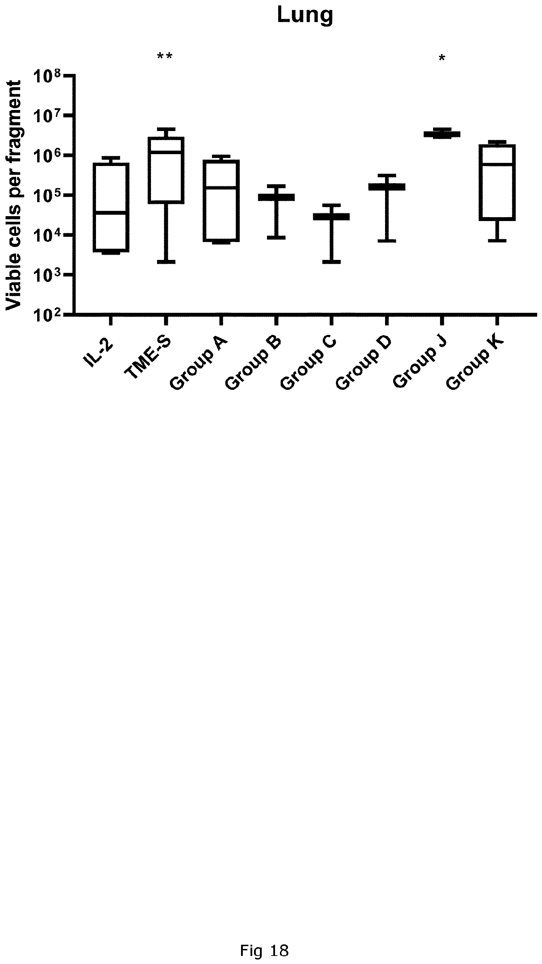

Figure 18 shows number of viable cells per fragment after incubation of tumor tissue from lung cancer in a G-Rex flask containing IL-2 +/- TME-S. Refer to table 43 for the specific stimulator of each group. Statistics performed by two-tailed Mann-Whitney U test comparing each group to controls (IL-2). p>0.05 was considered non-significant, *p<0.05, **p<0.01, ***p<0.001, ****p<0.0001.

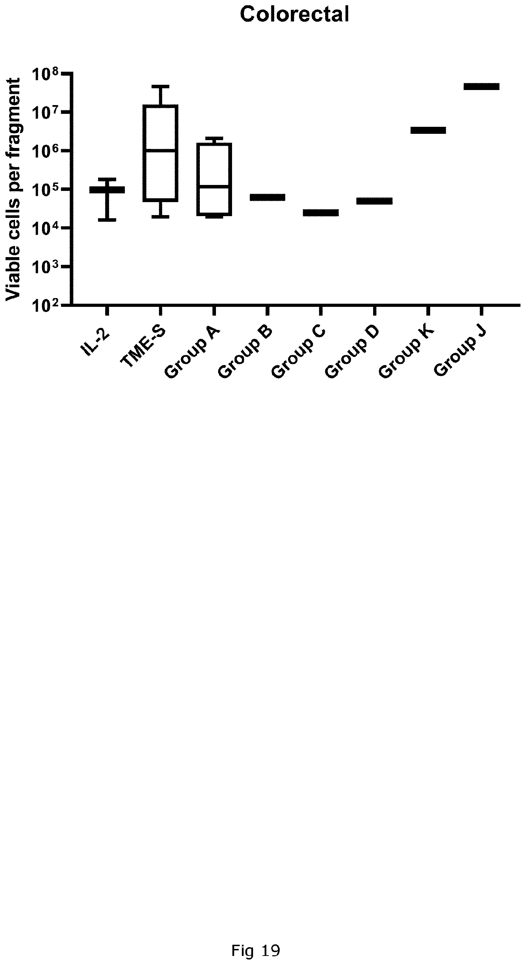

Figure 19 shows number of viable cells per fragment after incubation of tumor tissue from colorectal cancer in a G-Rex flask containing IL-2 +/- TME-S. Refer to table 43 for the specific stimulator of each group. Statistics performed by two-tailed Mann-Whitney U test comparing each group to controls (IL-2). p>0.05 was considered non-significant, *p<0.05, **p<0.01, ***p<0.001, ****p<0.0001.
Figure 20 shows number of viable cells per fragment after incubation of tumor tissue from any cancer type in a G-Rex flask containing IL-2 +/- antagonist, agonist,CD28 family, reinvigorating or depleting treatment. Refer to table 44 for the specific stimulator of each group. Statistics performed by two-tailed Mann-Whitney U test comparing each group to controls (IL-2). p>0.05 was considered non-significant, *p<0.05, **p<0.01, ***p<0.001, ****p<0.0001.

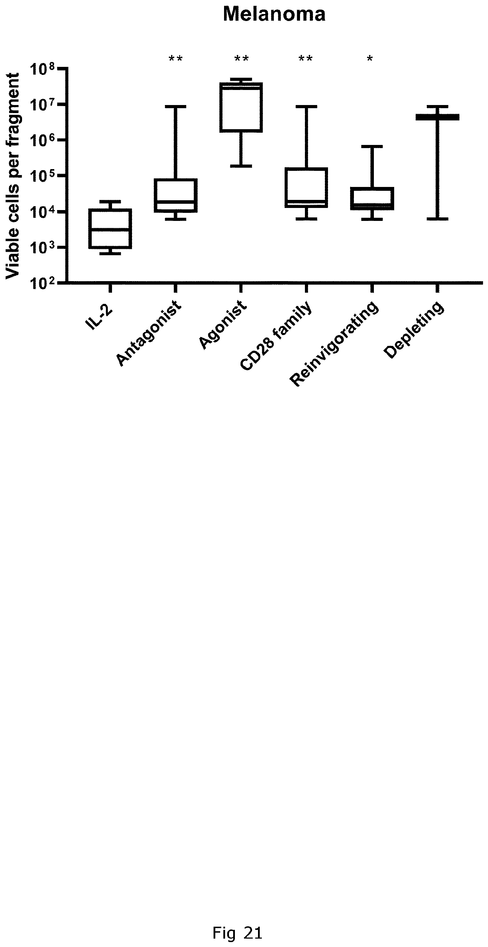

Figure 21 shows number of viable cells per fragment after incubation of tumor tissue from melanoma in a G-Rex flask containing IL-2 +/- antagonist, agonist, CD28 family, reinvigorating or depleting treatment. Refer to table 44 for the specific stimulator of each group. Statistics performed by two-tailed Mann-Whitney U test comparing each group to controls (IL-2). p>0.05 was considered non-significant, *p<0.05, **p<0.01, ***p<0.001, ****p<0.0001.

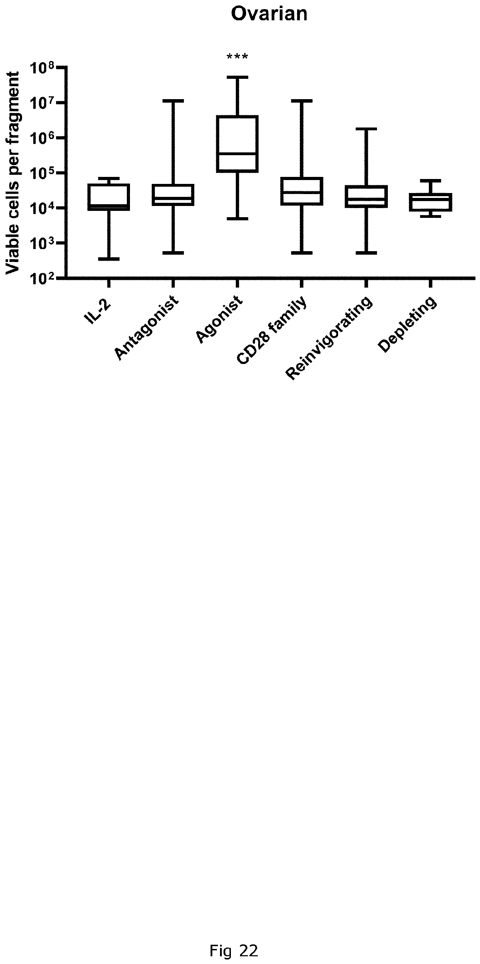

Figure 22 shows number of viable cells per fragment after incubation of tumor tissue from ovarian cancer in a G-Rex flask containing IL-2 +/- antagonist, agonist, CD28 family, reinvigorating or depleting treatment. Refer to table 44 for the specific stimulator of each group. Statistics performed by two-tailed Mann-Whitney U test comparing each group to controls (IL-2). p>0.05 was considered non-significant, *p<0.05, **p<0.01, ***p<0.001, ****p<0.0001.

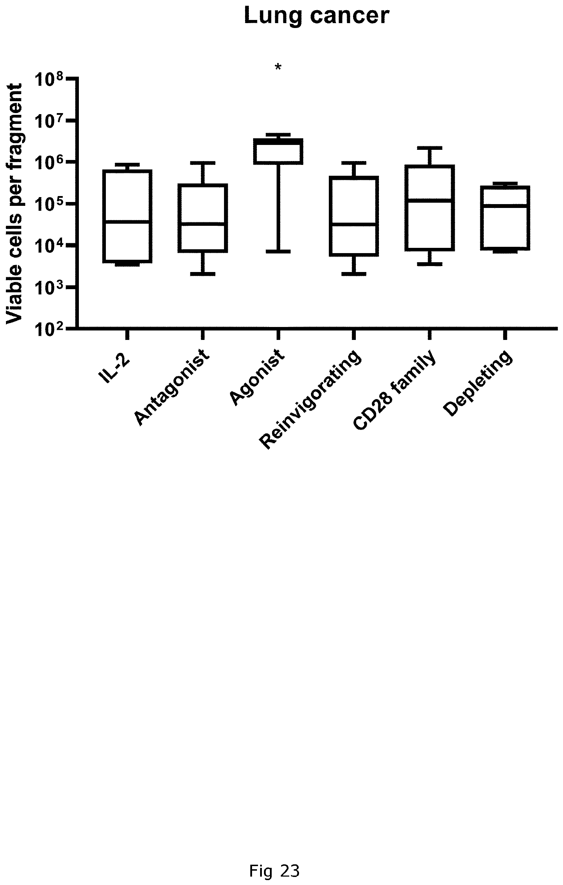

Figure 23 shows number of viable cells per fragment after incubation of tumor tissue from lung cancer in a G-Rex flask containing IL-2 +/- antagonist, agonist, CD28 family, reinvigorating or depleting treatment. Refer to table 44 for the specific stimulator of each group. Statistics performed by two-tailed Mann-Whitney U test comparing each group to controls (IL-2). p>0.05 was considered non-significant, *p<0.05, **p<0.01, ***p<0.001, ****p<0.0001.

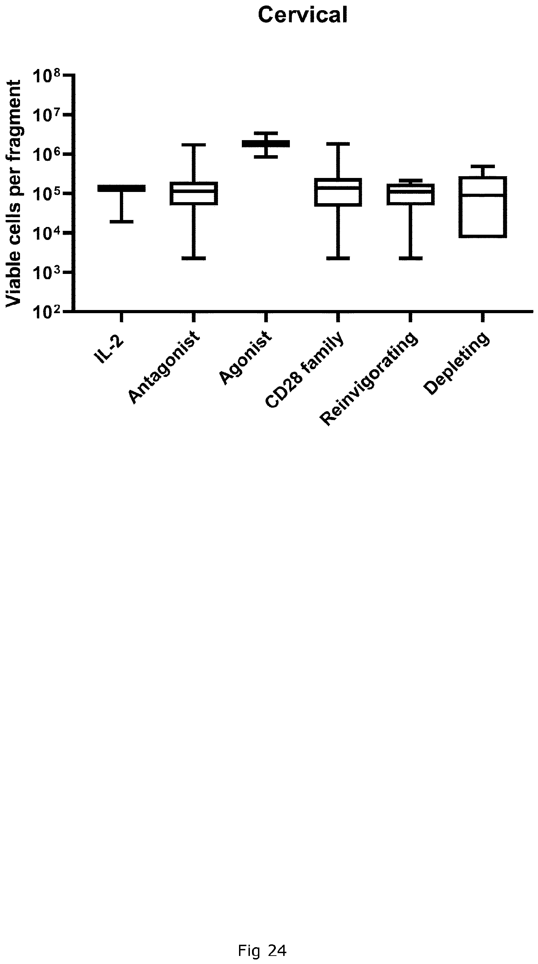

Figure 24 shows number of viable cells per fragment after incubation of tumor tissue from cervical cancer in a G-Rex flask containing IL-2 +/- antagonist, agonist, CD28 family, reinvigorating or depleting treatment. Refer to table 44 for the specific stimulators of each group. Statistics performed by two-tailed Mann-Whitney U test comparing each group to controls (IL-2). p>0.05 was considered non-significant, *p<0.05, **p<0.01, ***p<0.001, ****p<0.0001.

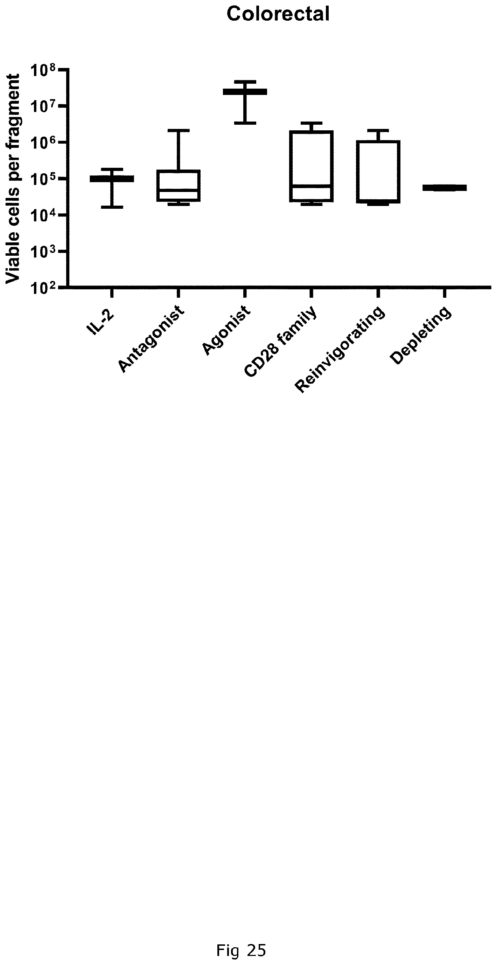

Figure 25 shows number of viable cells per fragment after incubation of tumor tissue from colorectal cancer in a G-Rex flask containing IL-2 +/- antagonist, agonist, CD28 family, reinvigorating or depleting treatment. Refer to table 44 for the specific stimulator of each group. Statistics performed by two-tailed Mann-Whitney U test comparing each group to controls (IL-2). p>0.05 was considered non-significant, *p<0.05, **p<0.01, ***p<0.001, ****p<0.0001.

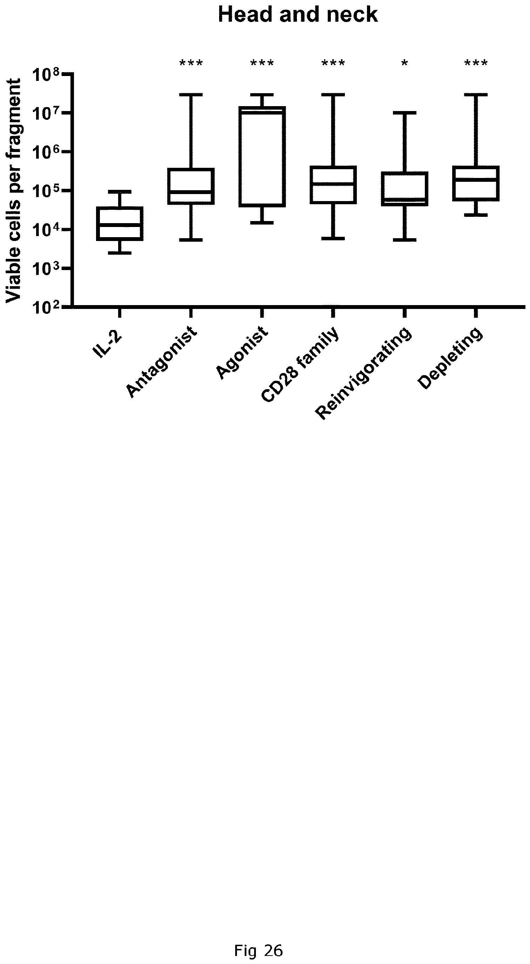

Figure 26 shows number of viable cells per fragment after incubation of tumor tissue from head and neck cancer in a G-Rex flask containing IL-2 +/- antagonist, agonist, CD28 family, reinvigorating or depleting treatment. Refer to table 44 for the specific stimulator of each group. Statistics performed by two-tailed Mann-Whitney U test comparing each group to controls (IL-2). p>0.05 was considered non-significant, *p<0.05, **p<0.01, ***p<0.001, ****p<0.0001.
Figure 27 shows number of viable cells per fragment after incubation of tumor tissue from any cancer type in a G-Rex flask containing IL-2 +/- PD-1, PD-L1. Refer to table 43 for the specific stimulator of each group. Statistics performed by two-tailed Mann-Whitney U test comparing each group to controls (IL-2). p>0.05 was considered non-significant, *p<0.05, **p<0.01, ***p<0.001, ****p<0.0001.
Figure 28 shows number of viable cells per fragment after incubation of tumor tissue from ovarian cancer in a G-Rex flask containing IL-2 +/- PD-1, PD-L1. Refer to table 43 for the specific stimulator of each group. Statistics performed by two-tailed Mann-Whitney U test comparing each group to controls (IL-2). p>0.05 was considered non-significant, *p<0.05, **p<0.01, ***p<0.001, ****p<0.0001.

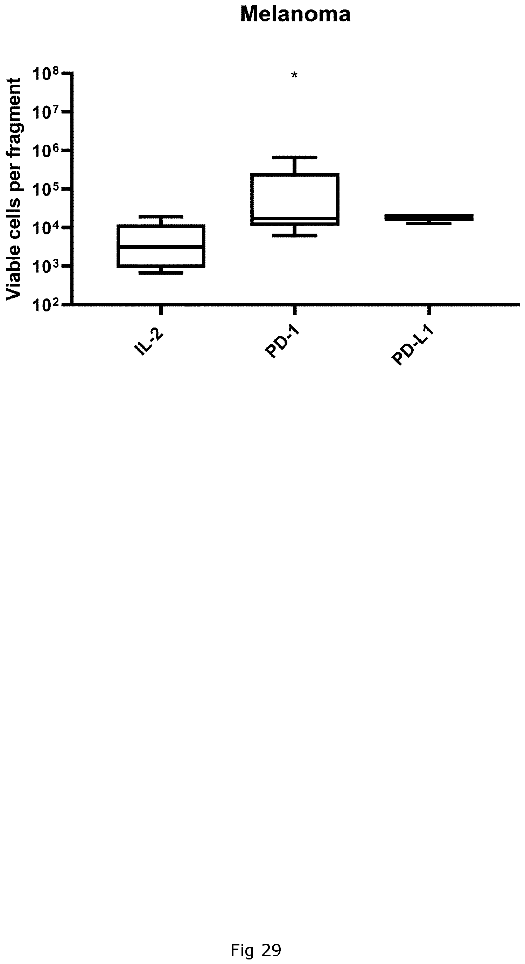

Figure 29 shows number of viable cells per fragment after incubation of tumor tissue from melanoma in a G-Rex flask containing IL-2 +/- PD-1, PD-L1. Refer to table 43 for the specific stimulator of each group. Statistics performed by two-tailed Mann-Whitney U test comparing each group to controls (IL-2). p>0.05 was considered non-significant, *p<0.05, **p<0.01, ***p<0.001, ****p<0.0001.

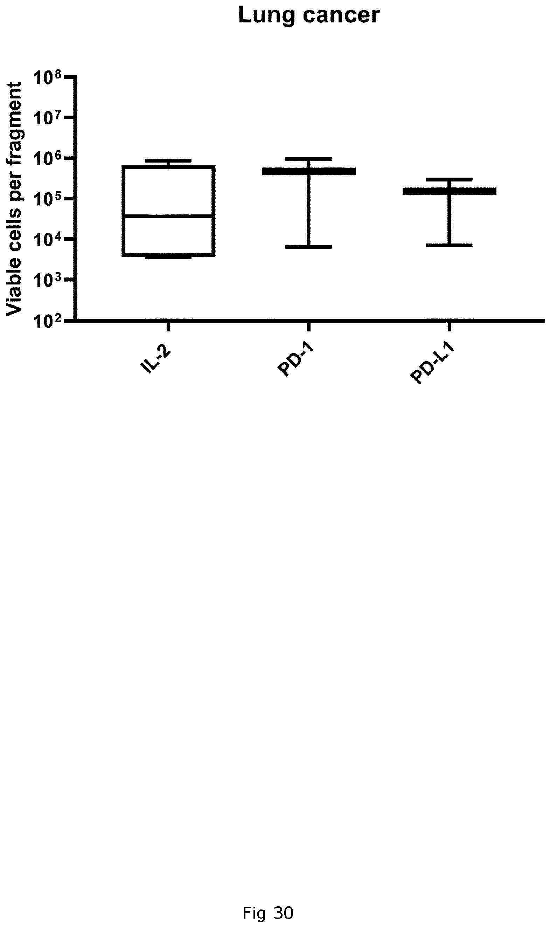

Figure 30 shows number of viable cells per fragment after incubation of tumor tissue from lung cancer in a G-Rex flask containing IL-2 +/- PD-1, PD-L1. Refer to table 43 for the specific stimulator of each group. Statistics performed by two-tailed Mann-Whitney U test comparing each group to controls (IL-2). p>0.05 was considered non-significant, *p<0.05, **p<0.01, ***p<0.001, ****p<0.0001.

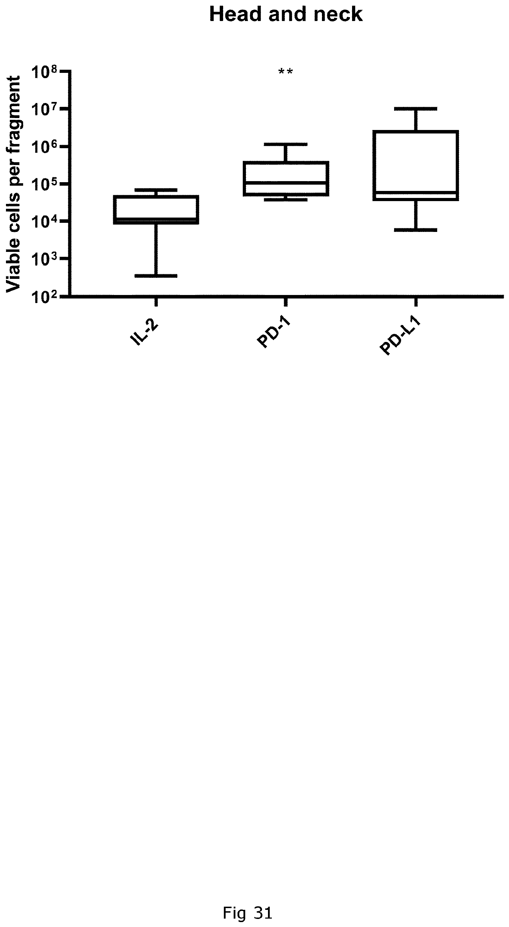

Figure 31 shows number of viable cells per fragment after incubation of tumor tissue from head and neck cancer in a G-Rex flask containing IL-2 +/- PD-1, PD-L1. Refer to table 43 for the specific stimulator of each group. Statistics performed by two-tailed Mann-Whitney U test comparing each group to controls (IL-2). p>0.05 was considered non-significant, *p<0.05, **p<0.01, ***p<0.001, ****p<0.0001.

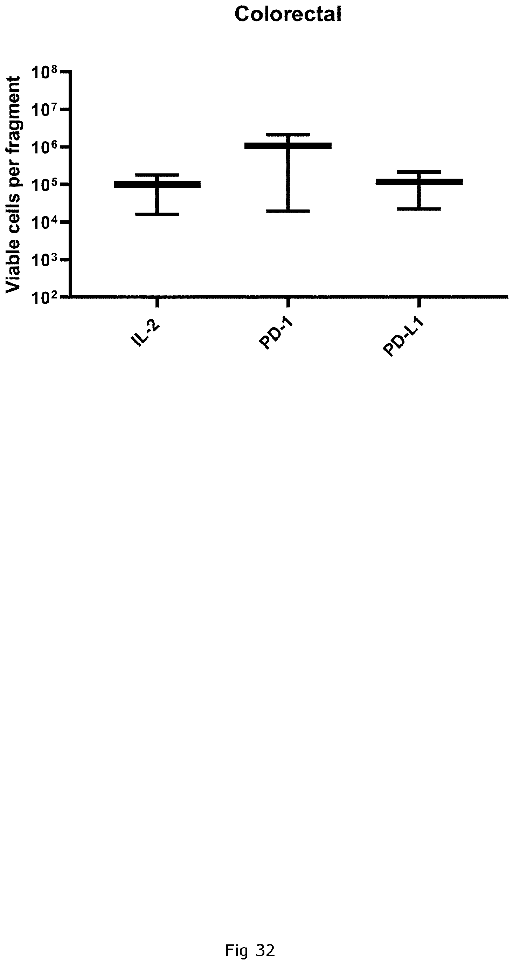

Figure 32 shows number of viable cells per fragment after incubation of tumor tissue from colorectal cancer in a G-Rex flask containing IL-2 +/- PD-1, PD-L1. Refer to table 43 for the specific stimulator of each group. Statistics performed by two-tailed Mann-Whitney U test comparing each group to controls (IL-2). p>0.05 was considered non-significant, *p<0.05, **p<0.01, ***p<0.001, ****p<0.0001.

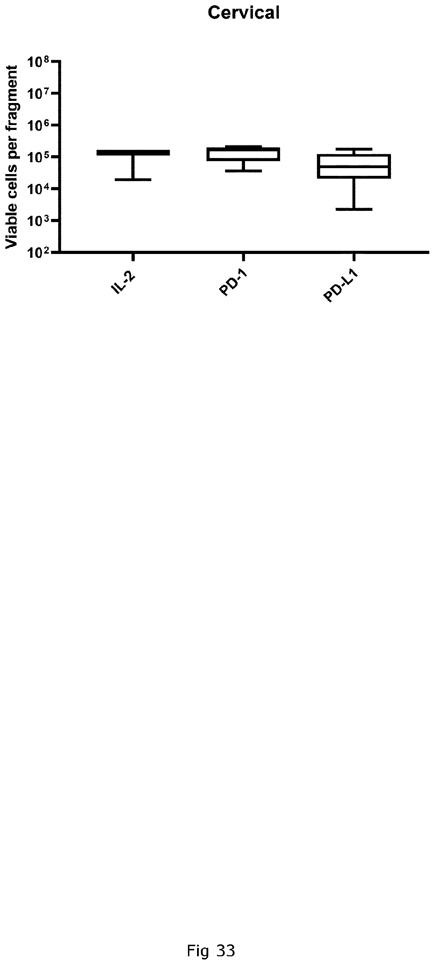

Figure 33 shows number of viable cells per fragment after incubation of tumor tissue from cervical cancer in a G-Rex flask containing IL-2 +/- PD-1, PD-L1. Refer to table 43 for the specific stimulator of each group. Statistics performed by two-tailed Mann-Whitney U test comparing each group to controls (IL-2). p>0.05 was considered non-significant, *p<0.05, **p<0.01, ***p<0.001, ****p<0.0001.
Figure 34 shows number of viable cells per fragment after incubation of tumor tissue from any cancer type in a G-Rex flask containing IL-2 +/- group A, pembrolizumab, nivolumab, durvalumab, avelumab. Refer to table 43 for the specific stimulator of each group. Statistics performed by two-tailed Mann-Whitney U test comparing each group to controls (IL-2). p>0.05 was considered non-significant, *p<0.05, **p<0.01, ***p<0.001, ****p<0.0001.
Figure 35 shows number of viable cells per fragment after incubation of tumor tissue from ovarian cancer in a G-Rex flask containing IL-2 +/- group A, pembrolizumab, nivolumab, durvalumab, avelumab. Refer to table 43 for the specific stimulator of each group. Statistics performed by two-tailed Mann-Whitney U test comparing each group to controls (IL-2). p>0.05 was considered non-significant, *p<0.05, **p<0.01, ***p<0.001, ****p<0.0001.

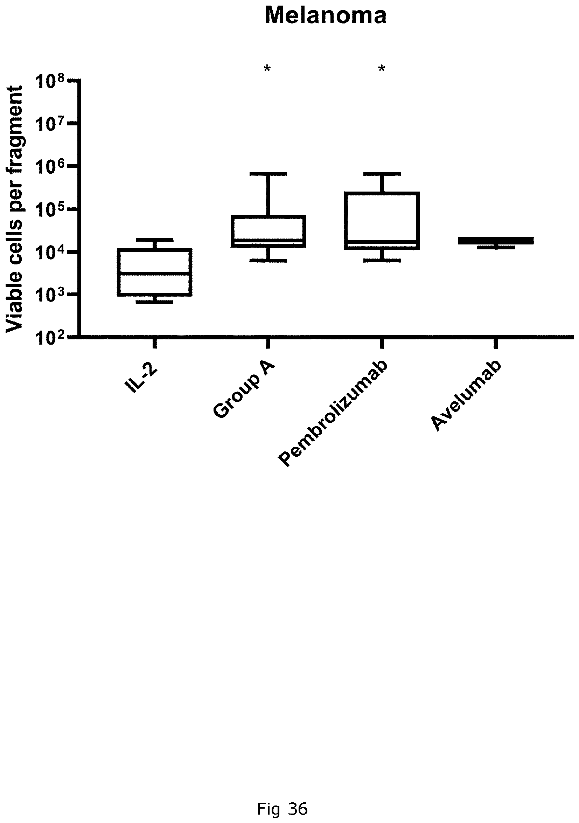

Figure 36 shows number of viable cells per fragment after incubation of tumor tissue from melanoma in a G-Rex flask containing IL-2 +/- group A, pembrolizumab, nivolumab, durvalumab, avelumab. Refer to table 43 for the specific stimulator of each group. Statistics performed by two-tailed Mann-Whitney U test comparing each group to controls (IL-2). p>0.05 was considered non-significant, *p<0.05, **p<0.01, ***p<0.001, ****p<0.0001.

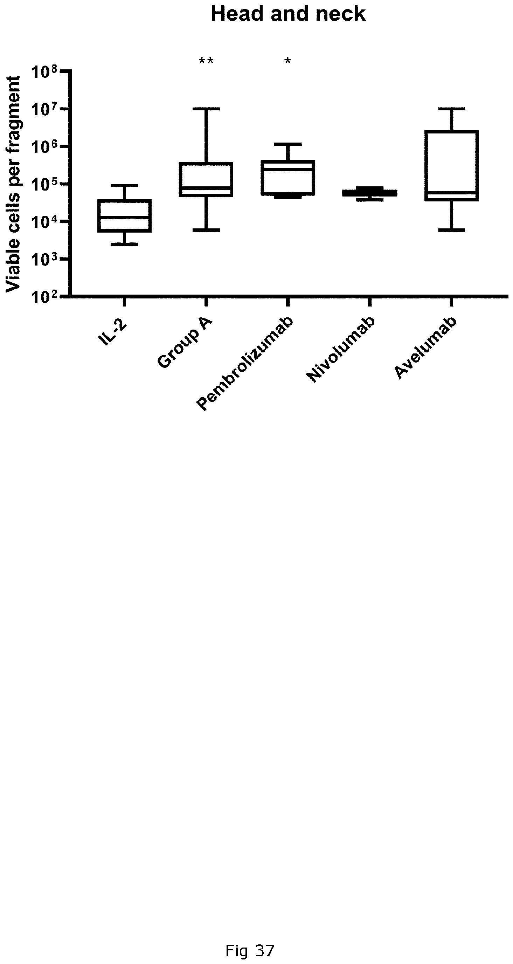

Figure 37 shows number of viable cells per fragment after incubation of tumor tissue from head and neck cancer in a G-Rex flask containing IL-2 +/- group A, pembrolizumab, nivolumab, durvalumab, avelumab. Refer to table 43 for the specific stimulator of each group. Statistics performed by two-tailed Mann-Whitney U test comparing each group to controls (IL-2). p>0.05 was considered non-significant, *p<0.05, **p<0.01, ***p<0.001, ****p<0.0001.

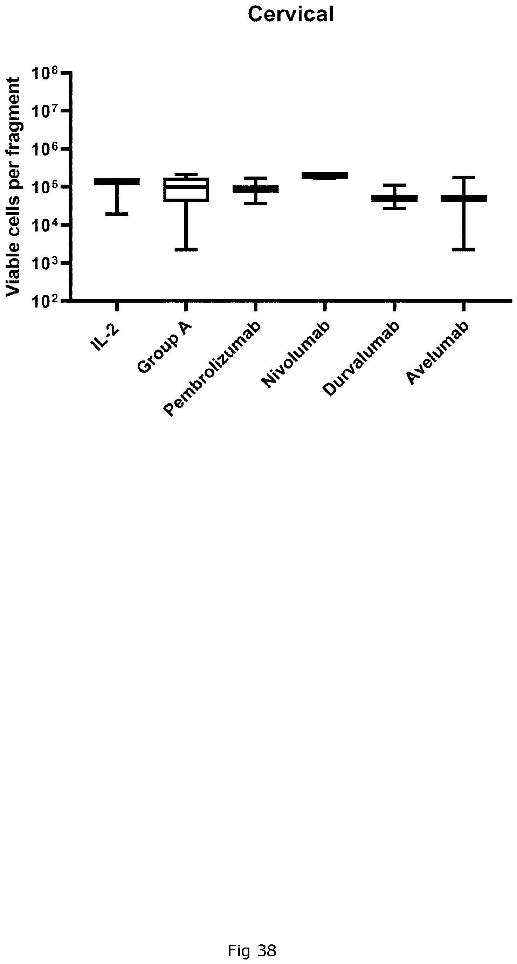

Figure 38 shows number of viable cells per fragment after incubation of tumor tissue from cervical cancer in a G-Rex flask containing IL-2 +/- group A, pembrolizumab, nivolumab, durvalumab, avelumab. Refer to table 43 for the specific stimulator of each group. Statistics performed by two-tailed Mann-Whitney U test comparing each group to controls (IL-2). p>0.05 was considered non-significant, *p<0.05, **p<0.01, ***p<0.001, ****p<0.0001.
Figure 39 shows number of viable cells per fragment after incubation of tumor tissue from any cancer type in a G-Rex flask containing IL-2 +/- Group A, Group B, or Group A+B. Refer to table 43 for specific stimulator of each group. Statistics performed by two-tailed Mann-Whitney U test comparing each group to controls (IL-2). p>0.05 was considered non-significant, *p<0.05, **p<0.01, ***p<0.001, ****p<0.0001.
Figure 40 shows number of viable cells per fragment after incubation of tumor tissue from any cancer type in a G-Rex flask containing IL-2 +/- urelumab/OKT3, ipilimumab (ipi), pembrolizumab (pembro). Statistics performed by two-tailed Mann-Whitney U test comparing each group to controls (urelumab/OKT3). p>0.05 was considered non-significant, *p<0.05, **p<0.01, ***p<0.001, ****p<0.0001.
Figure 41 shows number of viable cells per fragment after incubation of tumor tissue from any cancer type in a G-Rex flask containing IL-2 +/- urelumab/OKT3, ipilimumab (ipi), pembrolizumab (pembro) with and without time delay.. Statistics performed by two-tailed Mann-Whitney U test. p>0.05 was considered non-significant, *p<0.05, **p<0.01, ***p<0.001, ****p<0.0001.
Figure 42 shows number of viable cells per fragment after incubation of tumor tissue from any cancer type in a G-Rex flask containing IL-2 + theralizumab or IL-2 + theralizumab, ipilimumab (ipi) andpembrolizumab (pembro). Statistics performed by two-tailed Mann-Whitney U test. p>0.05 was considered non-significant, *p<0.05, **p<0.01, ***p<0.001, ****p<0.0001.

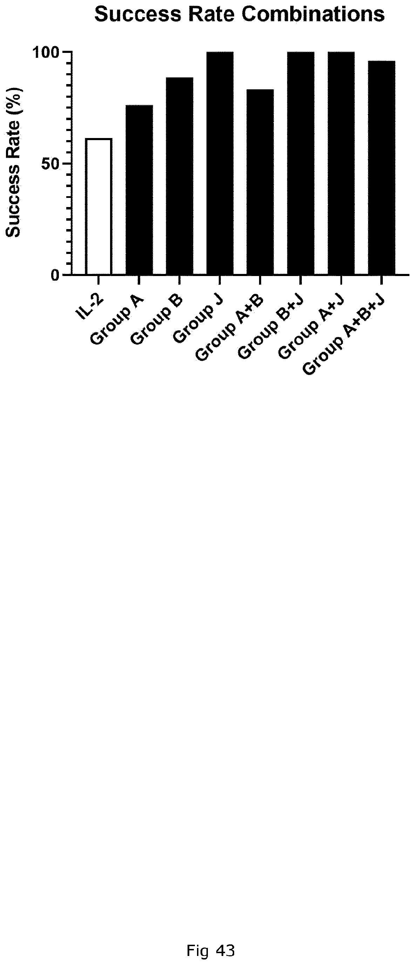

Figure 43 shows percentage of successful cell expansions (> 50,000 cells/fragment) for all cancer types using IL-2 +/- TME-S. Refer to table 43 for the specific stimulator of each group.

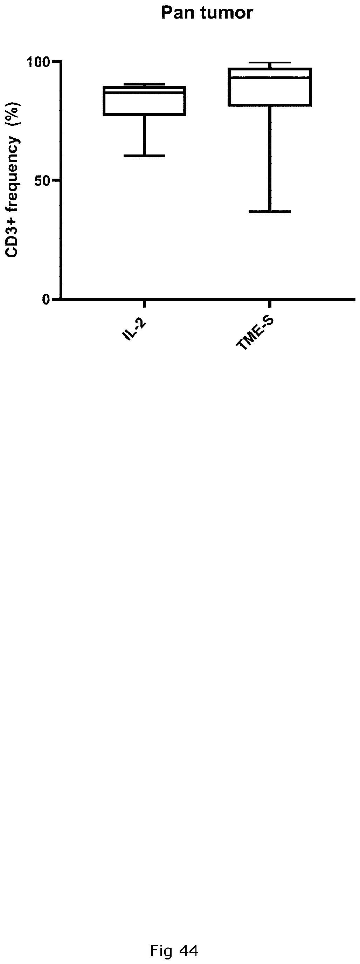

Figure 44 shows frequency of T cells (CD3+) for all cancer types using IL-2 +/- TME-S. Refer to table 43 for the stimulators used. Statistics performed by two-tailed Mann-Whitney U test. p>0.05 was considered non-significant.

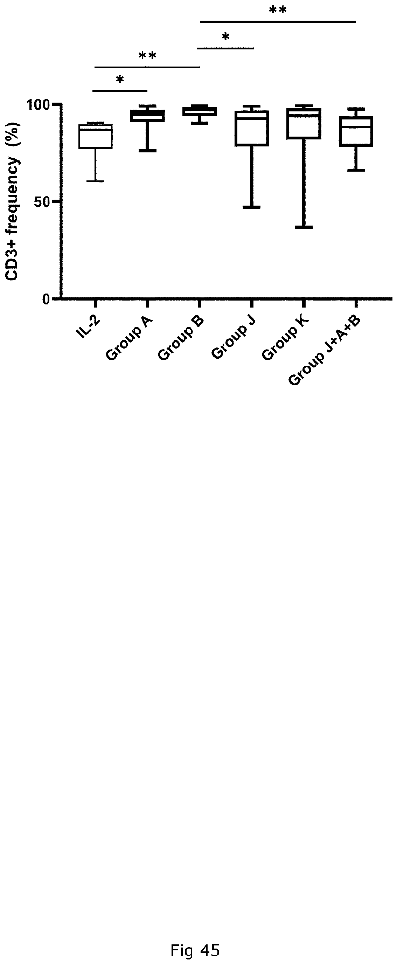

Figure 45 shows frequency of T cells (CD3+) for all cancer types using IL-2 +/- TME-S. Refer to table 43 for the specific stimulator of each group. Statistics performed by two-tailed Mann-Whitney U test. p>0.05 was considered non-significant, *p<0.05, **p<0.01.

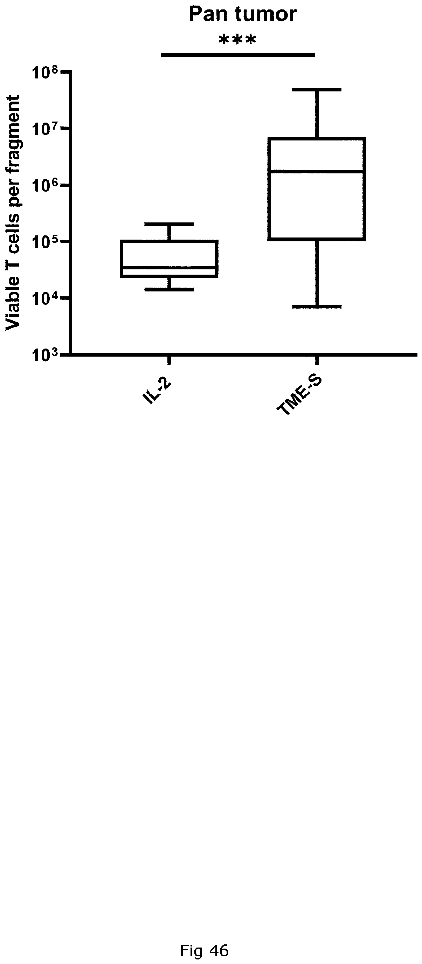

Figure 46 shows number of viable T cells (CD3+) per tumor fragment for all cancer types using IL-2 +/- TME-S. Refer to table 43 for the stimulators used. Statistics performed by two-tailed Mann-Whitney U test. p>0.05 was considered non-significant, *p<0.05, **p<0.01, ***p<0.001.

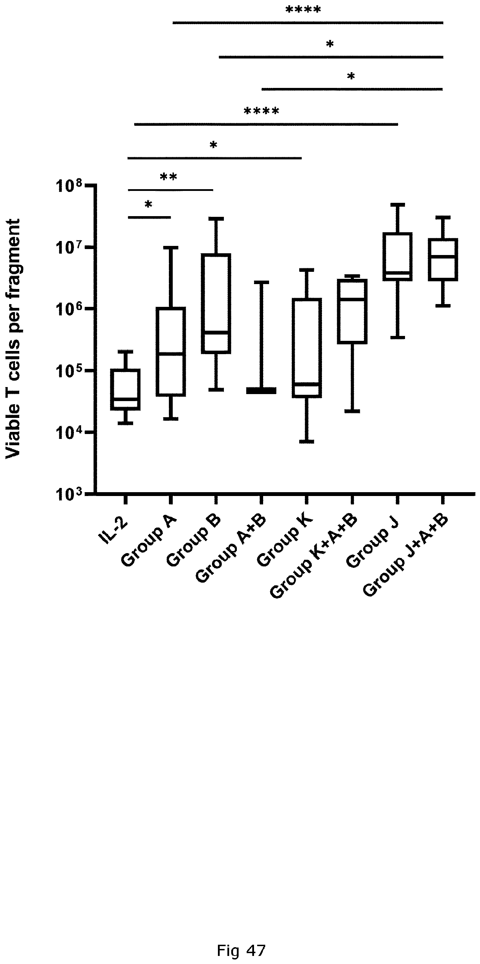

Figure 47 shows number of viable T cells (CD3+) per tumor fragment for all cancer types using IL-2 +/- TME-S. Refer to table 43 for the specific stimulator of each group. Statistics performed by two-tailed Mann-Whitney U test. p>0.05 was considered non-significant, *p<0.05, **p<0.01, ***p<0.001, ****p<0.0001.

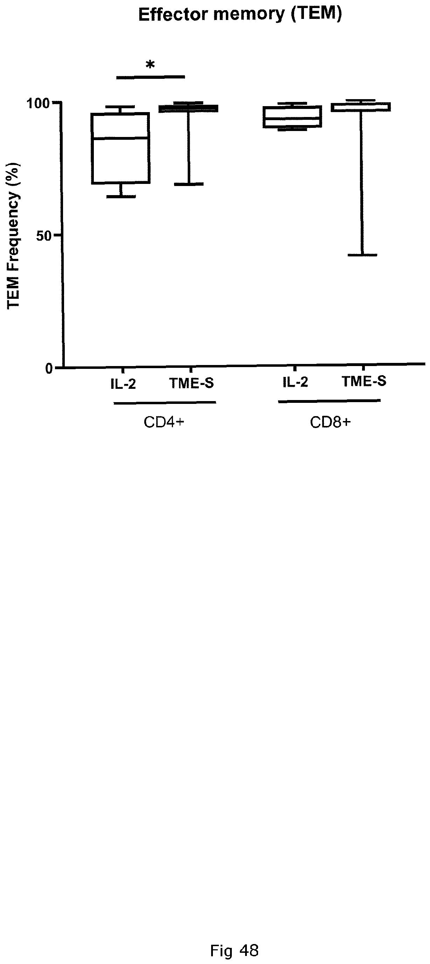

Figure 48 shows the frequency of effector memory T cells (TEM) for all cancer types of CD4+ (left) and CD8+ (right) T cells using IL-2 +/- TME-S. Refer to table 43 for the stimulators used. Statistics performed by two-tailed Mann-Whitney U test. p>0.05 was considered non-significant, *p<0.05.

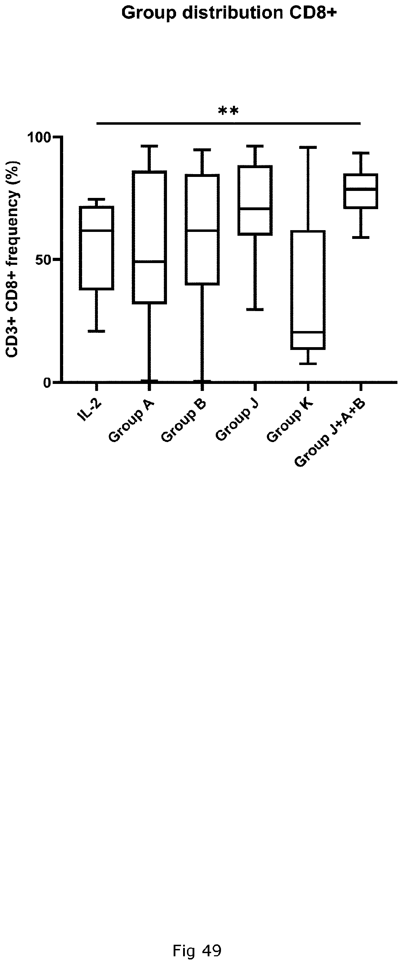

Figure 49 shows frequency of CD8+ T cells for all cancer types using IL-2 +/- TME-S. Refer to table 43 for the specific stimulator of each group. Statistics performed by two-tailed Mann-Whitney U test. p>0.05 was considered non-significant, *p<0.05, **p<0.01.

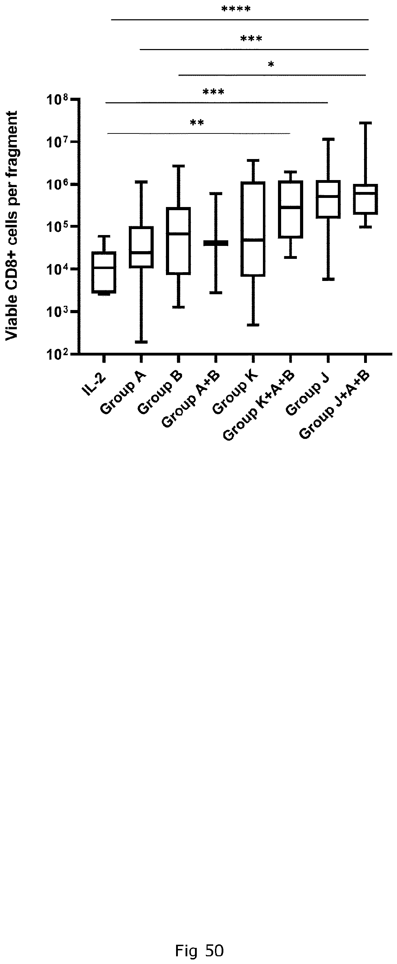

Figure 50 shows number of viable CD8+ T cells per tumor fragment for all cancer types using IL-2 +/- TME-S. Refer to table 43 for the specific stimulator of each group. Statistics performed by two-tailed Mann-Whitney U test. p>0.05 was considered non-significant, *p<0.05, **p<0.01, ***p<0.001, ****p<0.0001.

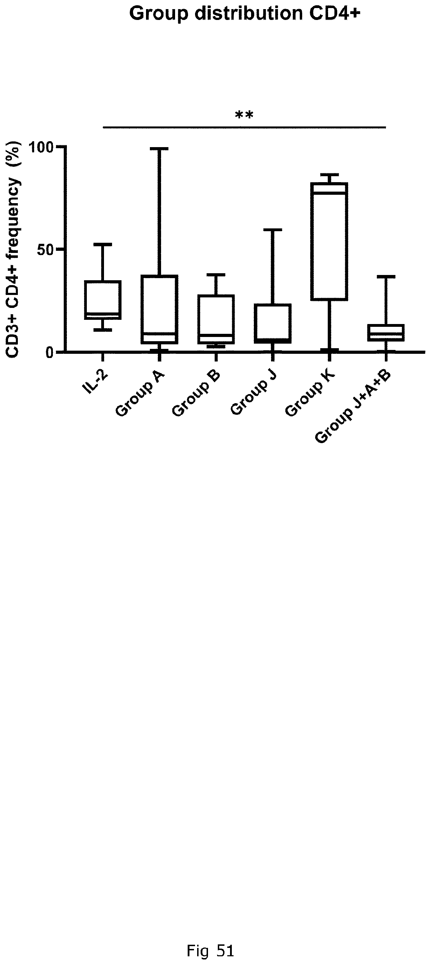

Figure 51 shows frequency of CD4+ T cells for all cancer types using IL-2 +/- TME-S. Refer to table 43 for the specific stimulator of each group. Statistics performed by two-tailed Mann-Whitney U test. p>0.05 was considered non-significant, *p<0.05, **p<0.01.

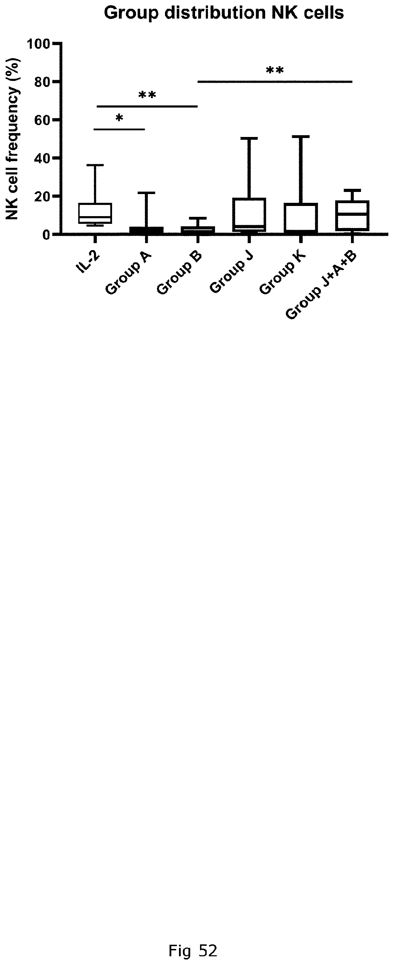

Figure 52 shows frequency of NK cells for all cancer types using IL-2 +/- TME-S. Refer to table 43 for the specific stimulator of each group. Statistics performed by two-tailed Mann-Whitney U test. p>0.05 was considered non-significant, *p<0.05, **p<0.01.

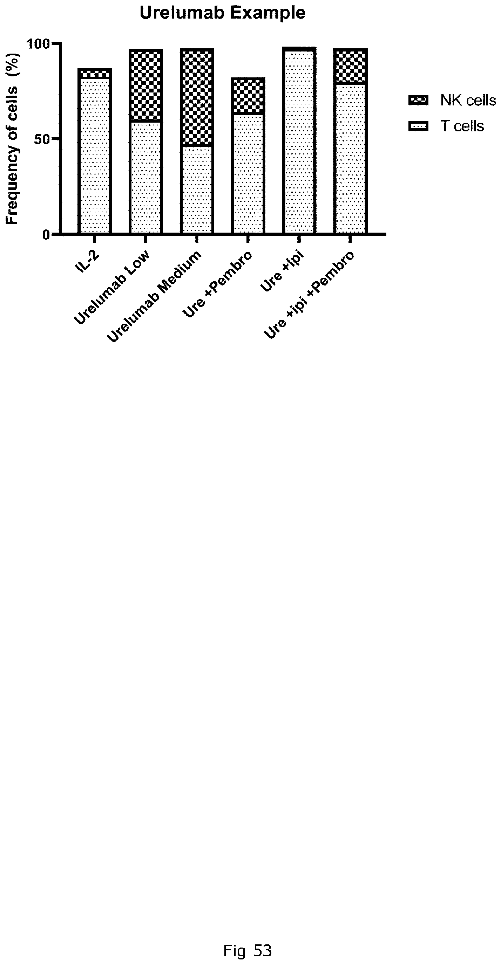

Figure 53 shows frequencies of T cells (CD3+) and NK (CD3- CD56+) cells for head and neck cancer using IL-2 +/- urelumab (Ure) low (2 µg/ml) with OKT3 (30 ng/mL), urelumab medium (10 µg/ml) with OKT3 (30 ng/ml), pembrolizumab (Pembro), Ipilimumab (Ipi).

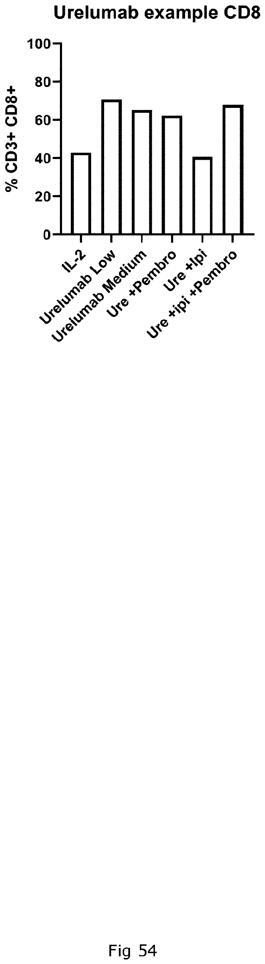

Figure 54 shows frequency ofCD8+ T cells for head and neck cancer using IL-2 +/- urelumab (Ure) low (2 µg/ml) with OKT3 (30 ng/ml), urelumab medium (10 µg/ml) with OKT3 (30 ng/ml), pembrolizumab (Pembro), Ipilimumab (Ipi).

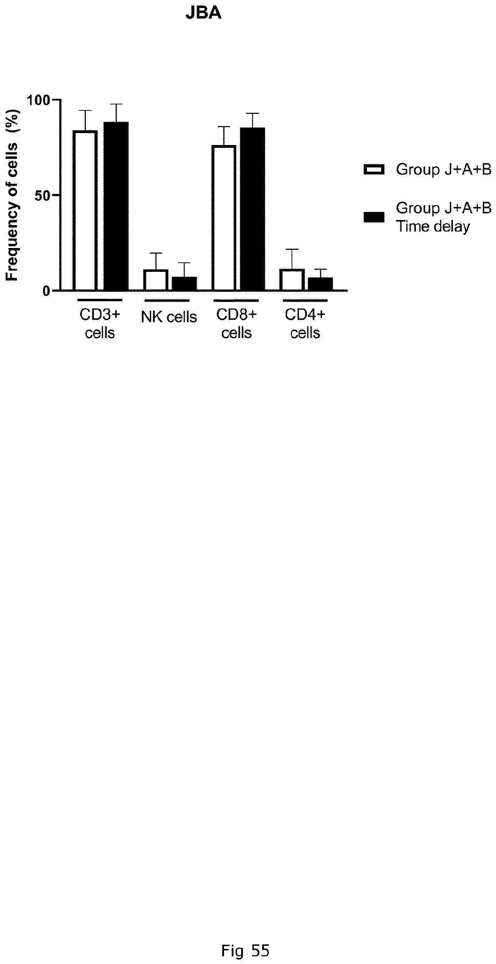

Figure 55 shows frequency of CD3+ cells, NK cells, CD8+ cells, and CD4+ cells for all cancer types using IL-2 +/- TME-S without (white bars) and with (black bars) time delay. Refer to table 43 for the specific stimulator of each group. Statistics performed by two-tailed Mann-Whitney U test. p>0.05 was considered non-significant.

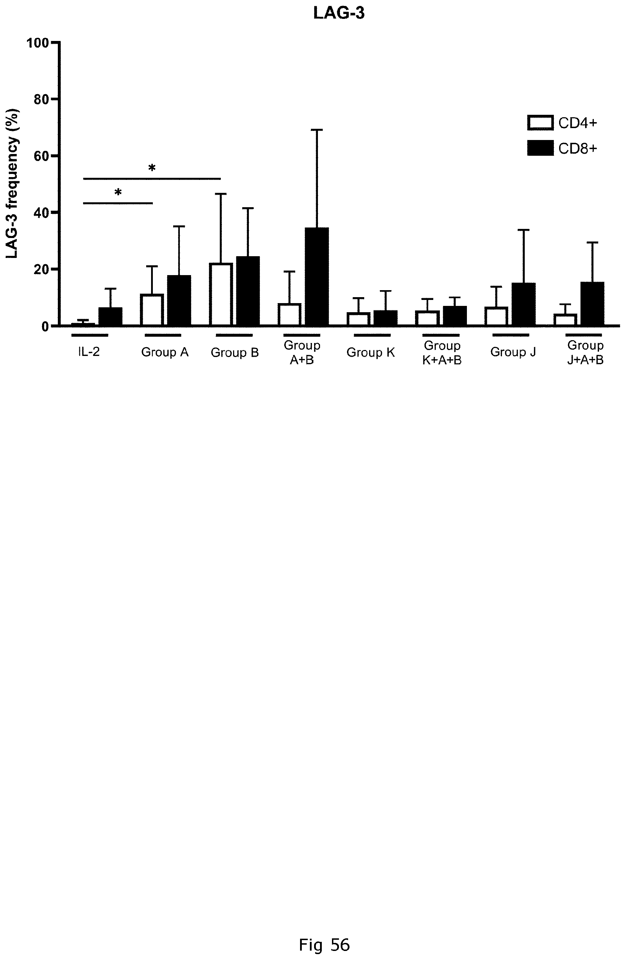

Figure 56 shows the frequency of LAG-3 on CD4+ cells (white bars) or CD8+ cells (black bars) for all cancer types using IL-2 +/- TME-S. Refer to table 43 for the specific stimulator of each group. Statistics performed by two-tailed Mann-Whitney U test. p>0.05 was considered non-significant, *p<0.05.

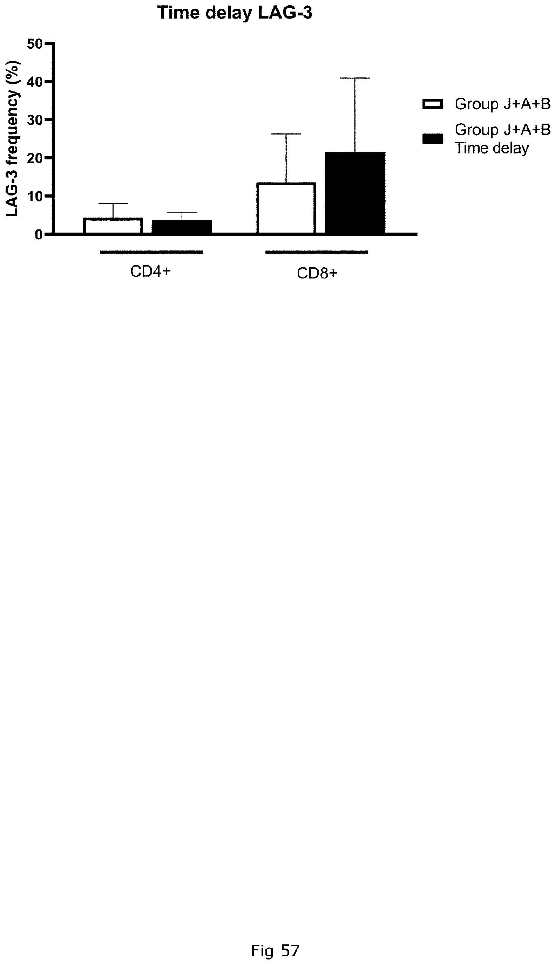

Figure 57 shows the frequency of LAG-3 on CD4+ cells or CD8+ cells for all cancer types using IL-2 +/- TME-S without (white bars) or with (black bars) time delay. Refer to table 43 for the specific stimulator of each group. Statistics performed by two-tailed Mann-Whitney U test. p>0.05 was considered non-significant.

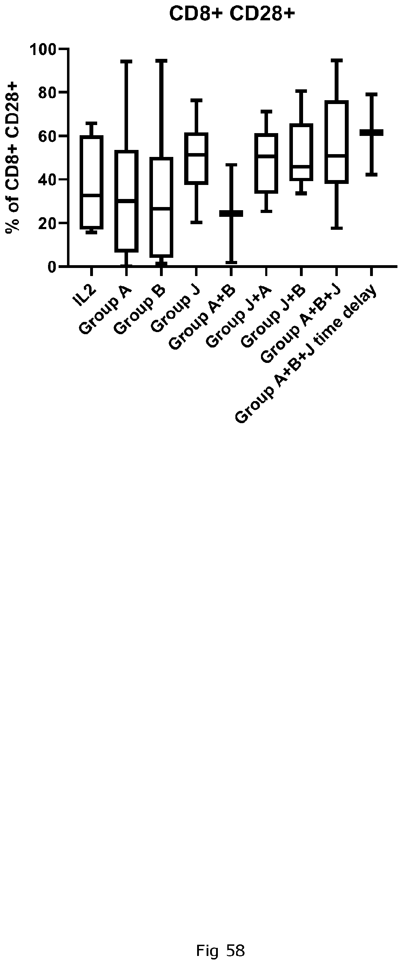

Figure 58 shows frequency of CD28 on CD8+ cells for all cancer types using IL-2 +/- TME-S. Refer to table 43 for the specific stimulator of each group. Statistics performed by two-tailed Mann-Whitney U test. p>0.05 was considered non-significant.

### EXAMPLES

### Example 1 - one or more immune modulators reinvigorate exhausted T-cells ex vivo

Step 1.1: Effect of single immune modulators
a. Resected TIL-containing tumor tissue from various patients is dissected 30-50 tumor fragments per cm³ tissue and transferred into 24-well cell culture plates. 2 mL of cell culture medium containing 6000 IU/mL IL-2 and either none (baseline) or a low, mid, or high concentration of each of the immune-modulators listed in Table 1.
b. The cell culture plates are incubated at 37°C, 5% CO₂ where cell culture medium is changed frequently. Cell cultures should not increase 1.5 × 10⁶ cells per well and should be split into new wells.
c. After a number of days, cells are harvested, cells are counted to determine amount, and analyzed by flowcytometry viability and phenotype

Step 1.2 effect of PD1 co-blockade and/or blockade/stimulation
a. As PD1 blockade is clearly identified as key pathway to reinvigorate exhausted T-cells, a new experiment including IL-2, optimal concentration of PD1 and the remaining immune modulators listed in Table 1, and the specific combinations with IL-2 listed in Tables 2-21 is setup and performed as above.

Step 1.3 Effect of CTLA4 co-blockade and/or blockade/stimulation
a. As CTLA4 blockade is clearly identified as key pathways to reinvigorate exhausted T-cells, a new experiment including IL-2, optimal concentration of PD1 and the remaining immune modulators listed in Table 1, and the specific combinations with IL-2 listed in Tables 22-41 is setup and performed as above.

Step 2: possibly further finetune concentration of immune modulators

Step 3: Understanding of combinatorial effects
a. A new experiment is setup in a similar way using the best performing immune modulators at the optimal concentration from the first experiment in a combinatorial approach to determine possible synergistic effects by adding several immune modulators simultaneously with the same readout as described above.
b. The above is run in several iterations eventually revealing combinations with a shortened time, a higher expansion rate and/or improved phenotype

Step 4: validation of optimal combination in patients versus standard TIL manufacturing protocol
a. Initial TIL culture expansion is run in parallel in a number of TIL therapy eligible patients to validate the effects on a real patient setting

### Example 2 - "Young" tumor-infiltrating lymphocytes (TILs) with TME stimulators

This example demonstrates the manufacturing process for generation of "young" tumor-infiltrating lymphocytes (TILs) with TME stimulators.

Tumor material of various histologies was obtained from commercial sources. Fourteen independent patient tumors or tumor digests were obtained (3 ovarian cancer, 3 metastatic melanoma, 3 head and neck cancer, 2 lung cancer, 2 colorectal cancer, 1 cervical cancer; Table 42). Cryopreserved or fresh tumor material was shipped to Cbio A/S in sterile freezing or transport medium. The tumor material was handled in a laminar flow hood to maintain sterile conditions.

TILs were prepared as previously described in detail in the standard TIL manufacturing protocol (Friese, C. et al., CTLA-4 blockade boosts the expansion of tumor-reactive CD8+ tumor-infiltrating lymphocytes in ovarian cancer. Sci Rep 10, 3914 (2020); Jin, J. et al., Simplified Method of the Growth of Human Tumor Infiltrating Lymphocytes in Gas-permeable Flasks to Numbers Needed for Patient Treatment, Journal of Immunotherapy, 35 - Issue 3 (2012)). Briefly, TIL cultures were set up using tumor fragments or tumor digest. The tumors were divided into 1-3 mm³ fragments and placed into a G-Rex 6-well plate (WilsonWolf; 5 fragments per well) with 10 ml complete medium (CM) including 6000 IU/mL IL-2 (6000 IU/ml, Clinigen) only (baseline) or in combination with TME stimulators in low, medium, high, or very high concentrations of each of the PD-1/PD-L1 antagonists (group A), CTLA-4 antagonist (group B), LAG-3 antagonist (group C), TIGIT antagonist (group D), 4-1 BB agonist together with anti-CD3 (group J) and CD28 agonist (group K) listed in Table 43, in a humidified 37°C incubator with 5% CO₂. CM was added every 4-5 days until a total volume of 40 ml was reached. Subsequently, half of the medium was removed and replaced with CM and IL-2 every 4-5 days. TIL cultures from tumor digest were initiated by culturing single-cell suspensions (5×10⁵/ml) obtained by overnight enzymatic digestion in flat-bottom 96-well plates in 250 µL CM and IL-2 (6000 IU/ml, Clinigen) in a humidified 37°C incubator with 5% CO₂. Half of the medium was removed and replaced with CM every 2-3 days.

CM consisted of RPMI1640 with GlutaMAX, 25 mM HEPES pH 7.2 (Gibco), 10% heat-inactivated human AB serum (Sigma-Aldrich), 100 U/mL penicillin, 100 µg/mL streptomycin (Gibco), and 1.25 µg/ml Fungizone (Bristol-Myers Squibb).

This example demonstrates the generation of "young" tumor-infiltrating lymphocytes (TILs) with TME stimulators having an age of 10-28 days.

### Example 3 - Phenotype analysis of "young" TIL cultures with TME stimulators

This example demonstrates the phenotype analysis of "young" TIL cultures with TME stimulators performed as described in example 2.

When cultures designated for young TIL generation were harvested, their phenotype was assessed by flow cytometry.

TIL phenotype was determined by assessment of the viability and the CD3+ subset, the CD3+CD8+ subset, the CD3+CD4+ subset and the NK subset in both frequency and absolute cell count. Additionally, differentiation status, activation status, the expression of exhaustion markers and senescence of TILs were assessed. Flow cytometry was conducted using the following markers:
TIL Panel 1: CD3, CD4, CD8, CD45RA, CD56, CCR7, FVS780, BTLA, LAG-3, PD-1, TIM-3
TIL Panel 2: CD3, CD4, CD8, CD45RA, CD56, CCR7, FVS780, CD-27, CD28, CD57, CD69

Briefly, about 0.5×10⁶ young TILs per panel were washed and then incubated with titrated antibodies (BD Biosciences, Table 45) and Brilliant Stain Buffer (BD Biosciences) for 30 min at 4°C. Cells were washed twice with PBS and directly analyzed by flow cytometry (CytoFLEX, Beckman Coulter).

This example demonstrates the phenotype analysis of "young" TIL cultures with TME stimulators.

### Example 4 - TME-stimulators increased the success rate of TIL expansion ex vivo

This example demonstrated that the success rate of TIL expansion ex vivo was increased, when TME stimulators were added to the culture medium when TIL cultures were initiated performed as described in example 2.

The success rate of TIL expansion was investigated by determining cell number per tumor fragment when harvesting TIL cultures. 5×10⁴ TILs/fragment was set as a threshold for successful TIL culture.

Determining the success rate of TIL expansion demonstrated that the success rates of TIL cultures were increased when TME stimulators were added to the "young" TIL cultures (avelumab 68%, relatlimab 70%, tiragolumab 76.5%, pembrolizumab 82.1%, ipilimumab 88.5%, theralizumab 90.9%, nivolumab 92.3%, and urelumab/OKT3 100%) compared to baseline cultures 61.5%, illustrated in figure 2 and 3.

Grouping the TME stimulators according to their targets, the example also demonstrated that adding inhibitors from group C (70%, LAG-3 inhibitors), group A (76.3%, including inhibitors of PD1 and its ligand PD-L1), group D (76.5%, TIGIT inhibitors), group B (88.5%, inhibitors of CTLA-4 and ligand), group K (90.9%, CD28 agonist) and group J (96.3%, 4-1BB agonist together with anti-CD3) also increased the success rate of TIL cultures compared to baseline cultures 61.5%, illustrated in figure 4 and 5.

This example demonstrates that the success rate of TIL expansion ex vivo was increased, when TME stimulators were added to the culture medium when TIL cultures were initiated as compared to the standard TIL manufacturing protocol.

### Example 5 - Checkpoint blockade or co-stimulation increased the TIL yield and reduced culture time of TILs

This example demonstrated that the TIL yield was increased and the culture time of TILs was reduced, when TME stimulators were added to the culture medium when TIL cultures were initiated, performed as described in example 2.

The TIL yield and the culture time of TILs were investigated when harvesting TIL cultures. This analysis demonstrated that the TIL yield increased, and the culture time decreased, when TME stimulators were added to the culture medium when TIL cultures were initiated compared to TILs cultured in IL-2 alone (figure 6). Here it was shown that TME stimulators from groups K and J accelerated young TIL culture time alone - but also that stimulators from groups A, B and C induced faster growth rates in a similar manner as compared to the standard young TIL protocol. Especially, combining stimulators from group J with either A and/or B in double or triple combinations further accelerated the growth rate.

This example demonstrated that the TIL yield was increased and the culture time of TILs was reduced, when TME stimulators were added to the culture medium, when TIL cultures were initiated as compared to the standard TIL manufacturing protocol.

### Example 6 - Different concentrations of TME stimulators induced TIL expansion ex vivo

This example performed as described in example 2 demonstrated that the TIL yield was increased, when TME stimulators were added to the culture medium in different concentrations, when TIL cultures from various tumor types were initiated.

The TIL yield expansion was investigated when harvesting TIL cultures. The first analysis in figure 7 demonstrated that TIL expansion increased 1.15-fold, 1.85-fold, and 1.53-fold compared to IL-2 alone, when a low, a medium or a high concentration of CTLA-4 antagonist, ipilimumab (group B) was added to the culture medium, when TIL cultures were initiated. This example demonstrated that the TIL yield was increased, when CTLA-4 antagonist (group B) was added to the culture medium when TIL cultures were initiated in a dose-dependent manner. Despite that the CTLA-4 antagonist used (ipilimumab) is also known to deplete CTLA-4 expressing T cells by antibody-dependent cellular cytotoxicity (ADCC), this novel finding suggested how depleting certain subsets of T cells might have enabled faster growth rates for other T cells thereby improving the overall TIL yield.

In figure 8, it was demonstrated that when a low, a medium or a high concentration of a PD-1 antagonist, pembrolizumab (group A) was added to the culture medium when TIL cultures were initiated, TIL yield showed a tendency towards a dose-dependent increase compared to the standard young TIL protocol.

In figures 9-12 a similar dose-dependent effect in TIL yield compared to standard young TIL protocol is illustrated for a 4-1BB agonist, urelumab together with anti-CD3 (OKT3) (group J), a CD28 agonist, theralizumab (group K), a PD-L1 inhibitor, avelumab (group A - the ligand for PD-1), and another PD-1 inhibitor, nivolumab (group A), respectively.

This example 6 demonstrates how different concentrations of TME stimulators influenced TIL growth in a dose dependent manner.

### Example 7 - TME-stimulators as a whole and from different groupings enhances TIL growth

Example 7 illustrated in figure 13 demonstrated that adding TME-stimulators to the standard young TIL protocol performed as described in example 2 significantly enhanced TIL growth which resulted in higher numbers of viable cells per tumor fragment. This was illustrated using a representative number of tumor fragments from various solid cancers including ovarian, head and neck, colorectal, melanoma, cervical, colorectal, and lung cancer.

Breaking the TME stimulators up into the underlying subgroupings, the example also demonstrated that adding inhibitors from group A (including inhibitors of PD1 and its ligand PD-L1), group B (inhibitors of CTLA-4), group J (4-1BB agonist together with anti-CD3) and group K (CD28 agonist) also significantly increased TIL growth. Although not significant in this example there was a tendency that adding TME stimulators from groups C (LAG-3 inhibitors) and D (TIGIT inhibitors) also improved TIL growth.

In figures 14-19, the effect of adding TME stimulators to the initial TIL cultures from the same groupings as above was illustrated in cervical, melanoma, ovarian, head and neck, lung, and colorectal cancer, respectively. Although not significant in all conditions, the effect illustrated a similar pattern between cancers as the pan-tumor example in figure 13 - although the effect and magnitude of the individual stimulators in different cancers was varying.

Summing up this example, adding TME stimulators to the young TIL processing step provided a novel improvement over the existing standard TIL protocol that allowed for a faster TIL therapy manufacturing protocol.

### Example 8: TIL stimulator agonists, antagonists, T-cell depleting, T-cell reinvigorating, and stimulators of CD28 family origin significantly increased TIL growth rates

Example 8 illustrated in figure 20 demonstrates that adding TME-stimulators to the standard young TIL protocol performed as described in example 2 significantly enhanced TIL growth which resulted in higher numbers of viable cells per tumor fragment. This was illustrated using a representative number of tumor fragments from various solid cancers including ovarian, head and neck, colorectal, melanoma, cervicaland lung cancer.

Breaking the TME stimulators up into the subgroupings according to their functionality, the example also demonstrated that both T-cell antagonists, agonists, reinvigorating, depleting and members of the CD28 family of receptors all had a significant effect on TIL growth. Whereas a representative amount of different TME antagonists exemplified here including 2 different PD-1 inhibitors (pembrolizumab and nivolumab), 2 different PD-L1 inhibitors (avelumab and durvalumab), a CTLA-4 inhibitor (ipilimumab), a TIGIT inhibitor (tiragolumab), showed a 3-5-fold increase over the standard young TIL process, TME agonists here exemplified by stimulators targeting 4-1BB (urelumab together with anti-CD3 (OKT3)) and CD28 (theralizumab) seemed to further speed up growth.

Further dividing the TME antagonists into whether they allow for depletion of regulatory T cells through antibody-dependent cellular toxicity (ADCC) such as ipilimumab and tiragolumab or only allow for T-cell reinvigoration through checkpoint inhibition also both demonstrated a significant improvement in TIL growth rates over standard young TIL protocol conditions as illustrated in figure 20.

Looking specifically on TME stimulators originating from the CD28 family of proteins exemplified here by inhibitors of PD-1, CTLA-4 and CD28 or their ligands originating from the B7-family of proteins exemplified here by two different inhibitors of PD-L1, it was demonstrated that they also significantly enhanced TIL growth as compared to the standard young TIL protocol. Although not shown here, other receptors expressed on T cells originating from the CD28 protein family such as BTLA and ICOS could have a similar growth stimulating effect for young TIL cultures.

In figures 21-26, the effect of adding TME stimulators to the initial TIL cultures from the same groupings as above in this example was illustrated in melanoma, ovarian, lung, cervical, colorectal, and head and neck cancers, respectively. Although not significant in all conditions, the effect illustrated a similar pattern between cancers as the pan-tumor example in figure 20 - although the effect and magnitude of the individual stimulators in different cancers was varying.

Summing up this example, adding TME stimulators that were either antagonizing receptors expressed on T cells (or their ligands), agonizing receptors expressed on T-cells , reinvigorating exhausted T-cells (or their ligands), depleting regulatory T-cells and/or targeting receptors expressed on T cells originating from the CD28 family (or their ligands originating from the B7 family of proteins) to the young TIL processing step provided a novel improvement over the existing standard TIL protocol that allowed for a faster TIL therapy manufacturing protocol.

### Example 9 - TME stimulator antagonists targeting receptors expressed on T cells or their ligands demonstrated a similar TIL growth stimulating effect

Example 9 illustrated in figure 27 demonstrated that adding TME stimulators to the standard young TIL protocol as performed in example 2 significantly enhanced TIL growth which resulted in higher numbers of viable cells per tumor fragment by either stimulating a receptor expressed on T cells (in this case PD-1) or its ligand (PD-L1) expressed on tumor cells and other cells in the tumor microenvironment. This was illustrated using a representative number of tumor fragments from various solid cancers including ovarian, head and neck, colorectal, melanoma, cervicaland lung cancer. This was an example of how inhibiting receptors expressed on T-cells known to downregulate T-cell activity had a similar effect as inhibiting their ligands and that both could generate a similar effect on TIL growth that reinvigorated exhausted T cells and increased young TIL growth. The PD1/PD-L1 example thereby exemplified a more general tendency for receptor/ligand inhibition for other receptors expressed on T cells such as CTLA-4, LAG-3, TIGIT, KIR, TIM-3, BTLA and their ligands.

In figures 28-33, the effect of adding TME stimulators to the initial TIL cultures from either the standard TIL manufacturing protocol, the PD-1 group or the PD-L1 group was illustrated in ovarian, melanoma, lung, head and neck, colorectal, and cervical cancer, respectively. Although not significant in all conditions, the effect illustrated a similar pattern between cancers as the pan-tumor PD-1/PD-L1 example in figure 27.

### Example 10 - TME stimulators from different manufacturers demonstrated a similar TIL growth stimulating effect

Example 10 illustrated in figure 34 demonstrates that adding TME stimulators from different manufactures had a similar effect when added to the standard young TIL protocol performed as described in example 2, which significantly enhanced TIL growth and resulted in higher numbers of viable cells per tumor fragment independent of the manufacturing origin. This was illustrated using a representative number of tumor fragments from various solid cancers including ovarian, melanoma, head and neck, and cervical cancer.

Two PD-1 inhibitors (pembrolizumab, Merck Sharp Dome and nivolumab, Bristol Myers Squibb) and two PD-L1 inhibitors (avelumab, Merck KgaA and durvalumab, AstraZeneca) were tested in this example. All the different TME stimulators showed significant improvement over the standard young TIL protocol in the ability to accelerate TIL growth. There was a tendency that the four different antibodies showed similar effects as compared to group A as well as between the individual inhibitors.

This was an example of how TME stimulators from various manufacturers in general were interchangeable and could be used to optimize the young TIL manufacturing process.

In figures 35-38, the effect of adding inhibitors from group A from different manufacturers to the initial TIL cultures was illustrated in ovarian, melanoma, head and neck, and cervical cancer, respectively. Although not significant in all conditions, the effect illustrated a similar pattern between cancers as the pan-tumor example in figure 34.

### Example 11 - Combinations of TME stimulators further enhanced young TIL growth

This example performed as described in example 2 demonstrated that the TIL yield was increased compared to the standard TIL manufacturing protocol, when TME stimulators in various combinations were added to the culture medium, when TIL cultures from various tumor types were initiated.

The TIL yield was investigated when harvesting TIL cultures. The first analysis illustrated in figure 39 demonstrated that TIL expansion increased significantly when adding TME stimulators from group A (PD-1 inhibitor or its ligands), group B (CTLA-4 inhibitor), or when adding both group A and B as compared to the standard young TIL protocol. There was a tendency although not significant that co-adding TME stimulators from group A and B further improved TIL growth rates.

In another analysis illustrated in figure 40, a 4-1BB agonist, urelumab together with anti-CD3 (OKT3) (group J) either alone, or in combination with a CTLA-4 inhibitor, ipilimumab (group B), or in combination with a PD-1 inhibitor, pembrolizumab (group A), or in a triple combination of both ipilimumab and pembrolizumab all showed a very strong TIL growth in viable cells per tumor fragment from various cancers.

In figure 41 the effect of a time-delay for adding urelumab/OKT3 to the young TIL culture was investigated. Here, the triple combination of urelumab/OKT3, ipilimumab and pembrolizumab added to the initial young TIL culture on day zero (left side of the figure) was compared to adding ipilimumab and pembrolizumab and waiting 2 days to add urelumab/OKT3 in a time-delay (right side of the figure). There was no significant difference between the two conditions and both conditions showed very strong TIL growth.

In Figure 42, the effect of adding theralizumab alone (left side of the figure) or in combination with both ipilimumab and pembrolizumab to the initial young TIL culture was investigated. Although not significant, there was a tendency that the triple combination induced a faster growth rate in young TILs as compared to theralizumab alone.

### Example 12 - TME-stimulators alone or in combination increased the success rate of TIL expansion ex vivo

This example demonstrated that the success rate of TIL expansion ex vivo was increased, when TME stimulators were added to the culture medium during TIL culture initiation performed as described in example 2.

The success rate of TIL expansion was investigated by determining cell number per tumor fragment when harvesting TIL cultures. 5×10⁴ TILs/fragment was set as a threshold for successful TIL culture.

Determining the success rate of TIL expansion demonstrated that the success rates of TIL cultures were increased when TME stimulators from different groups were added to the "young" TIL cultures either alone or in combinations (group A 76.3%, group B 88.5%, group J 100.0%, group A+B 83.3%, group B+J 100.0%, group A+J 100.0%, and group A+B+J triple combo 96.0%) compared to baseline cultures 61.5%, illustrated in figure 43.

This example demonstrated that the success rate of TIL expansion ex vivo was increased, when TME stimulators alone or in combinations were added to the culture medium when TIL cultures were initiated compared to the standard TIL manufacturing protocol.

### Example 13 - TME-stimulators as a whole, from different groupings and in combinations enhance the frequency and the number of T cells

Example 13 illustrated in figure 44-47 demonstrated that adding TME-stimulators to the standard young TIL protocol performed as described in example 2 and staining T cells using anti-CD3 flow cytometry antibody as described in example 3 significantly enhanced TIL growth which resulted in an increased frequency of T cells (figure 44) and higher numbers of viable T cells per tumor fragment (figure 46) compared to IL-2 alone. This was illustrated using a representative number of tumor fragments from various solid cancers including ovarian, head and neck, colorectal, melanoma, cervical, colorectal, and lung cancer.

Breaking the TME stimulators up into the underlying subgroupings, the example also demonstrated that adding inhibitors from group A (including inhibitors of PD1 and its ligand PD-L1) or group B (inhibitors of CTLA-4 and ligand), also significantly increased the frequency of T cells compared to IL-2 alone (figure 45) by reinvigorating T cells. As T cells that have a higher affinity to tumor antigens might have an increased tendency to get exhausted, this can lead to the expansion of more tumor-reactive T cells. Furthermore, the example also demonstrated that adding TME stimulators from group B also significantly increased the frequency of T cells compared to group J (4-1BB agonist together with anti-CD3) or a combination of group J, A and B (figure 45).

Breaking the TME stimulators up into the underlying subgroupings, the example also demonstrated that adding inhibitors from group A (including inhibitors of PD1 and its ligand PD-L1), group B (inhibitors of CTLA-4 and ligand), group K (CD28 agonistsand group J (4-1 BB agonist together with anti-CD3) also significantly increased the number of viable T cells per tumor fragment compared to IL-2 alone (figure 47). Furthermore, the example also demonstrated that adding combinations of TME stimulators from group J, A and B also significantly increased the number of viable T cells per tumor fragment compared to group A, group B or a combination of group A and B (figure 47). Furthermore, there is a tendency for more viable T cells per fragment when adding combinations of TME stimulators from group J, A and B compared to adding TME stimulators from group J alone (figure 47).

Summing up this example, adding TME stimulators to the young TIL manufacturing step provided a novel improvement over the existing standard TIL protocol that allowed for generation of a TIL product containing an increased frequency of T cells and, an increased number of viable T cells.

### Example 14 - TME-stimulators as a whole, from different groupings and in combinations maintain the frequency of effector-memory T cells

Example 14 illustrated in figure 48 demonstrated that adding TME-stimulators to the standard young TIL manufacturing protocol performed as described in example 2 and staining T cells using anti-CD3, anti-CD45RA and anti-CCR7 flow cytometry antibodies as described in example 3 significantly increased the frequency of effector-memory T cells in CD4+ T cells and slightly increased the frequency of effector memory T cells in CD8+ T cells compared to IL-2 alone. This was illustrated using a representative number of tumor fragments from various solid cancers including ovarian, head and neck, colorectal, melanoma, cervical, colorectal, and lung cancer.

Summing up this example, adding TME stimulators to the young TIL manufacturing step provided a novel improvement over the existing standard TIL protocol that allowed for generation of a TIL product containing a comparable frequency of effector memory T cells.

### Example 15 - TME-stimulators in combination enhance the frequency and number of CD8+ T cells

Example 15 illustrated in figure 49-50 demonstrated that adding a combination of TME stimulators from group J (4-1BB agonist together with anti-CD3), group A (including inhibitors of PD1 and its ligand PD-L1) and group B (inhibitors of CTLA-4 and ligand) to the standard young TIL manufacturing protocol performed as described in example 2 and staining T cells using anti-CD3 and anti-CD8 flow cytometry antibodies as described in example 3 significantly enhanced CD8+ T-cell growth which resulted in a significantly increased frequency (figure 49) and number (figure 50) of CD8+ T cells compared to IL-2 alone. This was illustrated using a representative number of tumor fragments from various solid cancers including ovarian, head and neck, colorectal, melanoma, cervical, colorectal, and lung cancer.

The example also demonstrated that adding a combination of TME stimulators from group J (4-1BB agonist together with anti-CD3), group A (including inhibitors of PD1 and its ligand PD-L1) and group B (inhibitors of CTLA-4) to the standard young TIL showed a tendency to enhance CD8+ T cells growth compared to adding TME stimulators from group A, group B or group J alone (figure 49). Furthermore, the example also demonstrated that adding a combination of TME stimulators from group J, group A and group B significantly increased the number of viable CD8+ T cells compared to group A or group B alone and showed a tendency to increase the number of viable CD8+ T cells compared to group J alone (figure 50).

An increased frequency of CD8+ T cells in the TIL infusion product has previously been associated with beneficial clinical outcome of TIL therapy in patients with metastatic melanoma (Radvanyi, L. G. et al., Specific lymphocyte subsets predict response to adoptive cell therapy using expanded autologous tumor-infiltrating lymphocytes in metastatic melanoma patients. Clin. Cancer Res. 18, 6758-6770 (2012)). Thus, methods increasing CD8+ T-cell frequency could induce clinical responses in cancer patients that do not respond to TILs manufactured using the standard TIL protocol.

Summing up this example, adding TME stimulators alone and in combinations to the young TIL processing step provided a novel improvement over the existing standard TIL manufacturing protocol that allowed for generation of a TIL product containing an increased frequency of CD8+ T cells.

### Example 16 - TME-stimulators in combination reduce the frequency of CD4+ T cells

Example 16 illustrated in figure 51 demonstrated that adding a combination of TME stimulators from group J (4-1BB agonist together with anti-CD3), group A (including inhibitors of PD1 and its ligand PD-L1) and group B (inhibitors of CTLA-4) to the standard young TIL protocol performed as described in example 2 and staining T cells using anti-CD3 and anti-CD4 flow cytometry antibodies as described in example 3 significantly reduced CD4+ T-cell growth which resulted in a significantly reduced frequency of CD4+ T cells compared to IL-2 alone (figure 51). This was illustrated using a representative number of tumor fragments from various solid cancers including ovarian, head and neck, colorectal, melanoma, cervical, colorectal, and lung cancer.

Summing up this example, adding TME stimulators to the young TIL processing step provided a novel improvement over the existing standard TIL manufacturing protocol that allowed for generation of a TIL product containing a reduced frequency of CD4+ T cells.

### Example 17 - TME-stimulators from different groups reduce the frequency of NK cells

Example 17 illustrated in figure 52 demonstrated that adding TME stimulators from group A (including inhibitors of PD1 and its ligand PD-L1) or group B (inhibitors of CTLA-4) to the standard young TIL protocol performed as described in example 2 and staining NK cells using anti-CD3 and anti-CD56 flow cytometry antibodies as described in example 3 significantly reinvigorated T cells resulting in a reduced frequency of NK cells compared to IL-2 alone. This could lead to the expansion of more tumor-reactive T cells. This was illustrated using a representative number of tumor fragments from various solid cancers including ovarian, head and neck, colorectal, melanoma, cervical, colorectal, and lung cancer.

Furthermore, the example demonstrated that adding TME stimulators from group B showed a tendency to a reduced NK cell frequency compared to group J (4-1BB agonist together with anti-CD3) and group K (CD28 agonists).

Summing up this example, adding TME stimulators to the young TIL processing step provided a novel improvement over the existing standard TIL manufacturing protocol that allowed for generation of a TIL product containing a reduced frequency of NK cells.

### Example 18 - TME-stimulators from different groups or in combination affect the frequency of NK and T cells in total and CD8+ T cells specifically

Example 18 illustrated in figure 53 and figure 54 demonstrated that adding urelumab/OKT3 (group J) alone or in combination with pembrolizumab (group A) or ipilimumab and pembrolizumab (group B+A) to the standard young TIL protocol performed as described in example 2 and staining NK cells and T cells using anti-CD3, anti-CD56 and anti-CD8 flow cytometry antibodies as described in example 3 reinvigorated NK cells resulting in an increased frequency of NK cells and a reduced frequency of T cells compared to IL-2 alone (figure 53) in one head and neck cancer sample. This reinvigoration of NK cells was inhibited by adding ipilimumab in addition to urelumab/OKT3 to the TIL culture reinvigorating T cells resulting in a decreased frequency of NK cells and an increased frequency of T cells compared to adding urelumab/OKT3 (group J) alone or in combination with pembrolizumab (group A) or ipilimumab and pembrolizumab (group B+A) to the standard young TIL manufacturing protocol. This was illustrated using a representative sample of head and neck cancer.

Furthermore, the example demonstrated that adding urelumab/OKT3 (group J) and ipilimumab (group B) reduced the CD8+ T cell frequency compared to urelumab/OKT3 alone, urelumab/OKT3 and pembrolizumab (group A) and urelumab/OKT3, ipilimumab and pembrolizumab (figure 54).

Therefore, the example demonstrated that adding urelumab/OKT3 (group J), ipilimumab (group B) and pembrolizumab (group A) could be favorable compared to urelumab/OKT3 and ipilimumab only.

Summing up this example, adding TME stimulators to the young TIL processing step provided a novel improvement over the existing standard TIL manufacturing protocol that allowed for generation of a TIL product containing a reduced frequency of NK cells but an increased frequency of CD8+ T cells.

### Example 19 - TME-stimulators in combination added with time delay enhance the frequency of CD3+ and CD8+ T cells and reduce the frequency of NK cells and CD4+ T cells

Example 19 illustrated in figure 55 demonstrated that adding a combination of TME stimulators from group A (including inhibitors of PD1 and its ligand PD-L1) and group B (inhibitors of CTLA-4 on day 0 and a TME stimulator from group J (4-1BB agonist together with anti-CD3) on day 2 to the standard young TIL protocol performed as described in example 2 and staining T cells using anti-CD3, anti-CD56, anti-CD8 and anti-CD4 flow cytometry antibodies as described in example 3 enhanced T cell and CD8+ T-cell growth which resulted in an increased frequency of T cells in total (CD3+) and CD8+ T cells (figure 55) and reduced NK cell and CD4+ T cell frequency compared to the addition of TME stimulators from the same groups (A, B and J) on day 0. This was illustrated using a representative number of tumor fragments from various solid cancers including ovarian, head and neck, colorectal, melanoma, cervical, colorectal, and lung cancer. The time delay seemed to allow for a stronger effect of the group A and B antagonists in depleting regulatory CD-4+ T-cells and reinvigorating CD-8+ T-cells before the addition of the group J agonists.

Summing up this example, adding TME stimulators with a time delay to the young TIL processing step provided a novel improvement over the existing standard TIL manufacturing protocol that allowed for generation of a TIL product containing an increased frequency of T cells in total, CD8+ T cells and a reduced frequency of NK cells and CD4+ T cells.

### Example 20 - TME-stimulators alone or in combination enhance the frequency of LAG3+ T cells

Example 20 illustrated in figure 56 and figure 57 demonstrated that adding TME stimulators alone or in combination from group A (including inhibitors of PD1 and its ligand PD-L1), group B (inhibitors of CTLA-4), group K (CD28 agonists) or group J (4-1BB agonist together with anti-CD3) to the standard young TIL protocol performed as described in example 2 and staining T cells using anti-CD3, anti-CD8, anti-CD4 and anti-LAG-3 flow cytometry antibodies as described in example 3 enhanced reinvigoration of tumor-specific T cells which resulted in increased frequency of LAG-3+ T cells in both CD4+ and CD8+ T cells (figure 56) compared to IL-2 alone. Especially the frequency of CD4+ LAG-3+ T cells were significantly higher when adding TME stimulators from group A and B. This was illustrated using a representative number of tumor fragments from various solid cancers including ovarian, head and neck, colorectal, melanoma, cervical, colorectal, and lung cancer.

Furthermore, the example demonstrated that adding a combination of TME stimulators in group A (including inhibitors of PD1 and its ligand PD-L1), group B (inhibitors of CTLA-4), or group J (4-1 BB agonist together with anti-CD3) in a time delay as described in example 19 compared to adding TME stimulators in combination from group A, B and J showed a tendency to increased reinvigoration of tumor-specific CD8+ T cells resulting in an increased frequency of CD8+ LAG-3+ T cells (figure 57).

Summing up this example, adding TME stimulators to the young TIL processing step provided a novel improvement over the existing standard TIL manufacturing protocol that allowed for generation of a TIL product containing an increased frequency of tumor-specific LAG-3+ T cells. As LAG-3 is known to be a marker for T-cell exhaustion and that T cells that have a higher affinity to tumor antigens generally have an increased tendency to get exhausted, expansion of CD8+ LAG-3+ T cell clones can lead to a higher proportion of tumor-reactive T-cells possibly leading to an improved clinical outcome of this novel approach to TIL therapy.

### Example 21 - TME-stimulators increased the frequency of CDS T-cells with a younger phenotype being CD28+

Example 21 illustrated in figure 58 demonstrated that adding TME stimulators alone or in combination from group A (including inhibitors of PD1 and its ligand PD-L1), group B (inhibitors of CTLA-4), group K (CD28 agonists) or group J (4-1BB agonist together with anti-CD3) to the standard young TIL protocol performed as described in example 2 and staining T cells using anti-CD3, anti-CD8 and anti-CD28 flow cytometry antibodies as described in example 3 enhanced expansion of T cells with a younger phenotype which resulted in an increased frequency of CD8+ CD28+ T cells (figure 58) compared to IL-2 alone. This was illustrated using a representative number of tumor fragments from various solid cancers including ovarian, head and neck, colorectal, melanoma, cervical, colorectal, and lung cancer.

Furthermore, the example demonstrated that adding a combination of TME stimulators from group A (including inhibitors of PD1 and its ligand PD-L1), group B (inhibitors of CTLA-4), or group J (4-1BB agonist together with anti-CD3) compared to adding TME stimulators from group A or group B alone showed a tendency to increased expansion of T cells with a younger phenotype resulting in an increased frequency of CD8+ CD28+ T cells (figure 58).

Furthermore, the example demonstrates that adding a combination of TME stimulators from group A (including inhibitors of PD1 and its ligand PD-L1), group B (inhibitors of CTLA-4) and group J (4-1BB agonist together with anti-CD3) with time delay as described in example 19 compared to adding TME stimulators from group A or group B alone or a combination of TME stimulators from group A, group B and group J without time delay showed a tendency to increased expansion of T cells with a younger phenotype resulting in an increased frequency of CD8+ CD28+ T cells (figure 58).

Summing up this example, adding TME stimulators to the young TIL processing step provided a novel improvement over the existing standard TIL manufacturing protocol that allowed for generation of a TIL product containing an increased frequency of CD8+ T cells with a younger phenotype expressing CD28.

## Claims

1. Expanded tumor infiltrating lymphocytes (TILs) for use in treating a subject with cancer, the treatment comprising the steps of:
- (a) culturing autologous T cells by obtaining a first population of TILs from a tumor resected from a mammal
- (b) performing a first expansion by culturing the first population of TILs in a cell culture medium comprising IL-2 and one or more TME stimulators to produce a second population of TILs, wherein the one or more TME stimulators comprises at least a substance of group B selected from one or more from the group consisting of ipilimumab and tremelimumab,
- (c) performing a second expansion by supplementing the cell culture medium of the second population of TILs with additional IL-2, anti-CD3 antibody, and antigen presenting cells (APCs), to produce a third population of TILs, wherein the third population of TILs is a therapeutic population; and
- (d) after administering nonmyeloablative lymphodepleting chemotherapy, administering to the mammal the therapeutic population of T cells, whereupon the regression of the cancer in the mammal is promoted.

2. Expanded tumor infiltrating lymphocytes (TILs) for use in promoting regression of a cancer in a subject with cancer, the regression comprising the steps of:
- (a) culturing autologous T cells by obtaining a first population of TILs from a tumor resected from a mammal
- (b) performing a first expansion by culturing the first population of TILs in a cell culture medium comprising IL-2 and one or more TME stimulators to produce a second population of TILs, wherein the one or more TME stimulators comprises at least a substance of group B selected from one or more from the group consisting of ipilimumab and tremelimumab,
- (c) performing a second expansion by supplementing the cell culture medium of the second population of TILs with additional IL-2, anti-CD3 antibody, and antigen presenting cells (APCs), to produce a third population of TILs, wherein the third population of TILs is a therapeutic population; and
- (d) after administering nonmyeloablative lymphodepleting chemotherapy, administering to the mammal the therapeutic population of T cells, whereupon the regression of the cancer in the mammal is promoted.

3. A method for expanding tumor infiltrating lymphocytes (TILs) into a therapeutic population of TILs comprising:
- (a) culturing autologous T cells by obtaining a first population of TILs from a tumor resected from a mammal
- (b) performing a first expansion by culturing the first population of TILs in a cell culture medium comprising IL-2 and one or more TME stimulators to produce a second population of TILs, wherein the one or more TME stimulators comprise at least a substance of group B is selected from one or more from the group consisting of ipilimumab and tremelimumab, and
- (c) performing a second expansion by supplementing the cell culture medium of the second population of TILs with additional IL-2, anti-CD3 antibody, and antigen presenting cells (APCs), to produce a third population of TILs, wherein the third population of TILs is a therapeutic population.

4. The Expanded tumor infiltrating lymphocytes (TILs) for use according to any of claims 1-2, or the method of claim 3, wherein the one or more TME stimulators comprises at least a substance of group A selected from one or more from the group consisting of pembrolizumab, nivolumab, cemiplimab, sym021, atezolizumab, avelumab, and durvalumab, and a substance of group B selected from one or more from the group consisting of ipilimumab and tremelimumab.

5. The expanded tumor infiltrating lymphocytes (TILs) for use according to claim 4, or the method of claim 4, further comprising one or more substances of group J selected from one or more from the group consisting of urelumab and utomilumab.

6. The expanded tumor infiltrating lymphocytes (TILs) for use according to claim 5, or the method of claim 5, wherein group J is used in combination with an anti-CD3 substance.

7. The expanded tumor infiltrating lymphocytes (TILs) for use according to any of claims 1-2 or 4-6, or the method of any of claims 3-6, wherein the concentration of the substance is 0.1 µg/mL to 300 µg/mL, such as 1 µg/mL to 100 µg/mL, such as 10 µg/mL to 100 µg/mL, such as 1 µg/mL to 10 µg/mL, such as 2-20 µg/mL.

8. The expanded tumor infiltrating lymphocytes (TILs) for use according to any of claims 1-2 or 4-7, or the method of any of claims 3-7, wherein steps (a) through (b) are performed within a period of about 7 days to about 28 days.

9. The expanded tumor infiltrating lymphocytes (TILs) for use according to any of claims 1-2 or 4-8, or the method of any of claims 3-8, wherein step (c) is performed within a period of about 7 days to about 21 days.

10. The expanded tumor infiltrating lymphocytes (TILs) for use according to any of claims 1-2 or 4-9, or the method of any of claims 3-9, wherein the therapeutic population of T cells is used to treat a cancer type selected from the groups consisting of breast cancer, renal cell cancer, bladder cancer, melanoma, cervical cancer, gastric cancer, colorectal cancer, lung cancer, head and neck cancer, ovarian cancer, Hodgkin lymphoma, pancreatic cancer, liver cancer, and sarcomas.

11. The expanded tumor infiltrating lymphocytes (TILs) for use according to any of claims 1-2 or 4-10, or the method of any of claims 3-10, wherein step (c) results in 1 × 10⁷ to 1× 10¹² cells, such as 1 × 10⁸ to 5x 10⁹ cells, such as 1 × 10⁹ to 5x 10⁹ cells, such as 1 × 10⁸ to 5× 10¹⁰ cells, such as 1 × 10⁹ to 5× 10¹¹ cells.

12. The expanded tumor infiltrating lymphocytes (TILs) for use according to claims 1-2 or 4-11 or, the method of any of claims 3-11, wherein the anti-CD3 antibody is OKT3.

13. The expanded tumor infiltrating lymphocytes (TILs) for use according to claims 1-2 or 4-12, or the method of any of claims 3-12, wherein the TME stimulators are added together or in time lapse, *i.e.* one day apart, or such as 2, 3, 4, 5, 6 or 7 days apart.

14. The expanded tumor infiltrating lymphocytes (TILs) for use according to claims 1-2 or 4-13, or the method of any of claims 3-13, wherein the antigen-presenting cells (APCs) are selected from the group consisting of allogeneic feeder cells, PBMCs, and artificial antigen-presenting feeder cells.

## Patentansprüche

1. Expandierte tumorinfiltrierende Lymphozyten (TILs) zur Verwendung beim Behandeln einer Zielperson mit Krebs, wobei die Behandlung die folgenden Schritte umfasst:
(a) Züchten von autologen T-Zellen durch Erlangen einer ersten Population von TILs aus einem Tumor, der von einem Säugetier reseziert wurde
(b) Durchführen einer ersten Expansion durch Züchten der ersten Population von TILs in einem Zellkulturmedium, umfassend IL-2 und einen oder mehrere TME-Stimulatoren, um eine zweite Population von TILs zu erzeugen, wobei der eine oder die mehreren TME-Stimulatoren mindestens eine Substanz der Gruppe B, ausgewählt aus einer oder mehreren aus der Gruppe, bestehend aus Ipilimumab und Tremelimumab, umfasst,
(c) Durchführen einer zweiten Expansion durch Ergänzen des Zellkulturmediums der zweiten Population von TILs mit zusätzlichem IL-2, Anti-CD3-Antikörper und antigenpräsentierenden Zellen (APCs), um eine dritte Population von TILs zu erzeugen, wobei die dritte Population von TILs eine therapeutische Population ist; und
(d) nach Verabreichen einer nicht myeloablativen Chemotherapie zur Lymphodepletion, Verabreichen der therapeutischen Population von T-Zellen an das Säugetier, woraufhin die Regression des Krebses in dem Säugetier gefördert wird.

2. Expandierte tumorinfiltrierende Lymphozyten (TILs) zur Verwendung beim Fördern der Regression eines Krebses bei einer Zielperson mit Krebs, wobei die Regression die folgenden Schritte umfasst:
(a) Züchten von autologen T-Zellen durch Erlangen einer ersten Population von TILs aus einem Tumor, der von einem Säugetier reseziert wurde
(b) Durchführen einer ersten Expansion durch Züchten der ersten Population von TILs in einem Zellkulturmedium, umfassend IL-2 und einen oder mehrere TME-Stimulatoren, um eine zweite Population von TILs zu erzeugen, wobei der eine oder die mehreren TME-Stimulatoren mindestens eine Substanz der Gruppe B, ausgewählt aus einer oder mehreren aus der Gruppe, bestehend aus Ipilimumab und Tremelimumab, umfasst,
(c) Durchführen einer zweiten Expansion durch Ergänzen des Zellkulturmediums der zweiten Population von TILs mit zusätzlichem IL-2, Anti-CD3-Antikörper und antigenpräsentierenden Zellen (APCs), um eine dritte Population von TILs zu erzeugen, wobei die dritte Population von TILs eine therapeutische Population ist; und
(d) nach Verabreichen einer nicht myeloablativen Chemotherapie zur Lymphodepletion, Verabreichen der therapeutischen Population von T-Zellen an das Säugetier, woraufhin die Regression des Krebses in dem Säugetier gefördert wird.

3. Verfahren zum Expandieren von tumorinfiltrierenden Lymphozyten (TILs) zu einer therapeutischen Population von TILs, umfassend:
(a) Züchten von autologen T-Zellen durch Erlangen einer ersten Population von TILs aus einem Tumor, der von einem Säugetier reseziert wurde
(b) Durchführen einer ersten Expansion durch Züchten der ersten Population von TILs in einem Zellkulturmedium, umfassend IL-2 und einen oder mehrere TME-Stimulatoren, um eine zweite Population von TILs zu erzeugen, wobei der eine oder die mehreren TME-Stimulatoren mindestens eine Substanz der Gruppe B, ausgewählt aus einer oder mehreren aus der Gruppe, bestehend aus Ipilimumab und Tremelimumab, umfasst, und
(c) Durchführen einer zweiten Expansion durch Ergänzen des Zellkulturmediums der zweiten Population von TILs mit zusätzlichem IL-2, Anti-CD3-Antikörper und antigenpräsentierenden Zellen (APCs), um eine dritte Population von TILs zu erzeugen, wobei die dritte Population von TILs eine therapeutische Population ist.

4. Expandierte tumorinfiltrierende Lymphozyten (TILs) zur Verwendung nach einem der Ansprüche 1 bis 2, oder das Verfahren nach Anspruch 3, wobei der eine oder die mehreren TME-Stimulatoren mindestens eine Substanz der Gruppe A, ausgewählt aus einer oder mehreren aus der Gruppe, bestehend aus Pembrolizumab, Nivolumab, Cemiplimab, sym021, Atezolizumab, Avelumab und Durvalumab, und eine Substanz der Gruppe B, ausgewählt aus einer oder mehreren aus der Gruppe, bestehend aus Ipilimumab und Tremelimumab, umfasst.

5. Expandierte tumorinfiltrierende Lymphozyten (TILs) zur Verwendung nach Anspruch 4, oder Verfahren nach Anspruch 4, ferner umfassend eine oder mehrere Substanzen der Gruppe J, ausgewählt aus einer oder mehreren aus der Gruppe, bestehend aus Urelumab und Utomilumab.

6. Expandierte tumorinfiltrierende Lymphozyten (TILs) zur Verwendung nach Anspruch 5, oder Verfahren nach Anspruch 5, wobei Gruppe J in Kombination mit einer Anti-CD3-Substanz verwendet wird.

7. Expandierte tumorinfiltrierende Lymphozyten (TILs) zur Verwendung nach einem der Ansprüche 1 bis 2 oder 4 bis 6, oder Verfahren nach einem der Ansprüche 3 bis 6, wobei die Konzentration der Substanz 0,1 pg/ml bis 300 pg/ml, wie 1 pg/ml bis 100 pg/ml, wie 10 pg/ml bis 100 pg/ml, wie 1 pg/ml bis 10 pg/ml, wie 2 bis 20 pg/ml, beträgt.

8. Expandierte tumorinfiltrierende Lymphozyten (TILs) zur Verwendung nach einem der Ansprüche 1 bis 2 oder 4 bis 7, oder Verfahren nach einem der Ansprüche 3 bis 7, wobei die Schritte (a) bis (b) innerhalb eines Zeitraums von etwa 7 Tagen bis etwa 28 Tagen durchgeführt werden.

9. Expandierte tumorinfiltrierende Lymphozyten (TILs) zur Verwendung nach einem der Ansprüche 1 bis 2 oder 4 bis 8, oder Verfahren nach einem der Ansprüche 3 bis 8, wobei Schritt (c) innerhalb eines Zeitraums von etwa 7 Tagen bis etwa 21 Tagen durchgeführt wird.

10. Expandierte tumorinfiltrierende Lymphozyten (TILs) zur Verwendung nach einem der Ansprüche 1 bis 2 oder 4 bis 9, oder Verfahren nach einem der Ansprüche 3 bis 9, wobei die therapeutische Population von T-Zellen verwendet wird, um einen Krebstyp zu behandeln, der ausgewählt ist aus der Gruppe, bestehend aus Brustkrebs, Nierenzellkrebs, Blasenkrebs, Melanom, Zervixkrebs, Magenkrebs, Dickdarmkrebs, Lungenkrebs, Kopf- und Halskrebs, Ovarialkrebs, Hodgkin-Lymphom, Bauchspeicheldrüsenkrebs, Leberkrebs und Sarkomen.

11. Expandierte tumorinfiltrierende Lymphozyten (TILs) zur Verwendung nach einem der Ansprüche 1 bis 2 oder 4 bis 10, oder Verfahren nach einem der Ansprüche 3 bis 10, wobei Schritt (c) in 1 × 10⁷ bis 1 × 10¹² Zellen, wie 1 × 10⁸ bis 5 × 10⁹ Zellen, wie 1 × 10⁹ bis 5 × 10⁹ Zellen, wie 1 × 10⁸ bis 5 × 10¹⁰ Zellen, wie 1 × 10⁹ bis 5 × 10¹¹ Zellen, resultiert.

12. Expandierte tumorinfiltrierende Lymphozyten (TILs) zur Verwendung nach Ansprüchen 1 bis 2 oder 4 bis 11, oder Verfahren nach einem der Ansprüche 3 bis 11, wobei der Anti-CD3-Antikörper OKT3 ist.

13. Expandierte tumorinfiltrierende Lymphozyten (TILs) zur Verwendung nach Ansprüchen 1 bis 2 oder 4 bis 12, oder Verfahren nach einem der Ansprüche 3 bis 12, wobei die TME-Stimulatoren zusammen oder mit Zeitverzögerung, d. h. einen Tag auseinander, oder wie 2, 3, 4, 5, 6 oder 7 Tage auseinander, hinzugefügt werden.

14. Expandierte tumorinfiltrierende Lymphozyten (TILs) zur Verwendung nach Ansprüchen 1 bis 2 oder 4 bis 13, oder Verfahren nach einem der Ansprüche 3 bis 13, wobei die antigenpräsentierenden Zellen (APCs) ausgewählt sind aus der Gruppe, bestehend aus allogenen Feederzellen, PBMCs und künstlichen antigenpräsentierenden Feederzellen.

## Revendications

1. Lymphocytes expansés infiltrant les tumeurs (TIL) destinés à être utilisés dans le traitement d'un sujet atteint d'un cancer, le traitement comprenant les étapes :
- (a) de culture des cellules T autologues en obtenant une première population de TIL à partir d'une tumeur réséquée chez un mammifère
- (b) de réalisation d'une première expansion en cultivant la première population de TIL dans un milieu de culture cellulaire comprenant de l'IL-2 et un ou plusieurs stimulateurs de TME pour produire une deuxième population de TIL, dans lesquels les un ou plusieurs stimulateurs de TME comprennent au moins une substance de groupe B choisi parmi un ou plusieurs membres du groupe constitué de l'ipilimumab et du tremelimumab,
- (c) de réalisation d'une seconde expansion en complétant le milieu de culture cellulaire de la deuxième population de TIL avec de l'IL-2 supplémentaire, un anticorps anti-CD3 et des cellules présentatrices d'antigène (APC), pour produire une troisième population de TIL, dans lesquels la troisième population des TIL constitue une population thérapeutique ; et
- (d) après avoir administré une chimiothérapie lymphodéplétive non myéloablative, d'administration au mammifère la population thérapeutique de cellules T, moyennant quoi la régression du cancer chez le mammifère est favorisée.

2. Lymphocytes expansés infiltrant les tumeurs (TIL) destinés à être utilisés pour favoriser la régression d'un cancer chez un sujet atteint de cancer, la régression comprenant les étapes :
- (a) de culture des cellules T autologues en obtenant une première population de TIL à partir d'une tumeur réséquée chez un mammifère
- (b) de réalisation d'une première expansion en cultivant la première population de TIL dans un milieu de culture cellulaire comprenant de l'IL-2 et un ou plusieurs stimulateurs de TME pour produire une deuxième population de TIL, dans lesquels les un ou plusieurs stimulateurs de TME comprennent au moins une substance de groupe B choisi parmi un ou plusieurs membres du groupe constitué de l'ipilimumab et du tremelimumab,
- (c) de réalisation d'une seconde expansion en complétant le milieu de culture cellulaire de la deuxième population de TIL avec de l'IL-2 supplémentaire, un anticorps anti-CD3 et des cellules présentatrices d'antigène (APC), pour produire une troisième population de TIL, dans lesquels la troisième population des TIL constitue une population thérapeutique ; et
- (d) après avoir administré une chimiothérapie lymphodéplétive non myéloablative, d'administration au mammifère la population thérapeutique de cellules T, moyennant quoi la régression du cancer chez le mammifère est favorisée.

3. Procédé destiné à développer des lymphocytes infiltrant les tumeurs (TIL) dans une population thérapeutique de TIL comprenant :
- (a) la culture des cellules T autologues en obtenant une première population de TIL à partir d'une tumeur réséquée chez un mammifère
- (b) la réalisation d'une première expansion en cultivant la première population de TIL dans un milieu de culture cellulaire comprenant de l'IL-2 et un ou plusieurs stimulateurs de TME pour produire une deuxième population de TIL, dans lequel les un ou plusieurs stimulateurs de TME comprennent au moins une substance de groupe B choisi parmi un ou plusieurs membres du groupe constitué de l'ipilimumab et du tremelimumab, et
- (c) la réalisation d'une seconde expansion en complétant le milieu de culture cellulaire de la deuxième population de TIL avec de l'IL-2 supplémentaire, un anticorps anti-CD3 et des cellules présentatrices d'antigène (APC), pour produire une troisième population de TIL, dans lequel la troisième population des TIL constitue une population thérapeutique.

4. Lymphocytes expansés infiltrant les tumeurs (TIL) destinés à être utilisés selon l'une quelconque des revendications 1 et 2, ou procédé selon la revendication 3, dans lequel les un ou plusieurs stimulateurs de TME comprennent au moins une substance du groupe A sélectionnée parmi un ou plusieurs membres du groupe constitué du pembrolizumab, du nivolumab, du cemiplimab, du sym021, de l'atezolizumab, de l'avelumab et du durvalumab, et d'une substance du groupe B choisie parmi un ou plusieurs membres du groupe constitué de l'ipilimumab et du tremelimumab.

5. Lymphocytes expansés infiltrant les tumeurs (TIL) destinés à être utilisés selon la revendication 4, ou procédé selon la revendication 4, comprenant en outre un ou plusieurs membres du groupe J choisies parmi une ou plusieurs substances du groupe constitué de l'urelumab et de l'utomilumab.

6. Lymphocytes expansés infiltrant les tumeurs (TIL) destinés à être utilisés selon la revendication 5, ou procédé selon la revendication 5, dans lesquels le groupe J est utilisé en combinaison avec une substance anti-CD3.

7. Lymphocytes expansés infiltrant les tumeurs (TIL) destinés à être utilisés selon l'une quelconque des revendications 1 et 2 ou 4 à 6, ou procédé selon l'une quelconque des revendications 3 à 6, dans lequel la concentration de la substance est de 0,1 µg/ml à 300 µg/ml, telle que 1 µg/ml à 100 µg/ml, telle que 10 µg/ml à 100 µg/ml, telle que 1 µg/ml à 10 µg/ml, telle que 2 à 20 pg/ml.

8. Lymphocytes expansés infiltrant les tumeurs (TIL) destinés à être utilisés selon l'une quelconque des revendications 1 et 2 ou 4 à 7, ou procédé selon l'une quelconque des revendications 3 à 7, dans lesquels les étapes (a) et (b) sont réalisées dans un délai d'environ 7 jours à environ 28 jours.

9. Lymphocytes expansés infiltrant les tumeurs (TIL) destinés à être utilisés selon l'une quelconque des revendications 1 et 2 ou 4 à 8, ou procédé selon l'une quelconque des revendications 3 à 8, dans lesquels l'étape (c) est réalisée dans un délai d'environ 7 jours à environ 21 jours.

10. Lymphocytes expansés infiltrant les tumeurs (TIL) destinés à être utilisés selon l'une quelconque des revendications 1 et 2 ou 4 à 9, ou procédé selon l'une quelconque des revendications 3 à 9, dans lesquels la population thérapeutique de cellules T est utilisée pour traiter un type de cancer choisi parmi les groupes comprenant le cancer du sein, le cancer des cellules rénales, le cancer de la vessie, le mélanome, le cancer du col de l'utérus, le cancer gastrique, le cancer colorectal, le cancer du poumon, le cancer de la tête et du cou, le cancer de l'ovaire, le lymphome hodgkinien, le cancer du pancréas, le cancer du foie et les sarcomes.

11. Lymphocytes expansés infiltrant les tumeurs (TIL) destinés à être utilisés selon l'une quelconque des revendications 1 et 2 ou 4 à 10, ou procédé selon l'une quelconque des revendications 3 à 10, dans lesquels l'étape (c) donne 1 × 10⁷ à 1 × 10¹² cellules, comme 1 × 10⁸ à 5 × 10⁹ cellules, comme 1 × 10⁹ à 5 × 10⁹ cellules, comme 1 × 10⁸ à 5 × 10¹⁰ cellules, comme 1 × 10⁹ à 5 × 10¹¹ cellules.

12. Lymphocytes expansés infiltrant les tumeurs (TIL) destinés à être utilisés selon les revendications 1 et 2 ou 4 à 11 ou procédé selon l'une quelconque des revendications 3 à 11, dans lesquels l'anticorps anti-CD3 est OKT3.

13. Lymphocytes expansés infiltrant les tumeurs (TIL) destinés à être utilisés selon les revendications 1 et 2 ou 4 à 12, ou procédé selon l'une quelconque des revendications 3 à 12, dans lesquels les stimulateurs de TME sont ajoutés ensemble ou en un laps de temps, c'est-à-dire à un jour d'intervalle, ou comme à 2, 3, 4, 5, 6 ou 7 jours d'intervalle.

14. Lymphocytes expansés infiltrant les tumeurs (TIL) destinés à être utilisés selon les revendications 1 et 2 ou 4 à 13, ou le procédé selon l'une quelconque des revendications 3 à 13, dans lesquels les cellules présentatrices d'antigène (APC) sont sélectionnées dans le groupe constitué de cellules nourricières allogéniques (PBMC) et de cellules nourricières artificielles présentant l'antigène.
